# EUROPEAN PATENT SPECIFICATION

(11) **EP 1 602 354 B1**
(45) Date of publication and mention of the grant of the patent: **05.11.2008**
(21) Application number: 04716754.9
(22) Date of filing: 03.03.2004
(51) Int. Cl.: A61K 8/88, A61Q 5/00

(54) **Use of polyglutamic acid or its salts**
Verwendung von Polyglutamatsäure oder deren Salze
Utilisation de l'acide polyglutamique ou ses sels.

(30) Priority: 10.03.2003 JP 2003062688
(43) Date of publication of application: 07.12.2005
(73) Proprietor: Meiji Seika Kaisha Ltd., Chuo-ku, Tokyo 104-8002 (JP)
(72) Inventor: HASEBE, Kohei, c/o Ichimaru Pharcos Co., Ltd., Motosu-shi, Gifu 501-0475 (JP); YAMADA, Kikumi, c/o Ichimaru Pharcos Co., Ltd., Motosu-shi, Gifu 501-0475 (JP)
(74) Representative: Gille Hrabal Struck Neidlein Prop Roos
(86) International application number: PCT/JP2004/002606
(87) International publication number: WO 2004/080433

(56) References cited:
- EP-A- 0 774 247
- WO-A1-20/04039339
- JP-A- 1 146 986
- JP-A- 8 291 036
- JP-A- 10 298 037
- JP-A- 10 298 042
- JP-A- 11 240 827
- JP-A- 59 209 635
- JP-A- 2001 354 542
- JP-A- 2002 145 723

## Description

### Technical Field

The present invention relates to the use of γ-polyglutamic acid or a salt thereof for imparting strength to damaged hair.

### Background Art

The protein structure of hair is denaturated by various kinds of external factors, including: environmental factors such as UV exposure; physical factors such as heat, friction, and dryness due to brushing, driers, and so on; and chemical factors such as components contained in hair dyes, permanent agents, and hair-bleaching agents, so that hair is damaged such as exfoliation of hair cuticles, decreased water contents in hair, loss in interaction or binding between keratin proteins, deletion of protein and other hair constituents of hair, or the like. The damaged state of hair leads to the generation of split hair and broken hair, an increase in frictional force of the surface of hair, a decrease in tension or elasticity of hair, tangled hair, drying out of hair, less smoothness of hair, and so on.

For preventing or alleviating the damage of hair, conventionally, in the cosmetic industry, there have been used silicones, polysaccharides, polypeptides, surfactants, polyols, amino acids, fats and oils, plant extracts, UV absorbers, UV-scattering agents, and so on. In late years, in particular, various useful substances related to the components conventionally used in the art have been newly developed, such as dextrin derivatives (see, JP-A 07-285834), new silicones (JP-A 08-127519, JP-A 08-59440, and JP-A 09-59132), and new peptide derivatives (JP-A 11-302300 and JP-A 2000-86462).

Furthermore, for allowing each component to exert its efficacy sufficiently in parallel with making up for the intrinsic defects of each component (e.g., creak and stiffness generated when applied on hair, and a decrease in feeling of smooth hair, such as a greasy feeling, and a decrease in residual percentage in hair), products have been generally constructed by combining various components, respectively and thus diverse combinations have been conventionally designed in the cosmetic industry (see JP-A 06-157247, JP-A 07-53332, JP-A 08-208439, JP-A 10-279436, JP-A 2000-191445, and JP-A 2002-145741).

However, when a single component is used for hair for example, silicones have excellent hair-coating effects to alleviate split ends and broken hair while causing a decrease in feeling of smooth hair. In addition, polyols have excellent moisture retention and prevent hair from drying but they have an extremely lower alleviating effect on the damage of hair itself. The UV absorber remarkably protects hair from being damaged by UV rays but shows low residual tendency to the hair. In other words, the UV absorber exerts excellent efficacy while simultaneously exerting undesired defects such as depletion in prolonged durability of effectiveness. Besides, making up the defects by the combination of the respective components requires a high prescription technology and an accumulation of a large quantity of technological information in the production. It is still difficult to manufacture a product without defects as described above while preventing or alleviating the damage of hair. In the cosmetics industry, it is strongly expected to develop a single component, which will prevent or alleviate the damage of hair without causing other defects.

On the other hand, polyglutamic acid is one of the components which have been conventionally used for hair cosmetic. The polyglutamic acid has, in addition to its thickening action, superior moisture retention (see JP-B 04-50286), hairdressing effects (see JP-B 47-46910), inflammation-depressing effects and UV-absorbing effects (see JP-A 01-146815), resistance to oxidation (see JP-A 01-146986), stimulatory effects on the production of pyrrolidone carboxylic acid (see JP-A 06-329529), alleviating effects on the feeling of stiffness and creak (see JP-A 10-298037), improving effects on the feeling of smooth hair with a combination of an oxidant, a cationic surfactant, and polyglutamic acid (see JP-A 2002-80330), depressing effects on discomfort due to polyhydric alcohol (JP-A 2002-145723), effects to protect hair and improve the feeling of smooth hair with a combination of wax, silicones, and polyglutamic acid (JP-A 2002-193752), and so on. The efficacies thereof toward various directions have been confirmed, so that the polyglutamic acid will be one of the very beneficial components with respect of its use for hair.

In consideration of the aforementioned problems, as a result of carrying out further investigation into polyglutamic acid which has been conventionally used as a multifunctional thickening agent for hair, the inventors of the present invention have found that γ-polyglutamic acid or a salt thereof can be used for damaged hair to provide the damaged hair with excellent strength while lowering the frictional force of the hair.

Furthermore, the inventors of the present invention have found that an improvement in strength of damaged hair can be attained by constructing a composition for hair treatment containing γ-polyglutamic acid or a salt thereof or a hair cosmetic containing the composition to provide the hair with tension or elasticity while lowering its frictional force, thereby allowing improvements in combing and touch of hair. Furthermore, a moisturizing effect inherent to γ-polyglutamic acid or a salt thereof can prevent or alleviate the development of dandruff without causing the feeling of stickiness or creak. In addition, γ-polyglutamic acid or a salt thereof is a water-soluble polymer. Consequently, the inventors of the present invention have also found that the characteristic features of γ-polyglutamic acid or a salt thereof, such as a moderate residual tendency to hair, which have been conventionally reported, can be sufficiently exerted.

### Disclosure of the Invention

That is, according to claim 1 of the present invention, the use of γ-polyglutamic acid or a salt thereof for imparting strength to damaged hair and lowering the frictional force of hair is provided.

### Brief Description of the Drawings

Fig. 1 is a diagram illustrating the efficacy of γ-polyglutamic acid sodium salt corresponding to the strength of damaged hair.
Fig. 2 is a diagram illustrating the efficacy of γ-polyglutamic acid sodium salt corresponding to the mean frictional coefficient of damaged hair.

### Best Mode for carrying out the Invention

The γ-polyglutamic acid or a salt thereof used in the present invention is a compound having a chemical structure represented by Formula 1. 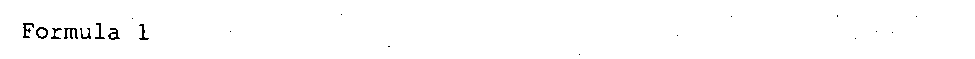 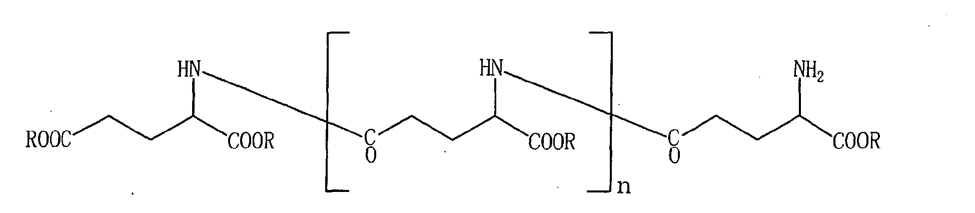 [wherein R is a hydrogen atom or one optionally selected from: alkali metal atoms such as sodium, potassium, and lithium; tertiary amines such as trimethylamine, triethylamine, dimethylamine, diethylamine, trimethanolamine, triethanolamine, dimethanolamine, diethanolamine, and ethanolamine; and quaternary amines such as tetramethylamine and tetraethylamine, where the atomic species or molecule species of R exist in the molecule may be identical to or different from each other, and n in Formula 1 is 400 or more].

The γ-polyglutamic acid or a salt thereof used in the present invention has an average molecular weight of at least 50,000 or more, preferably 100,000 or more, particularly preferably 500,000 or more. The upper limit of the average molecular weight is not specifically limited. However, the upper limit may be placed on an average molecular weight of 4,000,000, for example.

The γ-polyglutamic acid or a salt thereof used in the present invention may be produced by a fermentation process using a microorganism. The useful microorganisms include those belonging to the genus *Bacillus* having the ability to produce γ-polyglutamic acid. Of those, for example, *Bacillus subtilis, Bacillus anthracis, Bacillus licheniformis,* and *Bacillus megaterium* are preferable because of their enhanced abilities to produce γ-polyglutamic acid. Those microorganisms may be those trusted in a depositary institution or the like or may be bacteria or the like commercially available in the market.

Culture media, which can be used for the production of γ-polyglutamic acid in the fermentation process using the microorganism mentioned above, may include natural media composed of natural products, such as shochu-distillery effluent media and soybean extract media, and synthetic or semi-synthetic media composed of respective components with any content concentrations in the media optionally selected from: carbon sources such as glucose, fructose, galactose, sucrose, maltose, mannose, lactose, glycerol, and starch; inorganic nitrogen sources such as ammonium sulfate, ammonium phosphate, and hydrochloric acid ammonium; organic nitrogen sources such as glutamic acid or a salt thereof, aspartic acid or a salt thereof, glutamine, and asparagine; major inorganic salts containing sodium chloride, magnesium sulfate, monopotassium phosphate, and disodium phosphate; minor inorganic salts containing atoms of iron, copper, zinc, cobalt, nickel, boron, manganese, molybdenum, tin, selenium, silicon, arsenic, vanadium, chromium, fluorine, and the like; vitamins such as biotin, nicotinic acid amide, calcium pantothenate, thiamine, riboflavine, and pyridoxine hydrochloride; organic acids such as citric acid, tartaric acid, malic acid, and glycolic acid; natural extract products such as yeast extracts, meat extracts, potato extracts, tomato extracts, and soybean peptides; and so on.

With regard to the culture conditions of the microorganism in the present invention, temperatures are defined within the range of 20 to 37°C and further adjusted in the process of microorganism growth and the production of γ-polyglutamic acid, and pH is defined within the range of 5.0 to 8.0 and further adjusted in the process of microorganism growth and the production of γ-polyglutamic acid. Besides, the duration of culture may be up to the time at which γ-polyglutamic acid is sufficiently produced.

An extraction/purification method for γ-polyglutamic acid or a salt thereof from a culture medium after terminating the culture in the present invention may be optionally selected from known methods including an acid precipitation method, a solvent precipitation method, and a membrane purification method.

The form of γ-polyglutamic acid or a salt thereof in the present invention may be any of those including liquid, solid, powder, paste, and gel forms. The most suitable form can be arbitrarily selected to construct the composition for hair treatment or hair cosmetic for damaged hair.

The content of γ-polyglutamic acid or a salt thereof in the composition for hair treatment may vary slightly depending on the type, quality, desired degree of action, and so on of the composition and is not specifically limited. In general, it is effective when the content of γ-polyglutamic acid or a salt thereof is defined within the range of 0.001% by mass or more, preferably 0.01 to 50.0% by mass in terms of solid content in the total amount of the preparation. In addition, the dosage form of the composition for hair treatment is arbitrary, and examples thereof include ampule, capsule, powder, granule, pill, tablet, solid, liquid, gel, foam, emulsion, cream, ointment, sheet, moose, powder-dispersed, multiple-layered, and aerosol forms.

It is effective when the content of the composition for hair treatment with respect to the hair cosmetic for damaged hair, which may vary slightly depending on the type, quality, desired degree of action, and so on of the hair cosmetic and is not specifically limited, is defined within the concentration range of 0.01% by mass or more, preferably 0.1 to 20.0% by mass in the total amount of the preparation.

The hair cosmetic for damaged hair may find use in the field of any of cosmetics, quasi-drugs, and pharmaceutical preparations as far as it can be applied on hair, and the applications include, but not specifically limited to: cosmetics for washing hair, such as shampoo, rinse agents, and treatment agents; hair-styling cosmetics such as hair cream, hair spray, hair tonic, hair gel, hair lotion, hair oil, hair essence, hair water, hair wax, and hair foam; hair-restoration and growth agents; hair-coloring related products such as hair-coloring pre-treatment agents, post-treatment agents, and intermediate-treatment agents; and permanent agent-related products such as permanent pre-treatment agents, post-treatment agents, and intermediate-treatment agents. In addition, examples of the dosage form of the hair cosmetic include, but not specifically limited to, solid, paste, moose, gel, powder, solution, solubilized, emulsified, powder-dispersed, multiple-layered, and aerosol forms.

In the present invention, in addition to γ-polyglutamic acid or a salt thereof which is an essential ingredient for the composition used for hair treatment and hair cosmetic for damaged hair, the composition for hair treatment and the hair cosmetic for damaged hair may arbitrarily select and use together with, as an additive, a film former, a bioactive ingredient, and a preparation-regulating ingredients such as a coloring agent, a surfactant, a fragrance and a vehicle, and so on. Thus, the superior composition for hair treatment and the hair cosmetic for damaged hair having more various functionalities can be constructed. The content of those ingredients in the preparation is generally within the concentration range of 0.0001 to 50% by mass.

The following component or additive may be added in advance or during a manufacturing process of the composition for hair treatment and hair cosmetic for damaged hair used in the present invention, and whether it is added in advance or during the production process may be chosen appropriately in consideration of workability.

Generally, a film-forming agent can be optionally selected from compounds having property to form a film over a hair. Examples of the compound include: fats and oils such as avocado oil, almond oil, fennel oil, perilla oil, olive oil, orange oil, orange raffer oil, sesame oil, cacao butter, camomile oil, carrot oil, cucumber oil, tallowate, Kukui nut oil, safflower oil, silicone oil, shea butter, liquid shea butter, soybean oil, camellia oil, maize oil, rapeseed oil, persic oil, castor oil (polyoxyethylene (POE) hardened castor oil, derivatives thereof (40, 50, and 60 EO), and the like), jojoba oil, cottonseed oil, peanut oil, turtle oil, mink oil, egg yolk oil, palm oil, palm kernel oil, haze wax, coconut oil, beef tallow, lard, squalene, squalane, pristane, and hydrogenated products thereof (hardened oils); waxes such as beeswax (white beeswax or the like), carnauba wax, spermaceti wax, lanolin, liquid lanolin, reduced lanolin, hard lanolin, candellila wax, montan wax, shellac wax, wax, and rice wax; mineral oils such as liquid paraffin, paraffin wax, solid paraffin, vaseline, paraffin, ozokerite, ceresin, and microcrystalline wax; natural fatty acids such as lauric acid, myristic acid, palmitic acid, stearic acid, behenic acid, oleic acid, linoleic acid, linolenic acid, docosahexaenoic acid, eicosapentaenoic acid, 12-hydroxystearate, undecylenic acid, tall oil, and lanolin fatty acid; and fatty acids of synthetic fatty acids such as isononanoic acid, caproic acid, 2-ethylbutanoic acid, isopentanoic acid, 2-methylpentanoic acid, 2-ethylhexanoic acid, isopentanoic acid, and isostearic acid.

Examples of the compound further include polyethylene glycol, polypropylene glycol, gum arabic, gum benzoin, dammar gum, guaiac resin, Irish moss, karaya gum, tragacanth gum, carob gum, quince seed, agar, casein, dextrin, gelatin, pectin, starch, carageenan, carboxymethyl chitin or chitosan, hydroxyethyl chitosan, or hydroxyalkyl (C2 to C4) chitin or chitosan in which alkylene (C2 to C4) oxide such as ethylene oxide is added, low molecular chitin or chitosan, a chitosan salt, sulfated chitin or chitosan, phosphorylated chitin or chitosan, alginic acid or a salt thereof, hyaluronic acid or a salt thereof, chondroitin sulfate or a salt thereof (such as a sodium chondroitin sulfate salt), heparin, ethylcellulose, methylcellulose, carboxymethylcellulose, carboxyethylcellulose, sodium carboxyethylcellulose, hydroxyethylcellulose, hydroxypropylcellulose, nitrocellulose, crystalline cellulose, hydroxypropylmethylcellulose, polyvinyl alcohol, polyvinyl methyl ether, polyvinylpyrrolidone, vinylpyrrolidone/N,N-dimethylaminoethyl methacrylate copolymer diethylsulfate solution, polyvinyl methacrylate, a polyacrylic acid salt (such as an octylamide acrylate/acrylhydroxypropyl/butylaminoethyl methacrylate copolymer or an alkanolamine acrylic resin), polyalkyleneoxide such as polyethyleneoxide or polypropyleneoxide or a crosslinked polymer thereof (such as a methylsiloxane/polyoxyethylene copolymer), a carboxyvinyl polymer, an alkyl denaturated carboxyvinyl polymer, polyethyleneimine, dermatan sulfate, keratan sulfate, glycerin fatty acid pyrrolidone carboxylates, glycerin fatty acid acetyl amino acid esters, pyrrolidone carboxylate or a salt thereof (such as sodium pyrrolidone carboxylate), polyaspartic acid or a salt thereof, polylysine of a salt thereof, and a water-soluble high molecular polymer such as a 2-methacryloyloxyethyl phosphorylcholine copolymer.

Examples of the compound further include silicone compounds such as a polyether denaturated silicone (such as a polyoxyethylene/methylpolysiloxane copolymer or poly (oxyethylene/oxypropylene) methylpolysiloxane copolymer), oxazoline denaturated organopolysiloxane, alkyl denaturated organopolysiloxane, a styrylketone silicone derivative, dimethylpolysiloxane, methylphenylpolysiloxane, epoxy denaturated silicone, fluorine denaturated silicone, alcohol denaturated silicone, alkyl denaturated silicon, alkoxy denaturated silicone, amino denaturated silicone, ammonium denaturated polymeric silicone, organopolysiloxane, methylphenylpolysiloxane, polysiloxane, poly (N-acylalkyleneimine) denaturated silicone, dimethylpolysiloxane, methylphenylpolysiloxane, a nitrogen-containing acrylsilicone-based graft copolymer, decamethyl cyclopentanesiloxane, fluorine substituted alkyl denaturated silicone, organopolysiloxanyl silylalkyl denaturated silicone, tricyclic diterpene carboxylate denaturated silicone, trifluoroalkyl denaturated silicone, polydimethylsiloxane, divinylpolydimethylsiloxane, dihydrogenopolydimethylsiloxane, a dihydrogenopolydimethylsiloxane divinylpolydimethylsiloxane polymer, trimethylsiloxysilicic acid, a polyoxypropylene/methylpolysiloxane copolymer, ethylpolysiloxane, ethylmethylpolysiloxane, ethylphenylpolysiloxane, octamethylcyclotetrasiloxane, diorganopolysiloxane, octamethylcyclotetrasiloxane, tetramethyl tetraphenyl tetracyclosiloxane, polyether denaturated polysiloxane, amino denaturated polysiloxane, epoxy denaturated polysiloxane, fluorine denaturated polysiloxane, alcohol denaturated polysiloxane, alkyl denaturated polysiloxane, dodecamethyl cyclohexasiloxane, laurylmethicone copolyol, an aminoethyl aminopropylsiloxane/dimethylsiloxane copolymer, and eugenol denaturated silicone.

Generally, a bioactive ingredient to be used in the present invention can be optionally selected from compounds having any useful bioactivity to a hair. Examples thereof include a p-aminobenzoic acid derivative, a salicylic acid derivative, a benzenesulfonamide derivative, a imidazole derivative, a naphthalene derivative, hydroxyanthranilic acid or a salt thereof or a derivative thereof, an anthranilic acid derivative, a coumarin derivative, an amino acid derivative (2-amino-3-[1-carboxyl-2-(1H-imidazol-4-yl)ethyl]aminobutanoic acid, 2-amino-3-[1-carboxyl-2-(1H-imidazol-4-yl)ethyl]aminobutanoic acid hydrochloride, 2-amino-3- [1-carboxyl-2-(1H-imidazol-4-yl)ethyl]aminobutanoic acid sodium salt, or 2-amino-3-[1-carboxyl-2-(1H-imidazol-4-yl)ethyl]aminobutanoic acid potassium salt), a benzotriazole derivative, a tetrazole derivative, an imidazoline derivative, a pyrimidine derivative, a dioxane derivative, a camphor derivative, a furan derivative, a pyrone derivative, a nucleic acid derivative, an allantoin derivative, a nicotinic acid derivative, and ascorbic acid or a salt thereof or a derivative thereof (phosphoric acid-L-magnesium ascorbate, ascorbyl palmitate, ascorbyl dipalmitate, glucosyl ascorbic acid, hydroxyproline phosphate ascorbic acid, 5-o-α-D-glucopyranosyl-L-ascorbic acid, L-phosphate ascorbic acid sodium salt, L-phosphate ascorbic acid potassium salt, L-phosphate ascorbic acid magnesium salt, L-phosphate ascorbic acid calcium salt, L-phosphate ascorbic acid aluminum salt, L-sulfate ascorbic acid sodium salt, L-sulfate ascorbic acid potassium salt, L-sulfate ascorbic acid magnesium salt, L-sulfate ascorbic acid calcium salt, L-sulfate ascorbic acid aluminum salt, L-sodium ascorbate salt, L-potassium ascorbate salt, L-magnesium ascorbate salt, L-calcium ascorbate salt, L-aluminum ascorbate salt, 6-o-α-D-galactopyranosyl-L-ascorbic acid, 2-o-β-D-galactopyranosyl-L-ascorbic acid, an L-ascorbic acid magnesium phosphate salt, an L-ascorbic acid sodium phosphate salt, an L-ascorbic acid sodium sulfate salt, a 6-o-acylascorbic acid sodium phosphate salt, a 6-o-acylascorbic acid ammonium phosphate salt, a 6-o-acylascorbic acid isopropanolamine salt, a 3-o-isopropyl-L-ascorbic acid, a 6-o-alkylascorbic acid potassium phosphate salt, a 6-o-alkylascorbic acid calcium phosphate salt, a 6-o-alkylascorbic acid barium phosphate salt, a 6-o-alkylascorbic acid ammonium phosphate salt, a 6-o-alkylascorbic acid monoethanolamine phosphate salt, a 6-o-alkylascorbic acid diethanolamine phosphate salt, a 6-o-alkylascorbic acid triethanolamine phosphate salt, a 6-o-alkylascorbic acid monoisopropanolamine phosphate salt, a 6-o-alkylascorbic acid diisopropanolamine phosphate salt, a 6-o-alkylascorbic acid triisopropanolamine phosphate salt, 3-o-glycosyl-L-ascorbic acid, a 6-o-β-Dgalactopyranosyl-L-ascorbic acid, cholesterol phosphated ascorbic acid, L-ascorbyl palmitate, L-ascorbyl isopalmitate, L-ascorbyl dipalmitate, L-ascorbyl diisopalmitate, L-ascorbyl stearate, L-ascorbyl isostearate, L-ascorbyl distearate, L-ascorbyl diisostearate, L-ascorbyl myristate, L-ascorbyl isomyristate, L-ascorbyl dimyristate, L-ascorbyl diisomyristate, L-ascorbyl 2-ethylhexanoate, L-ascorbyl di-2-ethylhexanoate, oleate-L-ascorbic acid, 2-o-α-D-glucosyl-L-ascorbic acid, 2-o-α-D-maltosyl-L-ascorbic acid, 2-o-α-D-maltotriosyl-L-ascorbic acid, 3-o-α-D-glucosyl-L-ascorbic acid, 3-o-α-D-maltosyl-L-ascorbic acid, 2-o-α-D-maltotriosyl-L-ascorbic acid, tetraisopalmitate L-ascorbic acid, tetralaurate L-ascorbic acid, tetra-2-ethylhexanoate L-ascorbic acid, tetraoleate L-ascorbic acid, 5,6-isopropylidene-L-ascorbic acid, retinoate L-ascobic acid, L-ascorbic acid-DL-tocopherol phosphate, L-3-o-ethylascorbic acid, L-ascorbic acid tristearate, L-ascorbic acid tripalmitate, L-ascorbic acid trioleate, L-ascorbic acid triphosphate, 2-o-ascorbyl cinnamate, 2-o-ascorbyl ferulate, 2-o-ascorbyl caffeate, 2-o-ascorbyl cinnapate, 2-o-[6-palmitoylascorbyl]-4'-acetoxyferulate, DL-α-tocopherol-2-L-ascorbic acid phosphodiester, an ascorbic acid inositol-binding derivative, a phosphoamide ascorbate derivative, an ascorbic acid arbutin conjugate, ascorbylphosphoryl cholestelol, a chromanil ascorbic acid derivative, an ascorbic acid cyalate derivative, or the like).

Examples of the compound further include: tocopherol, a salt thereof, and a derivative thereof (such as α-tocopherol, β-tocopherol, γ-tocopherol, δ-tocopherol, ε-tocopherol, α-tocopherylretinoate, aminomethylated tocopherol, hydroxymethylated tocopherol, tocopheryl phosphate, tocopherolacetate, tocopherolnicotinate, tocopherolsuccinate, tocopherollinoleate, tocopherolorotate DL-α-tocopherylglucoside, DL-α-tocopherylmaltoside, DL-β-tocopherylglucoside, DL-α-tocopherylmaltoside, DL-γ-tocopherylglucoside, DL-γ-tocopherylmaltoside, DL-δ-tocopherylglucoside, DL-δ-tocopherylmaltoside, D-α-tocopherylglucoside, D-α-tocopherylmaltoside, D-β-tocopherylglucoside, D-β-tocopherylmaltoside, D-γ-tocopherylglucoside, D-γ-tocopherylmaltoside, D-δ-tocopherylglucoside, D-δ-tocopherylmaltoside, L-α-tocopherylglucoside, L-α-tocopherylmaltoside, L-β-tocopherylglucoside, L-α-tocopherylmaltoside, L-γ-tocopherylglucoside, L-γ-tocopherylmaltoside, L-δ-tocopherylglucoside, L-δ-tocopherylmaltoside, 1-(sulphoethylamino)-3-(α-tocopheryl-6-yloxy)propan-2-ol, 1-(carboxypropylamino)-3-(α-tocopheryl-6-yloxy)propan-2-ol hydrochloride, s-[3-(α-tocopheryl-6-yloxy)-2-hydroxypropyl]cystein, s-[3-(α-tocopheryl-6-yloxy)-2-hydroxypropyl]-γ-glutamyl cystenyl glycine, N-[3-(α-tocopheryl-6-yloxy)-2-hydroxypropyl]aspartic acid, and N-[3-(α-tocopheryl-6-yloxy)-2-hydroxypropyl]glutamic acid); tocotrienol, a salt thereof, or a derivative thereof (such as α-tocotrienol, β-tocotrienol, γ-tocotrienol, δ-tocotrienol, tocotrienol acetate, tocotrienol nicotinate, tocotrienol succinate, tocotrienol linoleate, and tocotrienol orotate); and kojic acid or a derivative thereof (2-methoxymethylhydorxy-4H-pyran-4-one, 2-ethoxymethyl-5-hydroxy-4H-pyran-4-one, 2-benzoyloxymethyl-5-hydroxy-4H-pyran-4-on, 2-cinnamoyloxymethyl-5-hydroxy-4H-pyran-4-one, 2-phenoxymethyl-5-hydroxy-4H-pyran-4-one, a kojic acid glycoside, geranyl acetone, kojic acid monobutyrate, kojic acid monocaprate, kojic acid monopalmitate, kojic acid monostearate, kojic acid monocinnamate, kojic acid monobenzoate, kojic acid dibutyrate, kojic acid dipalmitate, kojic acid distearate, or kojic acid dioleate).

Examples of the compound further include oxybenzone, benzophenone, guaiazulen, sinicon, baicalin or a salt thereof or derivatives thereof, baicalein or a salt thereof or derivatives thereof, berberine or a salt thereof or derivatives thereof, chrysin or a salt thereof or derivatives thereof, apigenin or a salt thereof or derivatives thereof, lutherolin or a salt thereof or derivatives thereof, acacetin or a salt thereof or derivatives thereof, diosmetin or a salt thereof or derivatives thereof, kaempferol or a salt thereof or derivatives thereof, triforine or a salt thereof or derivatives thereof, astragalin or a salt thereof or derivatives thereof, quercetin or a salt thereof or derivatives thereof, quercitrin or a salt thereof or derivatives thereof, isoquercitrin or a salt thereof or derivatives thereof, rutin or a salt thereof or derivatives thereof, morin or a salt thereof or derivatives thereof, myricetin or a salt thereof or derivatives thereof, myricitrin or a salt thereof or derivatives thereof, dachiscetin or a salt thereof or derivatives thereof, quercetagetin or a salt thereof or derivatives thereof, isorhamnetin or a salt thereof or derivatives thereof, pinosembrin or a salt thereof or derivatives thereof, naringenin or a salt thereof or derivatives thereof, hesperetin or a salt thereof or derivatives thereof, eriodictyol or a salt thereof or derivatives thereof, pinobanksin or a salt thereof or derivatives thereof, aromadendrin or a salt thereof or derivatives thereof, engelitin or a salt thereof or derivatives thereof, taxofolin or a salt thereof or derivatives thereof, astilbin or a salt thereof or derivatives thereof, ampelopsin or a salt thereof or derivatives thereof, glutathion or a salt thereof or derivatives thereof, an isoflavone glycoside (6-o-apiosylpuerarin-4'-o-glucoside, 6-o-glucosylpuerarin, 3'-hydroxypuerarin-4'-o-glucoside, 6-o-apiosyl-3'-hydorxypuerarin, or the like), a γ-pyrone glycoside (maltol-3-o-(6'-o-apiosyl)-glucoside, maltol-3-o-(6'-o-apiosyl)-glucoside, or the like), isononylferulate, ellagic acid or a salt thereof or derivatives thereof (5,4-dimetyl ellagic acid, 3,3'-dimethyl ellagic acid, 3,3',4-trimethyl ellagic acid, 3,3',4,4'-tetramethyl-5-methoxy ellagic acid, 3-ethyl-4-methyl-5-hydroxy ellagic acid, amritoside, and the like), rucinol, onjisaponin, *Ophiopogonis* saponin, ruscogenin, sericoside, asiaticoside, hederin, senegin, benzoic anilides (4-hydroxy-N-(2-hydroxyphenyl)benzoic amide, 4-hydroxy-N-(3-hydroxyphenyl)benzamide, 4-hydroxy-N-(4-hydroxyphenyl)benzamide, 3,5-di-t-butyl-4-hydroxy-N-(4-hydroxyphenyl)benzamide,3,5-di-t-butyl-4-hydroxy-N-(3-hydroxyphenyl)benzamide, 3,5-di-t-butyl-4-hydroxy-N-(2-hydroxyphenyl)benzamide), diphenylpiralin, cyproheptadine, triprolidine, dimetindene, ozagrel, isothipendyl, iproheptine, homochlorcyclizine, alimemazine, bucillamine, oxotamide, vidarabine, xanthotoxol, hexachlorophene phenylmercury, mercuric oxide, mercurous chloride, a hydrogen peroxide solution, zinc peroxide, and a placenta (bovine-derived, porcein-derived, sheep-derived, or the like) extract.

Examples of the compound further include hydroquinone or a salt thereof or derivatives thereof including: a hexose glycoside such as hydroquinone α-D-glucose, hydroquinone β-D-glucose, hydroquinone α-L-glucose, hydroquinone β-L-glucose, hydroquinone α-D-galactose, hydroquinone β-D-galactose, hydroquinone α-L-galactose, or hydroquinone β-L-galactose; a pentose glycoside such as hydroquinone α-D-ribose, hydroquinone β-D-ribose, hydroquinone α-L-ribose, hydroquinone β-L-ribose, hydroquinone α-D-arabinose, hydroquinone β-D-arabinose, hydroquinone α-L-arabinose, or hydroquinone β-L-arabinose; an aminoglycoside such as hydroquinone α-D-glucosamine, hydroquinone β-D-glucosamine, hydroquinone α-L-glucosamine, hydroquinone β-L-glucosamine, hydroquinone α-D-galactosamine, hydroquinone β-D-galactosamine, hydroquinone α-L-galactosamine, or hydroquinone β-L-galactosamine; a hydroquinone glycoside of a uronate glycoside such as hydroquinone α-D-glucuronate, hydroquinone β-D-glucuronate, hydroquinone α-L-glucuronate, hydroquinone β-L-glucuronate, hydroquinone α-D-galacturonate, hydroquinone β-D-galacturonate, hydroquinone α-L-galacturonate, or hydroquinone β-L-galacturonate; and a hydroquinone hydroxyalkylether glycoside such as hydroquinone benzylether, 4-β-D-glucopyranosyloxy-1-(4-hydroxyphenoxy)butane, 5-β-D-glucopyranosyloxy-1-(4-hydroxyphenoxy)pentane, 6-β-D-glucopyranosyloxy-1-(4-hydroxyphenoxy)hexane, 2-β-D-glucopyranosyloxy-1-(4-hydroxyphenoxy)propane, 2-β-D-glucopyranosyloxy-1-(4-hydroxyphenoxy)butane, or 2-β-D-glucopyranosyloxy-1-(4-hydroxyphenoxy)propane-3-ol.

Examples of the compound further include: an isonitrile antibiotic such as isonitrin A, isonitrin B, isonitrin C, isonitrin D, isonitrate E, isonitrate F, delmadein, or tricobilidein; an orseillin derivative such as orsellinate, ethylester orcinol orseillinate, p-geranylorseillinate, p-ethylester geranylorcinol geranylorseillinate, p-farnesylorseillinate, p-ethylester farnesylorcinol farnesylorsellinate, p-dodecanylorseillinate, p-ethylester dodecanylorcinol dodecanylorseillinate, p-tetradecanylorseillinate, p-ethylester tetradecanylorcinol tetradecanylorseillinate, p-hexadecanylorseillinate, p-ethylester hexadecanylorcinol hexadecanylorseillinate, p-undecanylorseillinate, p-ethylester undecanylorcinol undenacylorseillinate, p-tridecanylorseillinate, p-ethylester undecanylorcinol tridecanylorseillinate, p-pentadecanylorseillinate, p-ethylester pentadecanylorcinol pentadecanylorseillinate, ethylhexylorseillinate, p-ethylester ethylhexylorcinol ethylhexylorseillinate, p-cyclohexylmethylorseillinate, p-ethylester cyclohexylmethylorcinol cyclohexylmethylorseillinate, p-hydroxyethylhexylorseillinate methylester, or p-hydroxyethylhexylorseillinate hydroxyethylhexylorcinol; umbellic acid; breferdin; oxydesveratrol; a resorcinol derivative (4-cyclohexylresorcinol); a 3-hydroxyketone compound such as 1,5-bis(p-hydroxyphenyl)-2-hydroxypentane-4-one, 1,5-bis(o,p-dihydroxyphenyl)-2-hydroxypentane-4-one, or 1,5-bis(p-hydroxyphenyl-m-methoxyphenyl)-2-hydroxypentane-4-one; a 1,3-diketone compound such as 1,5-bis(p-hydroxyphenyl)-2,4-pentanedione, 1,5-bis(o,p-dihydroxyphenyl)-2,4-pentanedione, or 1,5-bis(p-hydroxyphenyl-m-methoxyphenyl)-2,4-pentanedione; bishydroxybenzylamides; γ-aminobutyrate or a derivative thereof such as N-methyl-γ-aminobutyrate, N-dimethyl-γ-aminobutyrate, or γ-oleylester aminobutyrate; and rucinol.

Examples of the compound further include: lobeline or a lobeline derivative; a rikuiritin derivative such as rikuiritin-α-glucoside or rikuiritin-α-maltoside; a phenylchroman derivative; a chromone derivative such as 2-butylchromone, 2-pentylchromone, 2-heptylchromone, 2-nonylchromone, 2-hexadecylchromone, 2-(1-ethylpentyl)chromone, 2-butyl-7-methoxychromone, 2-pentyl-7-methoxychromone, 2-heptyl-7-methoxychromone, 2-nonyl-7-methoxychromone, 2-pentadecyl-7-methoxychromone, 2-(1-ethylpentyl)-7-methoxychromone, 7-hydroxy-2-methylchromone, 7-hydroxy-2-butylchromone, 7-hydroxy-2-pentylchromone, 7-hydroxy-2-heptylchromone, 7-hydroxy-2-nonylchromone, 7-hydroxy-2-pentadecylchromone, or 7-hydroxy-2-(1-ethylpentyl)chromone; an azelaic acid derivative such as monoalkylester azelaic acid or dialkylester azelaic acid; phosphatidylglucosamine; lysophosphatidylglucoamine; phenylhydroquinone; 3-β-D-glucopyranosylmanul; 3-β-D-maltopyranosylmanul; and a derivative of a substituted amino acid such as DL-N-formyl-3-(1-naphthyl)alanine, DL-N-acetyl-3-(1-naphthyl)alanine, DL-N-propionyl-3-(1-naphthyl)alanine, DL-N-butyryl-3-(1-naphthyl)alanine, DL-N-isobutyryl-3-(1-naphthyl)alanine, DL-N-valeryl-3-(1-naphthyl)alanine, DL-N-isovaleryl-3-(1-naphthyl)alanine, DL-N-(2-methylvaleryl)-3-(1-naphthyl)alanine, DL-N-(3-methylvaleryl)-3-(1-naphthyl)alanine, DL-N-(4-methylvaleryl)-3-(1-naphthyl)alanine, DL-N-t-butylacetyl-3-(1-naphthyl)alanine, DL-N-pivaloyl-3-(1-naphthyl)alanine, DL-N-caproyl-3-(1-naphthyl)alanine, DL-N-(2-ethylhexanoyl)-3-(1-naphthyl)alanine, DL-N-(2-methylhexanoyl)-3-(1-naphthyl)alanine, DL-N-heptanoyl-3-(1-naphthyl)alanine, DL-N-octanoyl-3-(1-naphthyl)alanine, DL-N-(2-propylpentanoyl)-3-(1-naphthyl)alanine, DL-N-nonanoyl-3-(1-naphthyl)alanine, DL-N-decanoyl-3-(1-naphthyl)alanine, DL-N-undecanoyl-3-(1-naphthyl) alanine, DL-N-dodecanoyl-3-(1-naphthyl)alanine, DL-N-tridecanoyl-3-(1-naphthyl)alanine, DL-N-tetradecanoyl-3-(1-naphthyl)alanine, DL-N-pentadecanoyl-3-(1-naphthyl)alanine, DL-N-hexadecanoyl-3-(1-naphthyl)alanine, DL-N-heptadecanoyl-3-(1-naphthyl)alanine, DL-N-octadecanoyl-3-(1-naphthyl)alanine, DL-N-nonadecanoyl-DL-3-(1-naphthyl)alanine, DL-N-icosanoyl-3-(1-naphthyl)alanine, DL-N-acroyl-3-(1-naphthyl)alanine, DL-N-crotonyroyl-3-(1-naphthyl)alanine, DL-N-methacryloyl-3-(1-naphthyl)alanine, DL-N-vinylacetyl-3-(1-naphthyl)alanine, DL-N-cycropropanoyl-3-(1-naphthyl)alanine, DL-N-(2-pentenoyl)-3-(1-naphthyl)alanine, DL-N-(4-pentenoyl)-3-(1-naphthyl)alanine, DL-N-(2-hexenoyl)-3-(1-naphthyl)alanine, DL-N-(3-hexenoyl)-3-(1-naphthyl)alanine, DL-N-(2-methyl-3-pentenoyl)-3-(1-naphthyl)alanine, DL-N-cyclohexenoyl-3-(1-naphthyl)alanine, DL-N-(10-undecenoyl)-3-(1-naphthyl)alanine, DL-N-linoleyl-3-(1-naphthyl)alanine, DL-N-hydroxyacetyl-3-(1-naphthyl)alanine, DL-N-(6-hydroxycaproyl)-3-(1-naphthyl)alanine, DL-N-(8-hydroxyoctanoyl)-3-(1-naphthyl)alanine, DL-N-(9-hydroxynonanoyl)-3-(1-naphthyl)alanine, DL-N-(10-hydroxydecanoyl)-3-(1-naphthyl)alanine, DL-N-(11-hydroxyundecanoyl)-3-(1-naphthyl)alanine, DL-N-(12-hydroxydecanoyl)-3-(1-naphthyl)alanine, DL-N-benzoyl-3-(1-naphthyl)alanine, DL-N-(2-hydroxybenzoyl)-3-(1-naphthyl)alanine, DL-N-(3-hydroxybenzoyl)-3-(1-naphthyl)alanine, DL-N-(4-hydroxybenzoyl)-3-(1-naphthyl)alanine, DL-N-(o-tolyl)-3-(1-naphthyl)alanine, DL-N-(m-tolyl)-3-(1-naphthyl)alanine, DL-N-(p-tolyl)-3-(1-naphthyl)alanine, DL-N-(1-naphtolyl)-3-(1-naphthyl)alanine, DL-N-(2-naphtolyl)-3-(1-naphthyl)alanine, DL-N-(2-carboxybenzoyl)-3-(1-naphthyl)alanine, DL-N-(3-carboxybenzoyl)-3-(1-naphthyl)alanine, DL-N-(4-carboxybenzoyl)-3-(1-naphthyl)alanine, DL-N-(2-picoliloyl)-3-(1-naphthyl)alanine, DL-N-(3-picoliloyl)-3-(1-naphthyl)alanine, DL-N-(4-picoliloyl)-3-(1-naphthyl)alanine, DL-N-phenylacetyl-3-(1-naphthyl)alanine, DL-N-(2-phenylpropanoyl)-3-(1-naphthyl)alanine, DL-N-(3-phenylbutyryl)-3-(1-naphthyl)alanine, DL-N-(4-methylvaleryl)-3-(1-naphthyl)alanine, DL-N-valeryl-3-(1-naphthyl)alanine, DL-N-(4-methylvaleryl)-3-(1-naphthyl)alanineamide, DL-N-(4-methylvaleryl)-3-(1-naphthyl)alanine methylester, DL-N-(4-methylvaleryl)-3-(1-naphthyl)alanine ethylester, DL-N-(4-methylvaleryl)-3-(1-naphthyl)alanine propylester, DL-N-(4-methylvaleryl)-3-(1-naphthyl)alanine-N-butylester, DL-N-(4-methylvaleryl)-3-(1-naphthyl)alanine pentylester, DL-N-(4-methylvaleryl)-3-(1-naphthyl)alanine isopropylester, DL-N-(4-methylvaleryl)-3-(1-naphthyl)alanine isobutylester, or DL-N-(4-methylvaleryl)-3-(1-naphthyl)alanine-t-butylester.

Examples of the compound further include: a benzolactam derivative; an indolactam derivative; cedrol; guaiol; 1-(4-hydroxyphenythio)-2-propanol; β-lactoglobulin; 2-methoxy-5-methylphenol; 5-ethyl-2-methoxyphenol; 5-N-propyl-2-methoxyphenol; 5-N-butyl-2-methoxyphenol; 5-N-hexyl-2-methoxyphenol; 5-N-heptyl-2-methoxyphenol; 5-N-decyl-2-methoxyphenol; 5-(1,1-dimethylpropyl)-2-methoxyphenol; 5-(1,1-dimethylbutyl)-2-methoxyphenol; 5-(1,1-dimethylethyl)-2-methoxyphenol; 2-methoxy-5-(1-methylpentyl)phenol; 2-methoxy-5-(1-methylhexyl)phenol; 2-methoxy-5-(3-methylhexyl)phenol; 2-methoxy-5-(6-methylheptyl)phenol; 5-(1,3-dimethylheptyl)-2-methoxyphenol; marbelin; ferruginol; sugiol; criptojaponol; 1,5-bis[p-hydroxyphenyl]-1,4-pentadiene-3-one; 1,5-bis[o-hydroxyphenyl]-1,4-pentadiene-3-one; 1,5-bis[2,4-dihydroxyphenyl]-1,4-pentadiene-3-one; 1,5-bis[3-methoxy-4-hydroxyphenyl]-1,4-pentadiene-3-one; haginin; agrimophol; agrimol; hydrangenol or a derivative thereof; alkylresorcinol or a derivative thereof (4-N-butylresorcinol or the like); aristron; calamenenes such as calamenene, 7-hydroxy calamenene, 5-hydroxy calamenene, and 7-methoxy calamenene; trans-umbellic acid; N-α-benzoyl-L-arginine; N-α-benzoyl-L-arginine ethylester; 5-methyl-2(3H)-furanone; 2-butene-4-oride; 2-hydroxymethylfuran; 2,5-dimethyl-4-hydroxy-3(2H)-furanone; 2-formylfuran; 3-formylfuran; methyl-α-furylketone; furfurylacetate; 2-hydroxy-3-methyl-2-cyclopentene-1-one; 2-hydroxy-3,5-dimethyl-2-cyclopentene-1-one; 2,5-dimethyl-4-hydroxy-3(2H)-thiophenone; 2-hydroxy-3-ethyl-2-cyclopentene-1-one; tetronic acid; pentanedion; iminodibenzyls such as 2,2'-iminodibenzyl, imipramine, imipramine hydrochloride, desipramine, desipramine hydrochloride, chlorimipramine, and trimipramine; dibenzocycloheptanedienes such as amitriptyline, amitriptyline hydrochloride, nortriptyline, and noxyptyline; tetrahydrocoparol glycosides such as ketotifen fumarate, labda-8(17),13-diene-15-ol, tetrahydromanul, tetrahydrocoparol, tetrahydrocoparolglucoside, tetrahydrocoparolgalactoside, tetrahydrocoparolmaltoside, tetrahydrocoparolcellobioside, and tetrahydrocoparolmaltotrioside; a spiroether compound; piokelin; a phenothiazine compound; promethazine; alimemazine; alimemazine tartrate; triflupromazine; levomepromazine; chlorpromazine; and cyclandelate.

Examples of the compound further include 4-carboxymethyloxybenzoic acid, 4-carboxymethyloxy-2-hydroxybenzoic acid, 3-(3-carboxypropyl-1-oxy)-2-hydroxybenzoic acid, 4-(3-carboxypropyl-1-oxy)benzoic acid, 4-(3-carboxypropyl-1-oxy)-2-hydroxybenzoic acid, 4-(3-carboxypropyl-1-oxy)-2-methoxybenzoic acid, 5-(3-carboxypropyl-1-oxy)-2-hydroxybenzoic acid, 4-(5-carboxypentyl-1-oxy)-2-hydroxybenzoic acid, 6-(3-carboxypropyl-1-oxy)-2-hydroxybenzoic acid, 4-(10-carboxydecane-1-oxy)-2-hydroxybenzoic acid, 4-(10-carbamoyldecane-1-oxy)-2-hydroxybenzoic acid, 4-(4-hydroxybutyl-1-oxy)benzoic acid, 4-(4-hydroxybutyl-1-oxy)-2-hydroxybenzoic acid, 4-(4-acetoxybutyl-1-oxy)benzoic acid, 4-(4-acetoxybutyl-1-oxy)-2-hydroxybenzoic acid, 4-(3-ethoxycarbonylpropyl-1-oxy)-2-hydroxybenzoic acid, 3-(2,3-dihydroxypropyl-1-oxy)-2-hydroxybenzoic acid, 4-(4-methoxybutyl-1-oxy)-2-hydroxybenzoic acid, 4-(2,3-dihydroxypropyl-1-oxy)-2-hydroxybenzoic acid, 4-carboxymethyloxy-2-hydroxybenzoic acid, 3-(3-carboxypropyl-1-oxy)-2-hydroxybenzoic acid, 4-(3-carboxypropyl-1-oxy)-2-hydroxybenzoic acid, 5-(3-carboxypropyl-1-oxy)-2-hydroxybenzoic acid, 6-(3-carboxypropyl-1-oxy)-2-hydroxybenzoic acid, 4-(5-carboxypentyl-1-oxy)-2-hydroxybenzoic acid, 4-(4-hydroxybutyl-1-oxy)-2-hydroxybenzoic acid, 9-(10-carboxydecane-1-oxy)-2-hydroxybenzoic acid, hydroxytrimethylcyclohexanes (a glycoside of monosaccharide, disaccharide, or trisaccharide such as 2-hydroxy-4-(2,2,6-trimethyl-1-yl-cyclohexane)butane, 4-(2,2,6-trimethyl-1-yl-cyclohexane)-1-butene, 4-(2,2,6-trimethyl-1-yl-cyclohexane)-2-butene, 4-(2,2,6-trimethyl-1-yl-cyclohexane)butane, 3-methyl-3-hydroxy-5-(2,2,6-trimethyl-1-yl-cyclohexane)pentane, 3-methyl-1-hydroxy-5-(2,2,6-trimethyl-1-yl-cyclohexane)pentane, 3-methyl-5-(2,2,6-trimethyl-1-yl-cyclohexane)pentane, 3-methyl-1-hydroxy-5-(2,2,6-trimethyl-1-yl-cyclohexane)-3-pentene, 3-methyl-3-hydroxy-5-(2,2,6-trimethyl-1-yl-cyclohexane)-1-pentene, 3-methyl-1-hydroxy-5-(2,2,6-trimethyl-1-yl-cyclohexane)-2-pentene, or 2-hydroxy-4-(2,2,6-trimethyl-1-yl-cyclohexane)butane), escinol, a glycoside of monosaccharide, disaccharide, or trisaccharide such as parahydroxycinnamate-4-(2,2,6-trimethyl-yl-cyclohexane)-2-butylester, onjisaponin, *Ophiopogon* saponin, ruscogenin, sericoside, asiaticoside, herein, and senegin.

Examples of the compound further include 4-(2,2,6-trimethyl-1-yl-cyclohexane)-2-keto-butane, 4-(2,2,6-trimethyl-1-yl-6-cyclohexene)-2-keto-butane, 4-(2,2,6-trimethyl-1-yl-cyclohexane)-2-keto-3-butene, 4-(2,2,6-trimethyl-1-yl-6-cyclohexene)-2-keto-3-butene (β-ionone), L-p-hydroxyphenylglycine, D-p-hydroxyphenylglycine, N-benzyloxycarbonyl-L-p-hydroxyphenylglycine, N-benzyloxycarbonyl-D-p-hydroxyphenylglycine, N-benzoyl-L-p-hydroxyphenylglycine, N-benzoyl-D-p-hydroxyphenylglycine, N-(p-methylxybenzoyl)-L-p-hydroxyphenylglycine, N-(p-methoxybenzoyl)-D-p-hydroxyphenylglycine, N-(p-hydroxybenzoyl)-L-p-hydroxyphenylglycine, N-(p-hydroxybenzoyl)-D-p-hydroxyphenylglycine, N-acetyl-L-p-hydroxyphenylglycine, N-acetyl-D-p-hydroxyphenylglycine, N-acetyl-L-p-hydroxyphenylglycine ethylester, N-acetyl-D-p-hydroxyphenylglycine ethylester, N-acetyl-L-p-hydroxyphenylglycineamide, N-acetyl-D-p-hydroxyphenylglycineamide, L-p-methoxyphenylglycine, D-p-methoxyphenylglycine, L-p-methoxyphenylglycine hydrochloride, D-p-methoxyphenylglycine hydrochloride, 4-hydroxy-3-methoxy-L-phenylglycine, 4-hydroxy-3-methoxy-D-phenylglycine, L-p-hydroxyphenylglycine ethylamide, D-p-hydroxyphenylglycine ethylamide, N-tert-butoxycarbonyl-L-p-hydroxyphenylglycine, N-tert-butoxycarbonyl-D-p-hydroxyphenylglycine, N-tert-butoxycarbonyl-L-p-methoxyphenylglycine, N-tert-butoxycarbonyl-D-p-methoxyphenylglycine, N-9-fluorenylmethyloxycarbonyl-L-p-methoxyphenylglycine, N-9-fluorenylmethyloxycarbonyl-D-p-methoxyphenylglycine, N-9-fluorenylmethyloxycarbonyl-L-p-methoxyphenylglycine benzylester hydrochloride, N-9-fluorenylmethyloxycarbonyl-D-p-methoxyphenylglycine benzylester hydrochloride, L-p-hydroxyphenylglycineamid, D-p-hydroxyphenylglycineamide, L-p-hydroxyphenylglycine allylester p-toluenesulfonate, D-p-hydroxyphenylglycine allylester p-toluenesulfonate, L-p-hydroxyphenylglycine benzylester p-toluenesulfonate, D-p-hydroxyphenylglycine benzylester p-toluenesulfonate, L-p-hydroxyphenylglycine ethyl ester, D-p-hydroxyphenylglycine ethyl ester, L-p-hydroxyphenylglycine ethyl ester hydrochloride, D-p-hydroxyphenylglycine ethyl ester hydrochloride, L-p-hydroxyphenylglycine methyl ester, D-p-hydroxyphenylglycine methyl ester, 1,3-diallylindan-2-carboxylic acids, stachibocins, and a pheophorbide derivative.

Examples of the compound further include ereuserine, isoereuserine or 4-β-hydroxyisoereuserine, ereuserinol, a spiroketal derivative (such as 2-(2,4-hexadiynilidene)-1,6-dioxaspiro[4.5]deca-3-ene, 2-(2-hexynilidene)-1,6-dioxaspiro[4.4]nona-3-ene, 2-((4-methylphenyl)methylidene)-6,6-dioxaspiro[4.4]nona-3-ene, 2-(2-hexenylidene)-6,6-dioxaspiro[4.5]deca-3-ene, 2-(2-hexenylidene)-1,6-dioxaspiro[4.4]nona-3-ene, 2-hexyl-1,6-dioxaspiro[4.4]nonane, 2-(2-hexenyl)-1,6-dioxaspiro[4.4]nonane, 2-(2-hexynyl)-1,6-dioxaspiro[4.4]nonane, 2-pentyl-1,6-dioxaspiro[4.4]nonane, 1,6-dioxaspiro[4.4]nonane, 1,6-dioxaspiro[4.5]decane, 1,7-dioxaspiro[5.5]undecane, 2,3-benzo-4,4-dimethyl-1,6-dioxaspiro[4.4]nonane, 3,4-benzo-2-pentyl-1,6-dioxaspiro[4.4]nonane, 3,4-benzo-2-hexyl-1,6-dioxaspiro[4.4]nonane, 3,4-benzo-2-octyl-1,6-dioxaspiro[4.4]nonane, 2-hexyl-9,9-dimethyl-1,6-dioxaspiro[4.4]nonane, or 2-(2,4-hexadiynilidene)-1,6-dioxaspiro[4.4]nona-3-ene), malvalic acid, a phosphonic acid derivative or a salt thereof, aspergillomarasmin, a aminophosphonic acid derivative or a salt thereof, diphenhydramine or a salt thereof, pregnenolone or a derivative thereof, lutein, a farnesylisopropanol derivative, hexahydrofarnesylacetone, 4-benzoylamino-2-hydroxybenzoic acid, 5-benzoylamino-2-hydroxybenzoic acid, 4-benzoylaminobenzoic acid, 4-(1-naphthoylamino)-2-hydroxybenzoic acid, 5-(1-naphthoylamino)-2-hydroxybenzoic acid, 4-(2-naphthoylamino)-2-hydroxybenzoic acid, 5-(2-naphthoylamino)-2-hydroxybenzoic acid, 4-(1-naphthoylamino)benzoic acid, 4-(2-naphthoylamino)benzoic acid, 4-phenylaminocarbonylbenzoic acid, 4-phenylaminocarbonyl-2-hydroxybenzoic acid, 5-phenylaminocarbonyl-2-hydroxybenzoic acid, 4-(1-naphthylaminocarbonyl)benzoic acid, 4-(1-naphthylaminocarbonyl)-2-hydroxybenzoic acid, 5-(1-naphthylaminocarbonyl)-2-hydroxybenzoic acid, 4-(2-naphthylaminocarbonyl)benzoic acid, 4-(2-naphthylaminocarbonyl)-2-hydroxybenzoic acid, 5-(2-naphthylaminocarbonyl)-2-hydroxybenzoic acid, a hexahydrofarnesylisopropanol derivative, borneol-p-hydroxycinnamate ester glucoside, borneol-p-hydroxycinnamate ester maltoside, borneol-p-hydroxycinnamate ester maltotrioside, and a cinnamate-4-(2,2,6-trimethyl-yl-cyclohexane)-2-butyl ester derivative.

Examples of the compound further include 2,4-dihydroxybenzophenone, 1-(2,4-dihydroxyphenyl)-ethanone(2',4'-dihydroxyacetophenone), 1-(2,4-dihydroxyphenyl)-1-propanone(2',4'-dihydroxypropiophenone), 1-(2,4-dihydroxyphenyl)-1-butanone, 1-(2,4-dihydroxyphenyl)-1-pentanone, 1-(2,4-dihydroxyphenyl)-1-hexanone, 1-(2,4-dihydroxyphenyl)-1-heptanone, 1-(2,4-dihydroxyphenyl)-1-octanone, 1-(2,4-dihydroxyphenyl)-1-nonanone, 1-(2,4-dihydroxyphenyl)-1-decanone, 1-(2,4-dihydroxyphenyl)-1-undecanone, 1-(2,4-dihydroxyphenyl)-1-dodecanone, 1-(2,4-dihydroxyphenyl)-1-tetradecanone, 1-(2,4-dihydroxyphenyl)-1-hexadecanone, 1-(2,4-dihydroxyphenyl)-1-octadecanone, 1-(2-hydroxy-4-methoxyphenyl)-ethanone(2'-hydroxy-4'-methoxyacetophenone), 1-(2-hydroxy-4-methoxyphenyl)-1-propanone(2'-hydroxy-4'-methoxypropiophenone), 1-(2-hydroxy-4-methoxyphenyl)-1-butanone, 1-(2-hydroxy-4-methoxyphenyl)-1-pentanone, 1-(2-hydroxy-4-methoxyphenyl)-1-hexanone, 1-(2-hydroxy-4-methoxyphenyl)-1-heptanone, 1-(2-hydroxy-4-methoxyphenyl)-1-octanone, 1-(2-hydroxy-4-methoxyphenyl)-1-nonanone, 1-(2-hydroxy-4-methoxyphenyl)-1-decanone, 1-(2-hydroxy-4-methoxyphenyl)-1-undecanone, 1-(2-hydroxy-4-methoxyphenyl)-1-dodecanone, 1-(2-hydroxy4-methoxyphenyl)-1-tetradecanone, 1-(2-hydroxy-4-methoxyphenyl)-1-hexadecanone, 1-(2-hydroxy-4-methoxyphenyl)-1-octadecanone, 1-(4-hydroxy-2-methoxyphenyl)-ethanone(4'-hydroxy-2'-methoxyacetophenone), 1-(4-hydroxy-2-methoxyphenyl)-1-propanone(4'-hydroxy-2'-methoxypropiophenone), 1-(2,4-dimethoxyphenyl)-ethanone(2',4'-dimethoxyacetophenone), 1-(2,4-dimethoxyphenyl)-1-propanone (2',4'-dimethoxypropiophenone), a lactone derivative (such as 1,6-dioxaspiro[4.4]nonane-2,7-dione, 1,6-dioxaspiro[4.5]decane-2,7-dione, 4-tridecanolide, 4-dodecanolide, 4-undecanolide, 4-decanolide, 4-nonanolide, 4-octanolide, 4-heptanolide, 5-dodecanolide, 5-undecanolide, 5-decanolide, 5-nonanolide, 5-octanolide, 2-undecene-4-olide, 2-decene-4-olide, 2-nonene-4-olide, 2-heptene-4-olide, 2-undecene-5-olide, 2-decene-5-olide, 2-nonene-5-olide, 2-octene-5-olide, 4-methyl-4-dodecanolide, 4-methyl-4-undecanolide, 4-methyl-4-decanolide, 4-methyl-4-nonanolide, 4-methyl-4-heptanolide, 5-methyl-5-dodecanolide, 5-methyl-5-undecanolide, 5-methyl-5-decanolide, 5-methyl-5-nonanolide, 5-methyl-5-octanolide, 2-methoxycarbonyl-4-dodecanolide, 2-methoxycarbonyl-4-undecanolide, 2-methoxycarbonyl-4-decanolide, 2-methoxycarbonyl-4-nonanolide, 2-methoxycarbonyl-4-heptanolide, 2-methoxycarbonyl-5-undecanolide, 2-methoxycarbonyl-5-decanolide, 2-methoxycarbonyl-5-nonanolide, 2-methoxycarbonyl-5-octanolide, 2-allyl-4-undecanolide, 2-allyl-5-decanolide, 2-allyl-4-nonanolide, 2-pentyl-4-undecanolide, 2-pentyl-4-nonanolide, 2-methyl-4-undecanolide, 2-methyl-4-nonanolide, 2-(4-hydroxybutyl)-4-undecanolide, 2-(4-hydroxybutyl)-4-nonanolide, 2-(4-hydroxybutyl)-5-decanolide, 5-propyloxy-4-pentanolide, 5-allyloxy-4-pentanolide, 5-(2-hydroxyethoxy)-4-pentanolide, 8-hydroxy-4-octanolide, 6-propyloxy-5-hexanolide, 6-allyloxy-5-hexanolide, 6-(2-hydroxyethoxy)-5-hexanolide, or 9-hydroxy-5-nonanolide), echinomycin, iriflorental, iriparidal, 2'-8-C-glucosyl-7-methylaloesolcoumaroylester, 2'-8-C-glucosyl-7-methylaloesolcinnamoylester, and chloropyramine.

Examples of the compound further include: salicylic acid, a salt thereof, or derivatives thereof such as glucoside salicylic acid, fatty acid ester salicylic acid, alcohol ether salicylic acid, and salicylic acid amides; salicyl alcohol, a salt thereof, or derivatives thereof; apigenin; amentoflavone; ethylene oxide; ethylene glycol; diethylene glycol; triethylene glycol; ethylene glycol monoethyl ether; ethylene glycol monobutyl ether; diethyleneglycol monomethyl ether; diethylene glycol monoethyl ether; propylene oxide; propylene glycol; 1,3-butylene glycol; pentyl glycol; glycerin; polyalcohols such as erythritol, pentaerythritol, dipentaerythritol, threitol, arabitol, xylitol, ribitol, galactitol, sorbitol, mannitol, lactitol, maltitol, sitiritol, laminitol, valienamine, validamine, and validatol; a saccharide or ester thereof such as glucose, galactose, mannose, xylose, fructose, maltose, isomaltose, cellobiose, gentiobiose, trehalose, kojibiose, laminaribiose, nigerose, sanbebiose, neohesperidose, apiose, hamamelose, streptose, hydroxystreptose, dihydrostreptose, 2-methylerythrose or a derivative thereof (trehalose undecylenic acid or the like), 2-methylerythronolactone, micarose, cladinose, accenose, alcanose, olivomicose, chromose, evemicose, vinerose, nogarose, virenose, nobiose, moenuronic acid, garosamin, sibilosamin, N-acylkansosamin, bancosamin, evenitrose, rubranitrose, tetronitrose, pyrarose, α-octose, trioxacalcinose, aldogarose, or blastminocin; trehalose or a derivative thereof; D-mannosamin and a derivative thereof; and primeverose or a derivative thereof.

Examples of the compound further include: sodium lactate; hydantoin and a derivative thereof; N-p-vinylbenzyl-D-cellobionamide; N-p-vinylbenzyl-D-lactonamide; N-p-vinylbenzyl-D-maltonamide; N-p-vinylbenzyl-D-gluconamide; glucosyloxyethyl methacrylate; galactosyloxypropyl acrylate; mannosiloxyethyl methacrylate; glutamyl lysine; glutaurine; 1,2,4-butanetriol; kalan-3,4-diol; and red beans saponin (3-o-[β-D-glucopyranosyl-β-D-glucuronopyranosyl]-sophoradiol, 3-o-[β-D-glucopyranosyl-3-D-glucuronopyranosyl]-soya sapogenol B, 3-o-[β-D-glucopyranosyl-β-D-glucuronopyranosyl]-adsuki sapogenol, 3-o-[β-D-glucopyranosyl]-28-o-[β-D-glucopyranosyl-β-D-glucopyranosyl]-dipsogenic acid, 3-o-[α-L-rhamnopyranosyl-β-D-glucopyranosyl-β-D-glucuronopyranosyl]-soya sapogenol B, 3-o-[β-D-glucopyranosyl-β-D-glucuronopyranosyl]-29-o-[β-D-glucopyranosyl-β-D-glucopyranosyl]-adzuki sapogenol, and the like).

Examples of the compound further include: retinol, a salt thereof, or derivatives thereof (retinol unsaturated aliphatic ester such as retinyl linoleate, retinyl linolenate, retinyl oleate, or retinyl arachidonate); retinal, a salt thereof, or derivatives thereof; dehydroretinal, a salt thereof, or derivatives thereof; retinoic acid, a salt thereof, or derivatives thereof; retinoic acid analogues (4-[[[8-(3,5-dimethylphenyl)-2-naphthalenyl]carbonyl]amino]benzoic acid, 4-[[[8-(3-methylphenyl)-2-naphthalenyl)carbonyl]amino]benzoic acid, 4-[[[8-(4-methylphenyl)-2-naphthalenyl]carbonyl]amino]benzoic acid, 4-[[[8-(2-methylphenyl)-2-naphthalenyl]carbonyl]amino]benzoic acid, 4-[[[8-(3,4-dimethylphenyl)-2-naphthalenyl]carbonyl]amino]benzoic acid, 4-[[[8-(2,4-dimethylphenyl)-2-naphthalenyl]carbonyl]amino]benzoic acid, 4-[[[8-(2-isopropylphenyl)-2-naphthalenyl]carbonyl]amino]benzoic acid, 4-[[[8-(2-ethylphenyl)-2-naphthalenyl]carbonyl]amino]benzoic acid, 4-[[[8-(2-fluorophenyl)-2-naphthalenyl]carbonyl]amino]benzoic acid, 4-[[[8-(2-methoxyphenyl)-2-naphthalenyl]carbonyl]amino]benzoic acid, 4-[[(8-benzyl-2-naphthalenyl)carbonyl]amino]benzoic acid, and 4-[(E)-2-(8-phenyl-2-naphthalenyl)propenyl]benzoic acid); a retinoic acid derivative such as 4-hydroxyphenylretinamide; and retinoid analogues such as 4-[[(5,6-dihydro-5,5-dimethyl-8-ethyl-2-naphthalenyl)amino]carbonyl]benzoic acid, 4-[[(5,6-dihydro-5,5,8-trimethyl-2-naphthalenyl)amino]carbonyl]benzoic acid, 4-[[(5,6-dihydro-5,5-dimethyl-8-phenyl-2-naphthalenyl)amino]carbonyl]benzoic acid, 4-[[(5,6-dihydro-5,5-dimethyl-8-ethyl-2-naphthalenyl)carbonyl]amino]benzoic acid, 4-[[(5,6-dihydro-5,5,8-trimethyl-2-naphthalenyl)-carbonyl]amino]benzoic acid, 4-(E)-[2-(5,6-dihydro-5,5-dimethyl-8-phenyl-2-naphthalenyl)-1-propenyl]benzoic acid, 4-[[(5,6-dihydro-5,5-dimethyl-8-phenyl-2-naphthalenyl)oxy]carbonyl]benzoic acid, 4-[[(5,6-dihydro-5,5-dimethyl-8-phenyl-2-(naphthalenyl)carbonyl]amino]benzoic acid, 4-[[(5,6-dihydro-5,5-dimethyl-8-phenyl-2-(naphthalenyl)carbonyl]oxy]benzoic acid, 4-[[5,6-dihydro-5, 5-dimethyl-8-(2-fluorophenyl)-naphthalenyl]carbonyl]amino]benzoic acid, 4-[[(5,6-dihydro-5,5,6-trimethyl]-8-phenyl-2-(naphthalenyl)carbonyl]amino]benzoic acid, 4-[[(5,6-dihydro-5,5,7-trimethyl-8-phenyl-2-naphthalenyl)carbonyl]benzoic acid, 4-[[(E)-(5,6-dihydro-5,5-dimethyl-8-phenyl)-2-naphthalenyl]vinyl]benzoic acid, 4-[[[(5,6-dihydro-5,5-dimethyl-8-phenyl)-2-naphthalenyl]carbonyl]sulfamyl]benzoic acid, 4-[[[(5,6-dihydro-5,5-dimethyl-8-phenyl-2-naphthalenyl]sulfamyl]carbonyl]benzoic acid, 4-[[(5,6-dihydro-5,5-dimethyl-8-phenyl)-2-naphthalenyl]ethyl]benzoic acid, 4-[[[(5,6-dihydro-5,5-dimethyl-8-phenyl)-2-naphthalenyl]thiocarbonyl]amino]benzoic acid, 4-[[[(5,6-dihydro-5,5-dimethyl-8-phenyl)-2-naphthalenyl]carbonyl]methyl]benzoic acid, 4-[[[(5,6-dihydro-5,5-dimethyl-8-phenyl)-2-naphthalenyl]methyl]oxy]benzoic acid, 4-[[[(5,6-dihydro-5,5-dimethyl-8-phenyl)-2-naphthenyl]oxy]methyl]benzoic acid, 4-[[[(5,6-dihydro-5,5-dimethyl-8-(2,4-dimethylphenyl)]-2-naphthalenyl]carbonyl]amino]benzoic acid, 4-[[[(5,6-dihydro-5,5-dimethyl-8-(4-methylphenyl)]-2-naphthalenyl]carbonyl]amino]benzoic acid, 4-[[(5,6-dihydro-5,5-dimethyl-8-phenyl)-2-naphthalenyl]ethyl]benzoic acid, 4-[[[(5,6-dihydro-5,5-dimethyl-8-phenyl)-2-naphthalenyl]sulfamyl]methyl]benzoic acid, 4-[[(5,6-dihydro-5,5-dimethyl-8-phenyl)-2-naphthalenyl]methyl]amino)benzoic acid, 4-[[[(5,6-dihydro-5,5-dimethyl-8-phenyl)-2-naphthalenyl]amino]thiocarbonyl]benzoic acid, 4-[[[(5,6-dihydro-5,5-dimethyl-8-phenyl)-2-naphthalenyl]methyl]sulfamyl]benzoic acid, 4-[[[(5,6-dihydro-5,5-dimethyl-8-phenyl-2-naphthalenyl)amino]methyl]benzoic acid, 4-[[[(5,6-dihydro-5,5-dimethyl-8-phenyl)-2-naphthalenyl]carbonyl]amino]-2-hydroxybenzoic acid, 4-[[[(5,6-dihydro-5,5-dimethyl-8-phenyl)-2-naphthalenyl]carbonyl]amino]-2-nitrobenzoic acid, 4-[[[(5,6-dihydro-5,5-dimethyl-8-phenyl)-2-naphthalenyl]carbonyl]amino]-2-fluorobenzoic acid, 4-[[[(5,6-dihydro-5,5-dimethyl-8-phenyl-2-naphthalenyl)carbonyl]amino]-2-methoxybenzoic acid, 4-[[[(5,6-dihydro-5,5-dimethyl-8-(2-naphthalene))-2-naphthalenyl]carbonyl]amino]benzoic acid, 4-[[(5,6-dihydro-8-phenyl-2-naphthalenyl)carbonyl]aminobenzoic acid, 4-[[(5,6-dihydro-5,5-dimethyl-8-phenyl-2-naphthalenyl)carbonyl]amino]-3-fluorobenzoic acid, 4-[[(5,6-dihydro-5,5-dimethyl-8-phenyl-2-naphthalenyl)carbonyl]amino]-3-methylbenzoic acid, 4-[[(5,8,10,10a-tetrahydro-10,10-dimethyl-9-phenyl-2-anthracenyl)carbonyl]amino]benzoic acid, 4-[[(1,1-dimethyl-3-phenyl-1H1-indene-5-yl)amino]carbonyl]benzoic acid, 4-[(1,1-dimethyl-3-phenyl-1H-indene-5-yl-oxymethyl)benzoic acid, 4-[2-(1,1-dimethyl-3-phenyl-1H-indene-5-yl)vinyl]benzoic acid, and 4-[(1,1-dimethyl-3-phenyl-1H-indene-5-carbonyl)amino]benzoic acid] .

Examples of the compound further include: carotin, a salt, or derivatives thereof (carotenoids such as α-carotin, β-carotin, γ-carotin, lycopene, cryptoxanthin, lutein, zeaxanthin, isozeaxanthin, rhodoxanthin, capsanthin, and crocetin); lycopene, a salt, or derivatives thereof; thiamine, a salt, or derivatives thereof (thiamine hydrochloride, thiamine disulfide, bisbenthiamine, bisibuthiamine, thiamine monophosphate disulfide, benfothiamine, cycotiamine, octothiamine, dicethiamine, fursulthiamine, prosulthiamine, and astaxanthin thiamine phosphodiester); thiamine sulfate; riboflavin, a salt, or derivatives thereof (such as flavin adenine dinucleotide, flavin mononucleotide, riboflavin phosphodiester, 1-β-D-riboflanosyl nicotinamide pyrophosphate diester, and 1-β-D-riboflanosyl nicotinate); pyridoxine, a salt, or derivatives thereof (such as 3,4-pyridoxine dipalmitate, 3,4-pyridoxine dicaprylic acid, and disulfate pyridoxine); pyridoxal, a salt, or derivatives thereof; pyridoxamine, a salt, or derivatives thereof; cyanocobalamin, a salt, or derivatives thereof; cobalamins (such as methylcobalamin, adenosylcobalamin, hydroxocobalamin, and aquacobalamin); folic acid, a salt, or derivatives thereof; nicotinic acid, a salt, or derivatives thereof; pantothenic acid, a salt, or derivatives thereof; biotin, a salt, or derivatives thereof; choline, a salt, or derivatives thereof; and inositol, a salt, or derivatives thereof.

Examples of the compound further include: ergocalciferol, a salt thereof, or derivatives thereof; cholecalciferol, a salt therof, or derivatives thereof (such as 1α-hydroxy-22-[(1-hydroxy-1-methyl)-2-cyclopentene-4-yl-oxy]-23,24,25,26,27-pentanolcholecalciferol, [1R, 4R]-1α-hydroxy-22-[(1-hydroxy-1-methyl)-2-cyclopentene-4-yl-oxy]-23,24,25,26,27-pentanolcholecalciferol, [1R, 4S]-1α-hydroxy-22-[(1-hydroxy-1-methyl)-2-cyclopentene-4-yl-oxy]-23,24,25,26,27-pentanolcholecalciferol, [1S,4R]-la-hydroxy-22-[(1-hydroxy-1-methyl)-2-cyclopentene-4-yl-oxy]-23,24,25,26,27-pentanolcholecalciferol, and [1S, 4S]-1α-hydroxy-22-[(1-hydroxy-1-methyl)-2-cyclopentene-4-yl-oxy]-23,24,25,26,27-pentanolcholecalciferol); dihydrotachysterol, a salt thereof, or derivatives thereof; phytonadione, a salt thereof, or derivatives thereof; menaquinone, a salt thereof, or derivatives thereof; menadione, a salt thereof, or derivatives thereof; menadiol, a salt thereof, or derivatives thereof; carnitine, a salt thereof, or derivatives thereof; ferulic acid, a salt thereof, or derivatives thereof; γ-olizanol, a salt thereof, or derivatives thereof; orotic acid, a salt thereof, or derivatives thereof; rutin, a salt thereof, or derivatives thereof; eriocitrin, a salt thereof, or derivatives thereof; hesperidin, a salt thereof, or derivatives thereof; vitamin L complex: anthranilic acid, a salt thereof, or derivatives thereof; adenylthiomethylpentose, a salt thereof, or derivatives thereof; and methylmethionine sulphonium chloride or a derivative thereof.

Examples of the compound further include: valine, leucine, isoleucine, threonine, methionine, phenylalanine, tryptophan, lysine, glycine, alanine, asparagine, glutamine, serine, cysteine, cystine, tyrosine, proline, hydroxyproline, aspartic acid, glutamic acid, hydroxylysine, arginine, ornithine, and histidine and derivatives thereof (N-octyloxycarbonyl-β-alanyl-L-histidine, N-dodecyloxycarbonyl-β-alanyl-L-histidine, N-(12-amino-1-oxododecyl)-L-histidine, N-2-ethylhexyloxycarbonyl-β-alanyl-L-histidine hydrochloride, N-hexadecyloxycarbonyl-β-alanyl-L-histidine, N-octylaminocarbonyl-β-alanyl-L-histidine, N-dodecylaminocarbonyl-β-alanyl-L-histidine, N-dodecylsulfonyl-β-alanyl-L-histidine, N-dodecylamino-oxalyl-β-alanyl-L-histidine, and the like); sulfate, phosphate, nitrate, and citrate (sodium citrate or the like) thereof; amino acids such as an amino acid derivative of pyrrolidone carboxylate or the like; α-hydroxy acids such as glycolic acid, citric acid, malic acid, tartaric acid, lactic acid, and succinic acid; 2-hydroxycarboxylic acids (methyllactic acid, 2-hydroxybutanoic acid, 2-hydroxypentanoic acid, 2-hydroxyhexanoic acid, 2-hydroxyheptanoic acid, 2-hydroxyoctanoic acid, 2-hydroxynonanoic acid, 2-hydroxydecanoic acid, 2-hydroxyundecylic acid, α-hydroxylauric acid, α-hydroxymyristic acid, α-hydroxypalmitic acid, α-hydroxystearic acid, α-hydroxyarachidonic acid, cerebronic acid, α-hydroxynervonic acid, mandelic acid, benzilic acid, phenyllactic acid, atrolactic acid, 2-(4'-hydroxyphenyl)-2-hydroxyethanoic acid, 2-(4'-chlorophenyl)-2-hydroxyethanoic acid, 2-(3'-hydroxy-4'-methoxyphenyl)-2-hydroxyethanoic acid, 2-(4'-hydroxy-3'-methoxyphenyl)-2-hydroxyethanoic acid, 3-(2'-hydroxyphenyl)-2-hydroxypropanoic acid, 3-(4'-hydroxyphenyl)-2-hydroxypropanoic acid, 2-(3',4'-dihydroxyphenyl)-2-hydroxyethanoic acid, glyceric acid, erythronic acid, ribonic acid , arabinonic acid , xylonic acid, lyxonic acid, allonic acid, altronic acid, gluconic acid, mannonic acid, gulonic acid, idonic acid, galactonic acid, talonic acid, glucoheptonic acid, galactoheptonic acid, tartronic acid, mucic acid, and the like); polyhydroxy carboxylic acids or hydroxypolycarboxylic acids (gluconolactone, galactonolactone, glucuronolactone, galacturonolactone, glonolactone, ribonolactone, saccharic acid lactone, pantoyllactone, glucoheptonolactone, mannonolactone, galactoheptonolactone, and the like); 2-keto acids (glyoxalic acid, methyl 2-ketoethanoic acid, benzoylformic acid, methyl benzoylformic acid, ethyl benzoylformic acid, phenylpyruvic acid, methyl phenylpyruvic acid, ethyl phenylpyruvic acid, 2-ketobutanoic acid, 2-ketopentanoic acid, 2-ketohexanoic acid, 2-ketoheptanoic acid, 2-ketooctanoic acid, 2-ketododecanoic acid, 2-methyl ketooctanoic acid, and the like); quinic acid; isocitric acid; tropic acid; trethocanic acid; 3-chlorolactic acid; cerebronic acid; citramalic acid; agaricic acid; aroy ricinic acid; pantoic acid; lactobionic acid; hexuronic acid; photosensitive element 301; allantoin; glyceride pentadecanoic acid; estradiol; ethenylestradiol; antiarol or a glycoside thereof; lyoniresinol or a glycoside thereof; rhododendrol or a glycoside thereof; and platiphylonol or a glycoside thereof.

Examples of the compound further include: lactone compounds (D-glucurono-6,3-lactone, α-D-glucoheptonic-γ-lactone, α-glucuronolactone, α,β-glucooctonic-γ-lactone, L-glonic-γ-lactone, γ-D-galactonolactone, D-saccharic-1,4-lactone, D-saccharic-3,6-lactone, D-ribonic-γ-lactone, α-butyrolactone, γ-butyrolactone, α-octanoiclactone, γ-octanoiclactone, nonanoiclactone, γ-valerolactone, D-mannoic-α-lactone, D-xyloic-δ-lactone, D-arabinoic-δ-lactone, stearoyl-δ-gluconolactone, DL-pantolactone, palmitoyl-DL-pantolactone, and the like); 3-hydroxy-3,4-dicarboxy-1,4-butanolide or a derivative thereof; 6-benzylaminopurine or a derivative thereof; 1,4-diaza bicyclo octane; 2,5-dimethylfuran; 2-methylfuran; 2,5-diphenylfuran; 1,3-diphenyl isobenzofuran; rutin; tectorigenin 7-xylosylglucoside; captopril; alacepril; lisinopril; enalapril; delapril; benazepril; cilazapril; imidapril; quinapril; trandelapril; perindopril; temocapril; losartan; endothelin; a galactomannan-saccharide polymer; mucin; trimethyl glycine; proteoglycan; a lactobacillus extract; a lactobacillus medium extract; a lactobacillus fermented milk extract; a bifidobacterium extract; a bifidobacterium medium extract; a bifidobacterium-fermented milk extract; a placenta extract; a reishi mushroom extract; a spleen extract; a thymus extract; an yeast extract; an yeast medium extract; an yeast-fermented extract; a fungus extract; a fungus medium extract; a basidiomycete extract; a basidiomycete medium extract; a bacterial cell homogenized extract; a bacterial medium extract; and a cultured human dermis homogenized extract.

Examples of the compound further include: a benzochroman derivative; a nordihydroguaseretnic acid; butylhydroxytoluene(BHT); dibutylhydroxytoluene; butylhydroxyanisole(BHA); hydroxytyrosol; parahydroxyanisole; propyl gallate; sesamol; sesamolin; gossypol; maritimein; sulfuretin; xanthene-2,7-diols; caffeoylquinic acids; propolis; carotenoids (α-carotene, β-carotene, γ-carotene, lycopene, lutein, violaxanthin, spirilloxanthin, sphaeroiden, astaxanthin, and the like); phlorotannin; superoxide dismutase; catalase; glutathione peroxidase; bilirubin; quercetin; quercitrin; catechin; a catechin derivative; rutin or a derivative thereof; gallic acid, a salt thereof, or derivatives thereof; curcumine, a salt thereof, or derivatives thereof; transferrin; ceruloplasmin; coenzyme Q; uric acid; bilirubin; metallothionein; a stilbenegalloyl glycoside (3,5,4'-trihydroxystilbene4'-o-β-D-(6"galloyl)glucopyranoside, 3,5,4'-trihydroxystilbene4'-o-β-D-(2"galloyl)glucopyranoside, or the like); chlorogenic phospholipid ester; chlorogenic sphingosine ester and a derivative thereof; chlorogenic glycolipid ester; chlorogenic sugar ester; chlorogenic sterol ester; a thiazole derivative or a salt thereof (2-(3,4-diethoxyphenyl)-4-(3-carboxy-4-hydroxyphenyl)thiazole, 2-(3,4-diethoxyphenyl)-4-[3-carboxy-4-hydroxy-5-(2-methyl-2-propenyl)phenyl]thiazole, 2-(3,4-diethoxyphenyl)-4-(3-carboxy-4-hydroxy-5-methylphenyl)thiazole, or 2-(3,4-diethoxyphenyl)-4-(3-carboxy-5-methoxyphenyl)thiazole); hydroxymatairesinol; allohydroxymatairesinol; and a hydantoin derivative.

Examples of the compound further include: a phthalazine derivative such as 4-ethyl-1-(β-hydroxyethylamino)phthalazine, 4-N-propyl-1-(β-hydroxyethylamino)phthalazine, 4-N-butyl-1-(β-hydroxyethylamino)phthalazine, or 4-N-butyl-1-(β-hydroxypropylamino)phthalazine; a xanthin derivative such as caffeine, theophylline, theobromine, xanthine, aminophylline, cholintheophylline, diprophylline, proxyphylline or oxtriphylline; a benzophenone derivative such as 2-hydroxy-4-methoxybenzophenone, 2-hydroxy-4-methoxybenzophenone-5-sulfonic acid, sodium 2-hydroxy-4-methoxybenzophenone-5-sulfonic acid, dihydroxydimethoxybenzophenone, sodium dihydroxydimethoxybenzophenone sulfonic acid, 2,4-dihydroxybenzophenone, or tetrahydroxybenzophenone; and a 1,2-dihydroxy-4-(2-hydroxyethyl)benzene derivative such as 1-(2-(4-(3,4-dihydroxycinnamoyl)-3-rhamnosyl)glucosyl)ethyl-3-hydroxy-4-methoxybenzene, 1-(2-(6-(3,4-dihydroxycinnamoyl)-3-rhamnosyl)glucosyl)ethyl-3-hydroxy-4-methoxybenzene, 1-(2-(2-actyl-4-(3,4-dihydroxycinnamoyl)-3-rhamnosyl)glucosyl)ethyl-3,4-dihydroxybenzene, 1-(2-(2-actyl-6-(3,4-dihydroxycinnamoyl)-3-rhamnosyl)glucosyl)ethyl-3,4-dihydroxybenzene, 1-(2-(4-(3,4-dihydroxycinnamoyl)-3-rhamnosyl)glucosyl)ethyl-3,4-dihydroxybenzene, 1-(2-(6-(4-hydroxy-3-methoxycinnamoyl)-3-rhamnosyl)glucosyl)ethyl-3,4-dihydroxybenzene, 1-(2-(4-(3,4-dihydroxycinnamoyl)-3-rhamnosyl)glucosyl)-1-methoxyethyl-3,4-dihydroxybenzene, 1-(2-(4-(3,4-dihydroxycinnamoyl)-3-rhamnosyl)glucosyl)-1-hydroxyethyl-3,4-dihydroxybenzene, 1-(2-(6-(3,4-dihydroxycinnamoyl)-3-xylosyl)glucosyl)ethyl-3,4-dihydroxybenzene, 1-(2-(3-allosyl-6-(3-hydroxy-4-methoxycinnamoyl))glucosyl)ethyl-3,4-dihydroxybenzene, 1-(2-(3-glucosyl-6-(3-hydroxy-4-methoxycinnamoyl))glucosyl)ethyl-3-hydroxy-4-methoxybenzene, 1-(2-(3-allosyl-6-(3-hydroxy-4-methoxycinnamoyl)glucosyl)ethyl-3-hydroxy-4-methoxybenzene, 1-(2-(4-(3,4-dihydroxycinnamoyl)glucosyl)ethyl)-3,4-dihydroxybenzene, 1-(2-(3-(3,4-dihydroxycinnamoyl)glucosyl)ethyl)-3,4-dihydroxybenzene, 1-(2-(2-(3,4-dihydroxycinnamoyl)glucosyl)ethyl)-3,4-dihydroxybenzene, or 1-(2-(6-(3,4-dihydroxycinnamoyl)glucosyl)ethyl)-3,4-dihydroxybenzene.

Examples of the compound further include: a p-aminobenzoic acid derivative such as p-aminobenzoic acid, ethyl p-aminobenzoic acid, glyceryl p-aminobenzoic acid, amyl p-dimethylaminobenzoic acid, or octyl p-dimethylaminobenzoic acid; a methoxycinnamic acid derivative such as ethyl p-methoxycinnamic acid, isopropyl p-methoxycinnamic acid, octyl p-methoxycinnamic acid, p-methoxycinnamic acid-2-ethoxyethyl, sodium p-methoxycinnamic acid, potassium p-methoxycinnamic acid, or di-p-methoxycinnamic acid-mono-2-ethylhxananic acid glyceryl; an anthranilic acid derivative such as methyl anthranilic acid; an urocanic acid derivative such as urocanic acid or ethyl urocanic acid; a coumarin derivative; an amino acid-based compound; a benzotriazole derivative; a tetrazole derivative; an imidazoline derivative; a pyrimidine derivative; a dioxane derivative; a camphor derivative; a furan derivative; a pyrone derivative; a nucleic acid derivative; an allantoin derivative; a nicotinic acid derivative; a pyridoxine derivative; a pyridoxal derivative; a pyridoxamine derivative; amentoflavone; neosakuranin; 6-dehydrokawain; a triazine derivative such as 4,4'4"-(1,3,5-triazine-2,4,6-triyltriimino)trisbenzoic acid tris-2-ethylhexyl or the like; a phosphatidylchromanol derivative such as 1,2-dilaurorylglycero-3-phospho-2'-hydroxymethyl-2',5',7',8'-tetramethyl-6'-hydroxychroman, 1,2-dimyristoylglycero-3-phospho-2'-hydroxymethyl-2',5',7',8'-tetramethyl-6'-hydroxychroman, 1,2-dipalmitoylglycero-3-phospho-2'-hydroxymethyl-2',5',7',8'-tetramethyl-6'-hydroxychroman, 1,2-distearoylglycero-3-phospho-2'-hydroxymethyl-2',5',7',8'-tetramethyl-6'-hydroxychroman, 1,2-diarachidonylglycero-3-phospho-2'-hydroxymethyl-2',5',7',8'-tetramethyl-6'-hydroxychroman, 1-myristoyl-2-palmitoylglycero-3-phospho-2'-hydroxymethyl-2',5',7',8'-tetramethyl-6'-hydroxychroman, 1-myristoyl-2-stearoylglycero-3-phospho-2'-hydroxymethyl-2',5',7',8'-tetramethyl-6'-hydroxychroman, 1-palmitoyl-2-myristoylglycero-3-phospho-2'-hydroxymethyl-2',5',7',8'-tetramethyl-6'-hydroxychroman, 1-palmitoyl-2-stearoylglycero-3-phospho-2'-hydroxymethyl-2',5',7',8'-tetramethyl-6'-hydroxychroman, 1-stearoyl-2-myristoylglycero-3-phospho-2'-hydroxymethyl-2',5',7',8'-tetramethyl-6'-hydroxychroman, or 1-stearoyl-2-palmitoylglycero-3-phospho-2'-hydroxymethyl-2',5',7',8'-tetramethyl-6'-hydroxychroman; an L-carnosine zinc complex; umbelliferone; aesculin; benzyl cinnamate; cinoxate; oxybenzone; dioxybenzone; octabenzone; sulisobenzone; benzoresorcinol; arbutin; guaiazulen; shikonin; baicalin; baicalein; berberine; neoheliopan; chlorophylls; xanthophylls; escarole; zinc oxide; talc; and kaolin.

Examples of the compound further include: zinc sulfate; zinc oxide; calamine; zinc p-phenolsulfonic acid; potassium aluminium sulfate; resorcin; ferric chloride; tannins such as tannic acid, hamamelitannin, acertannin, gallotannin including tetragalloyl glucose, pentagalloyl glucose, hexagalloyl glucose, heptagalloyl glucose, octagalloyl glucose, nonagalloyl glucose, decagalloyl glucose, undecagalloyl glucose, or dodecagalloyl glucose, and ellagitannins including tellima glandin I, tellima glandin II, casuaricutin, pedunclagin, geranin, isotarcebin, granatin A, granatin B, chebulic acid, chebulagic acid, casualinin, nufalin, procyanidin B-2, thiasinensin A, and thiasinensin B; a quinolinon derivative; a dibenzoxepin derivative; thiotropocin; a phthalimide derivative; a flurbiprofen; felbinac; bufexamac; suprofen; a 1,4-diphenylpropylpyperadine derivative; a calxine compound; a chromanol glycoside such as 2-(α-D-glucopyranosyl)methyl-2,5,7,8-tetramethylchroman-6-ol); ichthammol; indomethacin; kaolin; diphenhydramine hydrochloride; d-camphor; DL-camphor; salicylate; sodium salicylate; methyl salicylate; acetyl salicylate; hydrocortisone; guaiazulen; chamazulen; chlorpheniramine maeate; diphenhydramine hydrochloride; clemastine fumarate; cyproheptadine hydrochloride; promethazine hydrochloride; a pyperadine derivative; and an α-D-phenylglucoside derivative.

Examples of the compound further include: glycyrrhizic acid, a salt thereof, and derivatives thereof such as α-glycyrrhizic acid, β-glycyrrhizic acid, α-glycyrrhizic acid methyl ester, β-glycyrrhizic acid methyl ester, α-glycyrrhizic acid trisodium, α-glycyrrhizic acid monopotassium, α-glycyrrhizic acid dipotassium, α-glycyrrhizic acid monoammonium, β-glycyrrhizic acid trisodium, β-glycyrrhizic acid monopotassium, β-glycyrrhizic acid dipotassium, and β-glycyrrhizic acid monoammonium; glycyrrhetic acid, a salt thereof, or derivatives thereof such as α-glycyrrhetic acid, β-glycyrrhetic acid, stearyl α-glycyrrhetic acid, stearyl β-glycyrrhetic acid, α-glycyrrhetic acid pyridoxine, β-glycyrrhetic acid pyridoxine, α-glycyrrhetic acid glycerin, β-glycyrrhetic acid glycerin, and 3-succinyloxyglycyrrhetic acid disodium; pantenol, a salt thereof, or derivatives thereof such as mefenamic acid, phenylbutazone, ibuprofen, ketoprofen, allantoin, calcium pantothenate, and pantothenyl ethyl ether; ε-aminocaproic acid; diclofenac sodium; tranexamic acid or a derivative thereof such as trans-4-benzyl oxycarbonylaminomethyl cyclohexanecarboxylic acid, trans-4-p-nitrobenzyl oxycarbonylaminomethyl cyclohexanecarboxylic acid, trans-4-p-chlorobenzyl oxycarbonylaminomethyl cyclohexanecarboxylic acid, trans-4-p-bromobenzyl oxycarbonylaminomethyl cyclohexanecarboxylic acid, trans-4-m-chlorobenzyloxy carbonylaminomethyl cyclohexanecarboxylic acid, trans-4-p-methoxybenzyloxy carbonylaminomethyl cyclohexanecarboxylic acid, trans-4-p-methylbenzyloxy carbonylaminomethyl cyclohexanecarboxylic acid, trans-4-benzyloxy carbonylaminomethyl cyclohexanecarboxylic acid sodium, trans-4-p-nitrobenzyloxy carbonylaminomethyl cyclohexanecarboxylic acid ammonium, trans-4-p-chlorobenzyloxy carbonylaminomethyl cyclohexanecarboxylic acid potassium, trans-4-p-bromobenzyloxy carbonylaminomethyl cyclohexanecarboxylic acid potassium, trans-4-m-chlorobenzyloxy carbonylaminomethyl cyclohexanecarboxylic acid calcium, trans-4-p-methoxybenzyloxy carbonylaminomethyl cyclohexanecarboxylic acid magnesium, trans-4-p-methylbenzyloxy carbonylaminomethyl cyclohexanecarboxylic acid monoethanolamine, trans-4-t-butyloxy carbonylaminomethyl cyclohexanecarboxylic acid, trans-4-(2-phenylisopropyloxy carbonylaminomethyl)cyclohexanecarboxylic acid, trans-4-(p-biphenyl isopropyloxycarbonylaminomethyl)cyclohxanecarboxylic acid, trans-4-(3,5-dimethoxyphenyl isopropyloxycarbonylaminomethyl)cyclohexanecarboxylic acid, trans-4-(p-methylphenyl isopropyloxycarbonylaminomethyl)cyclohexanecarboxylic acid, trans-4-t-butyloxycarbonylaminomethyl cyclohexanecarboxylic acid sodium, trans-4-(2-phenylisopropyloxycarbonylaminomethyl)cyclohexanecarboxylic acid potassium, trans-4-(p-biphenylisopropyloxycarbonylaminomethyl)cyclohexanecarboxylic acid calcium, trans-4-(3,5-dimethoxyphenylisopropyloxycarbonylaminomethyl)cyclohexanecarboxylic acid magnesium, trans-4-(p-methylphenylisopropyloxycarbonylaminomethyl)cyclohexanecarboxylic acid monoethanolamine, trans-4-(9-fluorenylmethyloxycarbonylaminomethyl)cyclohexanecarboxylic acid, trans-4-methylsulfonyl ethyloxycarbonylaminomethyl cyclohexanecarboxylic acid, trans-4-(9-fluorenylmethyloxycarbonylaminomethyl)cyclohexanecarboxylic acid sodium, trans-4-methylsufonyl ethyloxycarbonylaminomethyl cyclohexanecarboxylic acid potassium, trans-4-(pyridine-4'-methyloxycarbonylaminomethyl)cyclohexanecarboxylic acid, trans-4-(2,2,2-trichloroethyloxycarbonylaminomethyl)cyclohexanecarboxylic acid, trans-4-(2-trimethylsilylethoxycarbonylaminomethyl)cyclohexanecarboxylic acid potassium, trans-4-(pyridine-4'-methyloxycarbonylaminomethyl)cyclohexanecarboxylic acid sodium, trans-4-(2,2,2-trichloroethyloxycarbonylaminomethyl)cyclohexanecarboxylic acid potassium, or trans-4-(2-trimethylsilylethoxycarbonylaminomethyl)cyclohexanecarboxylic acid calcium; and sulfatide.

Examples of the compound further include: astaxanthin fatty acid diesters (astaxanthin dilauric ester, astaxanthin dimyristic ester, astaxanthin dipentadecanoate, astaxanthin dipalmitate, astaxanthin dipalmitolenate, astaxanthin diheptadecanorate, astaxanthin dielaidate, astaxanthin direcinolate, astaxanthin petroselinoate, astaxanthin vaccenoate, astaxanthin eleostearate, astaxanthin punicin ester, astaxanthin licanate, astaxanthin parinate, astaxanthin gadol acid ester, astaxanthin 5-eicosenoic ester, astaxanthin 5-docosenoic ester, astaxanthin cetolate, astaxanthin erucinate, astaxanthin 5,13-docosadiene ester, astaxanthin ceracol acid ester, astaxanthin decenoate, astaxanthin stering acid ester, astaxanthin dodecenoate, astaxanthin dioleate, astaxanthin distearate, astaxanthin dieicosapentaenoate, astaxanthin didocosahexaenoate, astaxanthin dilinoleate, astaxanthin dilinolenate, and astaxanthin diarachidonate, etc.); astaxanthin diglycerophosphates (astaxanthin diglycerophosphate, astaxanthin glycerophosphoric acid palmitic acid, astaxanthin glycerophosphatidylcholine palmitic acid, astaxanthin glycerophosphatidylcholine DHA, astaxanthin glycerophosphatidylinositol palmitic acid, astaxanthin glycerophosphatidylinositol DHA, astaxanthin glycerophosphatidylinositol linoleic acid, and astaxanthin glycerophosphatidylcholine linoleic acid, etc.); steviol glycosides; benzimidazole derivatives (1-(2-ethoxyethyl)-2-[1-(2-(4-(1-(4,4-dimethyl-2-oxazolin-2-yl)-1-methylethyl)phenyl)ethyl)pyperidin-4-yl]-1H-benzimidazole, 2-[4-(2-(4-(1-(2-ethoxyethyl)benzimidazol-2-yl)pyperidin-1-yl)ethyl)phenyl]-2-methyl propanoic acid, ethyl 2-[4-(2-(4-(1(2-ethoxyethyl)benzimidazol-2-yl)pyperidin-1-yl)ethyl)phenyl]-2-methyl propanoate, 1-(2-ethoxyethyl)-2-[1-(2-(4-(1,1-dimethyl-2-hydroxyethyl)phenyl)ethyl)pyperidin-4-yl]-1H-benzimidazol, 1-(2-hydroxyethyl)-2-[1-(2-(4-(1-(4,4-dimethyl-oxazolin-2-yl)-1-methylethyl)phenyl)ethyl)piperidin-4-yl]-1H-benzimidazol, and 2-[4-(2-(4-(1-(2-hydroxyethyl)benzimidazol-2-yl)piperidin-1-yl)ethyl)phenyl]-2-methyl propanoic acid, etc.); alanine derivatives or salts thereof (N-{3-isopentyl-4-methyl-2-[4-(3-trifluoromethylbenzamide)benzoylimino]-3H-thiazoline-5-carbonyl}-L-alanine, N-{3-isopentyl-4-methyl-2-[4-(3-trifluoromethylbenzamide)benzoylimino]-3H-thiazoline-5-carbonyl}-2-methylalanine, and N-{3-isopentyl-4-methyl-2-[4-(3-trifluoromethylbenzamide)benzoylimino]-3H-thiazoline-5-carbonyl}-N-methyl-L-alanine, etc.); thiazoline derivatives or salts thereof (3-isopentyl-4-methyl-2-[4-(3-trifluoromethylbenzamide)benzoylimino]-3H-thiazoline-5-carboxylic acid and 4-isobutyl-3-methyl-2-[4-(3-trifluoromethylbenzamide)benzoylimino]-3H-thiazoline-5-carboxylic acid, etc.); chlorpheniramine maleate, diphenhydramine hydrochloride, diphenylpyrarine hydrochloride, carbinoxamine maleate, hydrocortisone butyrate, sulfated acidic mucopolysaccharides or salts thereof, and sulfated dextran or its salt.

Examples of the compound further include a chroman derivative, pyridoxine or its salt and derivatives thereof, pyridoxal or its salt and derivatives thereof, pyridoxamine or its salt and derivatives thereof, sulfur, calcium gluconate, chlorhexidine gluconate, sulfamine, mercurochrome, lactoferrin or a hydrolysate thereof, alkyldiaminoethylglycine chloride solution, triclosan, sodium hypochlorite, chloramine T, bleaching powder, iodine compounds, iodoform, 1-alkyl carbapenem compounds, N-substituted azepan derivatives or salts thereof, sorbic acid or its salt, propionic acid or its salt, salicylic acid or its salt, dehydroacetic acid, parahydroxybenzoates, 2-keto-3-deoxyoctonate fatty acid ester, deoxyinositol derivatives (quercitol etc.), inositol unsaturated derivatives (conduritol etc.), inositol dimethyl ether, dodecylglycerin ether, methyl inositol, inositol methyl ether, inosamine, deoxyinosadiamine, shikimic acid, quinic acid, undecylenic acid, thiamine laurylsulfate, thiamine laurylnitrate, phenol, cresol, p-chlorophenol, p-chloro-m-xylenol, p-chloro-m-cresol, thymol, phenethyl alcohol, o-phenylphenol, irgasan CH3565, halocarban, hexachlorophene, chlorohexidine, ethanol, methanol, isopropyl alcohol, benzyl alcohol, ethylene glycol, propylene glycol, dipropylene glycol, 2-phenoxyethanol, 1,3-butanediol, 3-methyl 1,3-butanediol, 1,2-pentanediol, zincpyridione, chlorobutanol, isopropyl methylphenol, elemol, vetiverol, patchouli alcohol, formaldehyde, hexamine, brilliant green, malachite green, crystal violet, dermal, photopigment 101, photopigment 201, photopigment 401, and an N-long-chain acyl basic amino acid derivative and an acid addition salt thereof.

Examples of the compound further include cepharanthine, capronium chloride, eugenol derivatives (acetyl eugenol, methyl eugenol, methyl isoeugenol, ethyl eugenol, ethyl isoeugenol, and eugenol salicylate, etc.), minoxidil, capsicum tincture, vanilamide nonilate, cantharis tincture, ginger tincture, mint oil, L-menthol, camphor, benzyl nicotinate, cinnarizine, tolazoline, acetylcholine, verapamil, ichthammol, α-borneol, cyclandelate, nonyl vanillylamide, capsaicin, zingerone, estrogen (estrone, estradiol, and ethynyl estradiol, etc.), isoflavone, oxendolone, 4',5,7-trihydroxy-8-prenylflavanone, 4',5,7-trihydroxy-8-prenylflavone, 3,3',4',5,7-pentahydroxy-8-prenylflavone, nicorandil, cyclosporinic acid, carbostyril derivatives or salts thereof, dicarboxylic acids (glutaric acid, adipic acid, pimelic acid, suberic acid, azelaic acid, sebacic acid, 1,9-nonamethylene dicarboxylic acid, and 1,10-decamethylene dicarboxylic acid), rosmarinic acid, urusolic acid, oleanolic acid, a hydroxamic acid derivative, and an aesculetin derivative.

Examples of the compound further include anthocyanidins, nordihydroguaiaretic acid, 20-carboxy-16-hydroxy-21-nor-5 α-7,9(11)-lanostadiene-3,24-dione, ubiquinone, plastaquinone, juglone, shikonin, quinizarin, alizarin, abietine, levopimarl, betulin, α-amylin, catechin compounds (catechin, epigallocatechin, epigallocatechin gallate, epicatechin, epicatechin gallate, and catechin rhamnopyranoside catechin, etc.), diisopropyl fluorophosphate, (2R,6R)-2,6-dihydroxy-4-(tert-butyldimethylsilyloxy)-(3Z)-heptene-1,7-dimethyl dicarboxylate, N,N'-bis[(1S,2R)-2-hydroxyindan-1-yl]-(2R,6R)-2,6-dibenzyloxy-4-hydroxyheptane dicarboxylic-1,7-diamide, (2R,6R)-2,6-dihyroxy-4-(tert-butyldimethylsilyloxy)heptane-1,7-dimethyl dicarboxylic acid, (2R,6R)-2,6-dibenzyloxy-4-(tert-butyldimethylsilyloxy)heptane-1,7-dimethyl dicarboxylic acid, (2R,6R)-2,6-dibenzyloxy-4-(tert-butyldimethylsilyloxy)heptane-1,7-dicarboxylic acid, N,N'-bis[(1S,2R)-2-hydroxyindan-1-yl]-(2R,6R)-2,6-dibenzyloxy-4-(tert-butyldimethylsilyl)oxypentane-1,7-dicarboxylic diamide, N,N'-bis[(1S,2R)-2-hydroxyindan-1-yl]-(2R,6R)-2,6-dibenzyloxy-4-hydroxyheptane-1,7-dicarboxylic diamide, N,N'-bis[(1S,2R)-2-hydroxyindan-1-yl]-(2R,6R)-2,4,6-trihydroxyheptane-1,7-dicarboxylic diamide, N,N'-bis[(1S,2R)-2-hydroxyindan-1-yl]-(2S,6S)-2,6-dibenzyloxy-4-hydroxyheptane-1,7-dicarboxylic diamide, (2S,6S)-2,6-dihydroxy-4-(tert-butyldimethylsilyloxy)-(32)-heptene-1,7-dimethyl dicarboxylic acid, (2S,6S)-2,6-dihydroxy-4-(tert-butyldimethylsilyloxy)heptane-1,7-dimethyl dicarboxylic acid, (2S,6S)-2,6-dibenzyloxy-4-(tert-butyldimethylsilyloxy)heptane-1,7-dimethyl dicarboxylic acid, (2S,6S)-2,6-dibenzyloxy-4-(tert-butyldimethylsilyloxy)heptane-1,7-dicarboxylic acid, N,N'-bis[(1S,2R)-2-hydroxyindan-1-yl]-(2S,6S)-2,6-dibenzyloxy-4-(tert-butyldimethylsilyl)oxyheptane-1,7-dicarboxylic diamide, N,N'-bis[(1S,2R)-2-hydroxyindan-1-yl]-(2R,6R)-2,6-dibenzyloxy-4-hydroxyheptane-1,7-dicarboxylic diamide, 3-[[4-(4-fluorophenoxy)-benzenesulfonyl]-(1-hydroxycarbamoylcyclopentyl)-amino]-propionic acid, 4-[4-(4-fluorophenoxy)-benzenesulfonylamino]-tetrahydropyran-4-carboxylic hydroxyamide, 4-[4(4-chlorophenoxy)-benzenesulfonylmethyl]-tetrahydropyran-4-carboxylic hydroxyamide, 3-[[4-(4-fluorophenoxy)-benzenesulfonyl]-(1-hydroxycarbamoylcyclobutyl)-amino]-propionic acid, 4-(4'-chlorobiphenyl-4-yl)-2-[2-(1,3-dioxo-1,3-dihydroisoindol-2-yl)-ethyl]-4-oxobutyric acid, {1-[4-(4-fluorobenzyloxy)-benzenesulfonyl]-2-hydroxycarbamoylpiperidin-3-yl}carbamic acid isopropyl ester, 2-[4-(4-fluorophenoxy)-benzenesulfonylamino]-N-hydroxy-2-methylpropionamide, 3-[4-(4-fluorophenoxy)-benzenesulfonyl]-2,N-dihyroxypropionamide, 3-(4-phenoxybenzenesulfonyl)-7-oxabicyclo[2.2.1]heptane-2-carboxylic hydroxyamide, (4-benzylbenzyl)-[2-(2,2-dimethyl-1-methylcarbamoylpropylcarbamoyl)-4-(4'-fluorobiphenyl-4-yl)-butyl]-phosphinate, 2-amino-3-[4-(4-fluorophenoxy)-benzenesulfonyl]-N-hydroxypropionamide, and N-hydroxy-2-(4-phenylpiperidine-1-sulfonyl)-acetamide.

Examples of the compound further include an ethanolamine derivative, pentoxyfilline, a serine derivative, geraniol, crocetin, sodium benzoate, methyl 4-(2-ethylhexyloxy)-2-hydroxybenzoate, methyl 2-hydroxy-4-(3,5,5-trimethylhexyloxy)benzoate, methyl 4-cyclohexylmethoxy-2-hydroxybenzoate, methyl 4-(2-cyclohexylethoxy)-2-hydroxybenzoate, methyl 4-(3,7-dimethyl-6-octenyloxy)-2-hydroxybenzoate, ethyl 3-(2-ethylhexyloxy)-5-hydroxybenzoate, methyl 5-(2-ethylhexyloxy)-2-hydroxybenzoate, 2-hydroxy-5-(3,5,5-trimethylhexyloxy)-2-methyl benzoate, methyl 5-(2-cyclohexylethoxy)-2-hydroxybenzoate, methyl 4-N-hexyloxy-2-hydroxybenzoate, methyl 2-hydroxy-4-N-octyloxybenzoate, methyl 4-N-decyloxy-2-hydroxybenzoate, methyl 5-N-hexyloxy-2-hydroxybenzoate, 4-(2-ethylhexyloxy)-2-hydroxybenzoate, 2-hydroxy-4-(2,5,5-trimethylhexyloxy)benzoate, 4-cyclohexylmethoxy-2-hydroxybenzoate, 4-(2-cyclohexylethoxy)-2-hydroxybenzoate, 4-(3,7-dimethyl-6-octenyloxy)-2-hydroxybenzoate, 3-(2-ethylhexyloxy)-5-hydroxybenzoate, 5-(2-ethylhexyloxy)-2-hydroxybenzoate, 5-(2-ethylhexyloxy)-2-hydroxybenzoate, 2-hydroxy-5-(3,5,5-trimethylhexyloxy)benzoate, 5-(2-cyclohexylethoxy)-2-hydroxybenzoate, 4-N-hexyloxy-2-hydroxybenzoate, 5-N-hexyloxy-2-hydroxybenzoate, 2-hydroxy-4-N-octyloxybenzoate, 4-N-decyloxy-2-hydroxybenzoate, N-(2-hydroxyethyl)-4-(2-ethylhexyloxy)-2-hydroxybenzamide, N-ethyl-4-(2-ethylhexyloxy)-2-hydroxybenzamide, 2-acetoxy-4-cyclohexylmethoxy benzoate, sodium 4-(2-ethylhexyloxy)-2-hydroxybenzoate, methyl 4-{(2E)-3,7-dimethyl-2,6-octadienyloxy}-2-hydroxybenzoate, ethyl 4-{(2E)-3,7-dimethyl-2,6-octadienyloxy}-2-hydroxybenzoate, ethyl 5-{(2E)-3,7-dimethyl-2,6-octadienyloxy}-2-hydroxybenzoate, ethyl 3-{(2E)-3,7-dimethyl-2,6-octadienyloxy}-2-hydroxybenzoate, ethyl 3-{(2E)-3,7-dimethyl-2,6-octadienyloxy}-5-hydroxybenzoate, ethyl 4-{(2E)-3,7-dimethyl-2,6-octadienyloxy}-3-methoxybenzoate, methyl 4-{(2E,6E)-3,7,11-trimethyl-2,6,10-dodecatrienyloxy}-2-hydroxybenzoate, 4-{(2E)-3,7-dimethyl-2,6-octadienyloxy}-2-hydroxybenzoic(2E)-3,7-dimethyl-2,6-octadienyl, 4-{(2E)-3,7-dimethyl-2,6-octadienyloxy}-2-hydroxybenzoate, 5-{(2E)-3,7-dimethyl-2,6-octadienyloxy}-2-hydroxybenzoate, 3-{(2E)-3,7-dimethyl-2,6-octadienyloxy}-2-hydroxybenzoate, 3-{(2E)-3,7-dimethyl-2,6-octadienyloxy}-5-hydroxybenzoate, 2-hydroxy-4-{(2E,6E)-3,7,11-trimethyl-2,6,10-dodecatrienyloxy}benzoate, 4-{(2E)-3,7-dimethyl-2,6-octadienyloxy}-3-methoxybenzoate, 2-acetoxy-4-{(2E)-3,7-dimethyl-2,6-octadienyloxy}benzoate, N-(2-hydroxyethy)-4-{(2E)-3,7-dimethyl-2,6-octadienyloxy}-2-hydroxybenzamide, 4-{(2E)-3,7-dimethyl-2,6-octadienylamino}-2-hydroxybenzoate, dimethyl-2,6-octadienyloxy}benzoate, N-(2-hydroxyethyl)-4-{(2E)-3,7-dimethyl-2,6-octadienyloxy}-2-hydroxybenzamide, 4-{(2E)-3,7-dimethyl-2,6-octadienylamino}-2-hydroxybenzoate, 3-dodecyloxybenzoate, 3-(12-hydroxydodecyloxy)benzoate, 4-dodecyloxybenzoate, 4-(12-hydroxydodecyloxy)benzoate, 3-(12-hydroxyoctadecyloxy)benzoate, 4-(12-hydroxyoctadecyloxy)benzoate,3-(11-hydroxyundecyloxy)benzoate, 4-(11-hydroxyundecyloxy)benzoate, 3-{(2E)-3,7-dimethyl-2,6-octadienyloxy}benzoate, 4-{(2E)-3,7-dimethyl-2,6-octadienyloxy}benzoate, 3-{(2E,6E)-3,7,11-trimethyl-2,6,10-dodecatrienyloxy}benzoate, 4-{(2E,6E)-3,7,11-trimethyl-2,6,10-dodecatrienyloxy}benzoate, 4-[3,7-dimethyl-9-(2,6,6-trimethyl-1-cyclohexen-1-yl)-2,4,6,8-nonatetraenyloxy]benzoate, 4-[3,4-dihydro-2,5,7,8-tetramethyl-2-(4,8,12-trimethyltridecyl)-2H-1-benzopyran-6-oxy]benzoate, methyl parahydroxybenzoate, propyl parahydroxybenzoate, 4-{(2E,6E)-3,7,11-trimethyl-2,6,10-dodecatrienyloxy}benzamide, 4-{(2E)-3,7-dimethyl-2,6-octadienyloxy}benzamide, 4-(2-methyl-2-butenyloxy)benzamide, 4-(2-ethylhexyloxy)benzamide, 4-dodecyloxybenzamide, 4-(12-hydroxydodecyloxy)benzamide, 4-(12-hydroxyoctadecyloxy)benzamide, 4-(11-hydroxyundecyloxy)benzamide, 4-(10-hydroxydecyloxy)benzamide, 4-isostearyloxybenzamide, N-(2-hydroxyethyl)-4-[(2E,6E)-3,7,11-trimetyl-2,6,10-dodecatrienyloxy]benzamide, N,N-dimethyl-4-[(2E,6E)-3,7,11-trimetyl-2,6,10-dodecatrienyloxy]benzamide, N,N-di[(2E)-3,7-dimethyl-2,6-octadienyl]-4-aminobenzamide, 4-[N'-methoxycarbonyl-N-[(2E)-3,7-dimethyl-2,6-octadienylamino]benzamide, 4-[N-acetyl-N-[(2E)-3,7-dimethyl-2,6-octadienyl]aminobenzamide, N-(2-hydroxyethyl)-4-[(2E,6E)-3,7,11-trimethyl-2,6,10-dodecatrienyloxy]-2-hydroxybenzamide, and N,N-diethyl-4-[(2E,6E)-3,7,11-trimethyl-2,6,10-dodecatrienyloxy]-2-hydroxybenzamide.

Examples of the compound further include anacardic acid or its derivative (6-pentadecatrienyl salicylic methylether, 6-pentadecatrienyl salicylic ethylether, 6-pentadecatrienyl salicylic propylether, 6-pentadecatrienyl salicylic butylether, 6-pentadecatrienyl salicyl alcohol methyl ether, 6-pentadecatrienyl salicyl alcohol ethyl ether, 6-pentadecatrienyl salicylalcohol propyl ether, 6-pentadecatrienyl salicyl alcohol butyl ether, 6-pentadecatrienyl salicyl aldehyde methyl ether, 6-pentadecatrienyl salicylaldehyde ethyl ether, 6-pentadecatrienyl salicylaldehyde propyl ether, 6-pentadecatrienyl salicylaldehyde butyl ether, 2-methylether-6-pentadecatrienyl cinnamate, 2-ethylether-6-pentadecatrienyl cinnamate, 2-propylether-6-pentadecatrienyl cinnamate, 2-butylether-6-pentadecatrienyl cinnamate, 2-methylether-6-pentadecatrienyl cinnamic alcohol, 2-ethylether-6-pentadecatrienyl cinnamic alcohol, 2-propylether-6-pentadecatrienyl cinnamic alcohol, and 2-butylether-6-pentadecatrienyl, etc.), polyisoprenylated benzophenone derivatives (garcinol, isogarcinol, xanthochymol, isoxanthoechymol, and guttiferone, etc.), a stilbene derivative or its salt, morgin or its salt and derivatives thereof, and N-acetylglucosamine.

Examples of the compound further include phospholipids (phosphatidylethanol, phosphatidylcholine, phosphatidyltriethanolamine, phosphatidylserine, phosphatidic acid, phosphatidylglycerol, phosphatidylinositol, diacylphosphatidylcholine, diacylphosphatidylethanolamine, diacylphosphatidylinositol, diacylphosphatidylserine, 1-cysteinylphosphatidic acid, 2-cysteinylphosphatidic acid, 1-glutathionylphosphatidic acid, 2-glutathionylphosphatidic acid, 1-azelaoylphosphatidic acid, 2-azelaoylphosphatidic acid, 1-hydroxyacylphosphatidic acid, 2-hydroxyacylphosphatidic acid, ceramide, glucosylceramide, galactosylceramide, cerebroside, phosphatidylglucosylacylglycerol, N-oleoylsphingosine, N-(12-hydroxyoctadecanoyl)sphingosine, N-(16-hydroxyhexadecanoyl)sphingosine, N-salicyloyl phytosphingosine, sphingomyelin, soybean lecithin, and egg-yolk lecithin, etc.), sterins (cholesterol, dihydrocholesterol, cholesteryl stearate, hydroxycholesteryl stearate, macadamia nut oil cholesteryl fatty acid, β-sitosterol, stigmasterol, campesterol, ergosterol, 5-dihydroergosterol, phytosterol, 25-hydroxycholesterol, 26-hydroxycholesterol, 19-hydroxycholesterol, 22-ketocholesteroloxime, 6-ketocholesterol, and 7-ketocholesterol or derivatives thereof, etc.), N-acetylneuraminic acid (sialic acid), N-glucosolneuraminic acid, gangliosides (galaose, ganglitriaose, ganglitetraose, globotetraose, neolactotetraose, neolactohexaose, and neolactooctaose, etc.), oligosulfated hyaluronic acid, a hydroxytamoxifen compound, glyceroglycolipids, pentoxyferin, 3-deazaadenosine, a carboxyamide derivative, inositolpolyamines, sialyl acid, a triterpenic acid derivative, lactose, a lactosamine derivative, a sulfated chitin derivative, and albumin.

Examples of the compound further include a tropolone and its derivative, guanidine, ethanolamine, triethanolamine, aminoguanidine, flavanones (naringin, naringenin, naringin, naringenin, liquiritin, liquiritigenin, digallic acid, luteic acid, ellagic acid, chlorogenic acid, glucogallin, tetralin, hamamelitannin, tannin gallate, tannic acid, geraniin, gallic acid, galloyl gallic acid, ellagitannin, hexagalloylglucose, heptagalloylglucose, tetragalloylglucose, trigalloylglucose, pentagalloylglucose, digalloylquininic acid, and trigalloylquininic acid), a 2-hydroxyphenylalkylamine derivative or its salt, phenylpropen derivatives (3-[2,3-bis(methoxymethoxy)phenyl]propenoate, 7-(4-hydroxy-3-methoxyphenyl)hepta-2,4,6-trienoic acid, 3-(3,5-dimethoxy-4-hydroxyphenyl)propenohydrazide, and N'-isopropylidene-3-(2-methoxyphenyl)propenohydrazide, etc.), γ-amino-β-hydroxybutyrates (methyl γ-amino-β-hydroxybutyrate, ethyl γ-amino-β-hydroxybutyrate, propyl γ-amino-β-hydroxybutyrate, butyl γ-amino-β-hydroxybutyrate, ethylhexyl γ-amino-β-hydroxybutyrate, hexadecyl γ-amino-β-hydroxyhexabutyrate, lauryl γ-amino-β-hydroxybutyrate, stearyl γ-amino-β-hydroxybutyrate, oleyl γ-amino-β-hydroxybutyrate, benzyl γ-amino-β-hydroxybutyrate, phenyl γ-amino-β-hydroxybutyrate, ethyl glycol γ-amino-β-hydroxybutyrate, sorbitol γ-amino-β-hydroxybutyrate, polyoxyethyleneglycol γ-amino-β-hydroxybutyrate, and glycerin γ-amino-β-hydroxybutyrate, etc.).

Examples of the compound further include: amineoxides (oleyldimethylamineoxide, stearyldimethylamineoxide, palmityldimethylamineoxide, myristyldimethylamineoxide, lauryldimethylamineoxide, dimethyllaurylethoxyamineoxide, dihydroxyethyllaurylamineoxide, and palm oil alkyldimethylamineoxide, etc.), alkylbetaines (palm oil fatty acid amide propylbetaine, palm oil alkylbetaine, lauryldimethylaminobetaine acetate, lauric acid amidopropylbetaine, laurylamidopropylbetaine, laurylhydroxysulfobetaine, 2-alkyl-N-carboxymethyl-N-hydroxyethylimidazolynium betaine, and dodecylcarboxyethylhydroxyethyl betaine, etc.), pyrimidine N-oxide derivatives (2-amino-4-methyl-6-piperidinopyrimidine-3-oxide, 2-amino-4-methyl-6-(1-pyrrolidinyl)pyrimidine-3-oxide, 2-amino-4-methyl-6-morpholinopyrimidine-3-oxide, 2-amino-4-methyl-6-[1-(4-methylpiperazinyl)pyrimidine-3-oxide, 2-amino-4-(1-hexahydroazepinyl)-6-methylpyrimidine-1-oxide, 2-amino-4-dimethylamino-6-methylpyrimidine-1-oxide, 2-amino-4-allylamino-6-methylpyrimidine-1-oxide, 2-amino-4-benzylamino-6-methylpyrimidine-1-oxide, 2-amino-4,5-methyl-6-piperidinopyrimidine-3-oxide, 2-amino-4-ethyl-6-morpholinopyrimidine-3-oxide, 2-amino-4-methyl-5-nitro-6-piperidinopyrimidin-3-oxide, 2,5-diamino-4-methyl-6-piperidinopyrimidine-3-oxide, 2-amino-4-methyl-5,6-bis(1-pyrodinyl)pyrimidine-3-oxide, 2-amino-4-methyl-5-piperidino-6-(1-pyroridinyl)pyrimidine-3-oxide, 2-methyl-4-amino-6-piperidinopyrimidine-3-oxide, 2-methyl-4-amino-5-bromo-6-(1-pyrodinyl)pyrimidine-3-oxide, 2-methyl-4-amino-5-nitro-6-piperidinopyrimidine-3-oxide, 2-methyl-4,5-diamino-6-piperidinopyrimidine-3-oxide, 2-methyl-4-amino-5,6-bis(1-pyrodinyl)pyrimidine-3-oxide, 2-amino-4-methyl-6-piperidinopyrimidine-3-oxide monohydrochloride, 2-acetylamino-4-methyl-6-piperidinopyrimidine-3-oxide monohydrochloride, 2,4-diamino-6-phenoxypyrimidine-3-oxide, 2,4-diamino-6-(2,4-dichlorophenoxy)pyrimidine-3-oxide, 2,4-diamino-6-(2,4,6-trichlorophenoxy)pyrimidine-3-oxide, 2,4-diamino-5-nitroso-6-(2,4-dichlorophenoxy)pyrimidine-3-oxide, 2,4-diamino-5-nitro-6-(2,4,6-trichlorophenoxy)pyrimidine-3-oxide, 2,4-diamino-5-nitro-6-(2,4-dichlorophenoxy)pyrimidine-3-oxide, 2,4,5-triamino-6-(2,4-dichlorophenoxy)pyrimidine-3-oxide, and 2,4-diamino-5-bromo-6-(2,4-dichlorophenoxy)pyrimidine-3-oxide, etc.), p-menthane-3,8-diol, monoglyceryl-D-glucoside monotridecanoate, 1-o-N-pentadecylglycero-D-glucoside, decylglycerylglucoside, ethylglucoside, eugenylglucoside, and glyceride sulfate such as monopentadecanic acid glyceride sulfate.

Examples of the compound further include monopentadecylglyceryl ether sulfate, 1-o-hexadecyl-2-o-methylglycerol, 1-o-octadecyl-2-o-methylglycerol, 1-o-oleyl-2-o-methylglycerol, acetylcarnitine or its salt, geranylgeranylacetone, a hydroxam derivative or its salt, a zingerone glycoside, a benzene oxyacetate derivative ([5-[2-[1-phenyl-1-(3-pyridyl)methylideneaminooxy]ethyl]-7,8-dihydronaphthalen-1-yloxy]acetate and the like), isorhamnetin-3-robinobioside, a xanthone derivative, proanthocyanidins (proanthocyanidin from a grape seed extract, proanthocyanidin derived from apples, proanthocyanidin derived from pine, purified proanthocyanidin oligomer, proanthocyanidin B-1, proanthocyanidin B-2, proanthocyanidin B-3, and proanthocyanidin C-1, etc.), phthalazinones, benzoxadinones, a phosphoate derivative, cyproterone, 5α-androstene-3α, 17β-diol, medoroxyprogesterone, norethisterone, and mestanolone.

Examples of the compound further include: a crista galli extract; a bovine, pork, or human placenta extract; pork or bovine belly, duodenum, intestinal, kidney extracts or digests thereof; a bovine or a pork brain extract; soluble collagen; collagen derivatives such as acylated collagen; collagen hydrolysates (tripeptide from collagen and the like); elastin; an elastin hydrolysate; a soluble elastin derivative; keratin and its digest or derivatives thereof; a silk protein and its digest or derivatives thereof; a pork or bovine hemocyte protein digest (globin peptide); a bovine or pork hemoglobin digest (hemin, hematin, hem, protohem, and hemoferrum, etc.); milk; casein and its digest (casein hydrolysate and the like) or derivatives thereof; skimmilk powder and its digest or derivatives thereof; lactoferrin or its digest; an egg component; a fish digest; a yeast metabolite; a yeast extract; a bacteria metabolite; a bacteria extract; a metabolite of fungi, mushroom, or the like; an actinomyceta metabolite; an extract of fungi, mushroom, or the like; an actinomyceta extract; bacillus natto metabolite; bacillus natto extract; a soya protein hydrolysate; a rice fermentative extract; rice bran (red bran and white bran) fermentative extracts; an euglena extract or its digest or soluble derivatives thereof; a lactic fermented product of raw milk or skimmilk powder; and trehalose, its derivative, and the like.

Examples of the compound further include and substances from natural products such as oceanic component including: salt waters such as deep water including sea water salt; a sea water dry substance; mineral salts obtained by sea water of Dead Sea, Atlantic Ocean, or Pacific Ocean (sodium chloride, magnesium chloride, potassium chloride, and the like); sea muds or muds (fungos) such as sea muds or muds (contained components: silicon dioxide, titanium dioxide, aluminum oxide, iron oxide, manganese oxide, sodium oxide, potassium oxide, magnesium oxide, calcium oxide, strontium oxide, sodium, potassium, magnesium, calcium, chrome, iron, copper, nickel, zinc, lead, manganese, arsenic, and water) such as Italian fango, German fango, Eifel fango, and Freiburg fango; shotokustone.

Examples of the compound further include various compounds, extracts of animals, plants, and microorganisms, naturally-derived from substances, and the like such as almond extract, *Phyllanthus emblica* extract, ukyoyo extract, *Atractylodes ovata* extract, *Atractylodes japonica* extract, konfuyou extract, uncaria extract, gaigicya extract, kakojuyo extract, glycyrrhiza extract, *Gardenia florida* extract, kuranigean extract, *Sophora angustifolia* extract, *Scutellaria baicalensis* root extract, triticum extract, rice extract, coriaria extract, sidowaya extract, sanukyu extract, sanbitro extract, *Cassia minosoides* extract, silane extract, *Cnidium officinale* root extract, senna leaf powdered extract, *Inula britannica* extract, *Lynthrum anceps* extract, surigaten extract, *Angelica decursiva* extract, coix seed extract, rotundifolia extract, *Vitex rotundifolia* extract, *Hamamelis virginiana* extract, palm extract, *Parietaria officinalis* extract, safflower extract, *Morus alba* extract, *Sophora flavescens* extract, irudaceas extract, *Iris florentina* extract, *Clematis terniflora* extract, yasyajitu extract, hongkong extract, *Sanguisorba officinalis* root extract, yuzuriha extract, *Polygonum multiflorum* extract, fatsia extract, myall extract, acerola extract, flowering maole extract, silver birch extract, gallic extract, *Paulownia tomentosa* extract, *Plectranthus kameba* extract, *Isodon trichocarpus* extract, *Plectranthus japonicus* extract, Japanese parsley extract, buckwheat extract, dabiria extract, *Capsella bursa-pastoris* extract, thoroughwort extract, camomile extract, mulberry extract, *Sophora angustifolia* extract, felon herb extract, paper birch extract, houttuynia extract, hoelen extract, *Lamium album* extract, hop vine extract, crataegus Fruit extract, *Eucalyptus globulus* leaf extract, *Althaea officinalis* root extract, cinnamon bark extract, Vitex *ritundifolia* extract, *Hamameris virginiana* extract, *Morus bombycis* extract, platycodon root extract, cuscuta seed extract, *Euphorbia lathyris* extract, *Belamcanda chinensis* extract, ephedra herba extract, *Araliae cardatas* rhizome extract, saikokeishikankyoto dry extract, *Saposhnikovia divaricate* extract, *Glehnia littoralis* extract, *Cortex moutan* extract, *Paeonia albiflora* root extract, *Geranium thunbergii* extract, *Radix puepariae* extract, licorice extract, praecox extract, aloe extract, *Cimicifuga* rhizome extract, green tea extract, black tea extract, gambir extract, anise extract, *Polygonum cuspidatum* extract, *Daphne pseudo-mezereum* extract, *Cassia obtusifolia* extract, *Cassiae semen* extract, *Astragali radix* extract, *Astragalus membranaceus* extract, *Trichosanthes bracteata* extract, *Xanthium stramarium* extract, *Gastrodia elata* extract, firethorn extract, *Polygonum sachalinense* extract, *Lindera strychnifolia* extract, pumpkin extract, *Typha latifolia* extract, *Euphoribia kansui* extract, *Agrimonia pilosa* extract, *Lindera umbellata* extract, *Saxfraga fusca* extract, sisal hemp extract, *Clematis chinensis* extract, *Clematis chinensis* extract, *Clematis chinensis* root extract, *Prunus speciosa* extract, *Prunus sargentii* extract, *Prunus incisa* extract, *Prunus nipponia* extract, *Prunus subhirtella* extract, *Prunus lannesiana* extract, *Aster tataricus* extract, *Trachycarpus fortunei* extract, *Iris florentina* extract, *Clematis terniflora* extract, *Magnolia salicifolia* extract, *Saxifraga fortunei* var. *incisolobata* extract, white evening primrose extract, *Cuscutae semen* extract, *Cuscuta australis* extract, *Cuscuta japonica* extract, *Artemisia absinthium* extract, *Achillea alpina* extract, *Dictamnus dasycarpus* extract, dill seed extract, *Polygonum cuspidatum* extract, *Tribulus terrestris* extract, *Mimachlamys nobilis* extract, *Pyrrosia lingua* extract, *Typha angustifolia* extract, *Angelica dahurica* extract, floral blanca extract, hamyura extract, *Artemisia priceps* extract, *Convolvulus arvensis* extract, *Santalum album* extract, *Ganoderma lucidum* extract, *Leonurus sibiricus* extract, *Salix gilgiana* extract, *Salix chaenomeloides* extract, *Salix gracilistyla* extract, *Salix* integra extract, *Salix kinuyanagi* extract, *Salix koriyanagai* extract, *Salix matsudana* extract, *Salix reinii* extract, *Salix sieboldiana* extract, *Toisusu urbaniana* extract, *Salix schwernii* extract, *Salix vulpina* extract, *Populus maximowiczii* extract, *Myrica rubra* extract, *Agave americana* extract, *Agave americana* var. *marginata* extract, *Agave americana* var. *marginata* extract, *Edgeworthia chrysantha* extract, *Enteromorpha* sp. extract, *Enteromorpha linza* extract, *Enteromorpha prolifera* extract, *Enteromorpha compressa* extract, *Enteromorpha intestinalis* extract, *Enteromorpha crinita* extract, *Laminaria* sp. extract, *Laminaria japonica* extract, *Laminaria ochotensis* extract, *Laminaria religiosa* extract, *Laminaria angustata* extract, *Undaria pinnIatifida* extract, *Undaria undaroides* extract, *Undaria peterseniana* extract, *Hizikia fusiformis* extract, *Fucus evanescens* extract, *Padina arborescens* extract, *Padina australis* extract, kirebanoumiuchiwa extract, *Padina boryana* extract, *Padina crassa* extract, *Padina japonica* extract, *Padina minor* extract, *Padina stipitata* extract, *Eucheuma serra* extract, *Eucheuma amakusaense* extract, *Eucheuma denticulatum* extract, *Eucheuma arnoldii* extract, *Chondrous ocellatus* extract, *Chondrus verrucosus* extract, *Chondrus nipponicus* extract, *Chondrus pinnulatus* extract, *Chondracanthus tenellus* extract, *Chondracanthus* teedii extract, *Chondracanthus intermedius* extract, *Dictyopteris latiuscula* extract, *Dictyopteris polypodioides* extract, *Padina arborescens* extract, *Nemacystus decipiens* extract, *Sphaerotrichia divaricata* extract, *Cymathaera* sp. extract, *Cymathaere japonica* extract, *Sargassum hemiphyllum* extract, *Sargassum segii* extract, *Sargassum hemiphyllum* extract, *Sargassum sagamianum* extract, *Saraggsum nigrifolium* extract, *Sargassum ringgoldianum* extract, *Sargassum tosaense* extract, *Sargassum piluliferum* extract, *Sargassum thunbergii* extract, *Sargassum ringgolodianum* extract, *Sargassum confusum* extract, *Sargassum muticum* extract, Sargassum siliguastrum extract, *Sargassum micracanthum* extract, *Sargassum giganteifolium* extract, *Grateloupia imbricata* extract, *Sebdenia flabellata* extract, *Sebdenia polydacty* extract, *Grateloupia acuminata* extract, *Carpopeltis affIinis* extract, *Gracilaria gigas* extract, *Ceratodictyon spongiosum* extract, *Lomentaria catenata* extract, *Lomentaria pinnata* extract, *Lomentania okamurae* extract, *Laurencia intermedia* extract, *Laurencia undulata* extract, *Laurencia pinnata* extract, *Laurencia brongniartii* extract, *Odonthalia corymbifera* extract, thira extract, kamote de azafuran extract, jamaica extract, poreo velde extract, nabo negro extract, *Zanthoxylum piperitum* extract, *Aralia cordata* extract, *Angelica pubescens* extract, gasho extract, ever green oak extract, *Circaea cordata* extract, *Ageratum conyzoides* extract, *Rumex japonicus* root extract, *Gymnema sylvestre* extract, karoou extract, *Tareana gracilipes* extract, *Mussaenda parviflora* extract, sabunryo extract, jasminum extract, *Alcea rosea* extract, shoraito extract, *Arabidopsis thaliana* extract, *Aspidistra elatior* extract, *Aconitum japonicum* extract, *Erythrina cristagalli* extract, *Aucommia ulmoides* extract, *Ruscus aculeatus* (Butcher's broom) extract, *Ecballium elaterium* extract, *Loropetalum chinense* extract, *Sapium sebiferum* extract, *Ailanthus moluccana* extract, *Dianthus hybridus* extract, *Polygonum cuspidatum* extract, *Polygonum aviculare* extract, *Corchorus olitorius* extract, *Salix subfragilis* extract, *Salix babylonica* extract, *Kummerowia striata* extract, *Coronilla varia* extract, *Wikstroemia indica* extract, *Dictyota dichotoma* extract, shellfishes extract (a cockles extract, *Perna viridis* extract, oyster extract, *Ostrea edulis* extract, scallop extract, clam extract, clam extract, mactridae extract, *Nuttallia olivacea* extract, ark shell extract, shell fish extract, top shell extract, *Babylonia japonica* extract, and the like), *Aucuba japonica* extract, bottle tree extract, *Firmiana* sp. extract, *Astilbe thunbergii* extract, *Trifolium pratense* extract, *Thalictrum minus* extract, Aguaje *(Mauritia flexuosa*) extract, *Pharbitis purpurea* extract, morning glory extract, *Canna edulis* extract, oilseed rape extract, *Gynostemma pentaphyllum* extract, *Tulipa edulis* extract, *Amaranthus* extract, *Amaranthus tricolor* extract, *Amaranthus caudatus* extract, velvet flower extract, *Amaranthus cruentus* extract, *Amaranthus patulus* extract, beetroot extract, spiny pigweed extract, *Epipactis papillosa* extract, *Amaranthus hypocondriacus* extract, *Alstroemeria* extract, alcanna extract, Algarrobo extract, *Aloe barbadensis* extract, igusa reed extract, yew extract, strawberry extract, locust bean extract, draba extract, rice extract, catmint extract, *Conandron* extract, *Selaginella* extract, *Selaginella tamariscina* extract, wheat *(Genipa americana)* extract, floating grass extract, extract, marshmallow extract, mallow extract, Cat's Claw (*Uncaria tomentosa*) extract, winter mushroom extract, eryngii extract, *Pleurotus eryngii* extract, *Opuntia maxima* extract, *Inula helenium* extract, *Eriocaulon* extract, Oca extract, *Cnidium monnieri* extract, okra extract, *Dioscorea tokoro* extract, tokoro extract, nagadokoro extract, ononis extract, olluquito extract, *Glechoma* extract, *Glechoma hederacea* extract, *Hydrangea macrophylla* extract, cascara sagrada extract, gypsophila extract, asian ginseng extract, canihua extract, carnation extract, gerbera extract, camu camu (camo camo) extract, calla extract, oat extract, Chinese quince extract, *Potentilla chinensis* extract, *Veronica undulata* extract, *Dianthus superbus var. longicalycinus* extract, *Dianthus superbus var. longicalycinus* extract, kantaraasa extract, *Dendranthema boreale* extract, quinua (quinoa) extract, cabbage extract, kiwi extract, cucumber extract, tamarisk extract, *Ajuga decumbens* extract, *Parthenium argentatum* extract, *Asparagus cochinchinensis* extract, kudzu extract, *Artemisia annua* extract, cupuacu extract, *Thlaspi arvense* extract, *Hovenia* extract, selenicereus extract, *Picrorrhiza kurrooa* extract, coconut extract, cosmos extract, *Clematis apiifolia* extract, rice oil extract, konjak extract, saffron extract, sapodilla extract, Sangre de grado (Cronton) extract, *Panax notoginseng* extract, Shea (karete) extract, parkii extract, shiitake mushroom extract, shimeji mushroom extract, hinshimeji extract, *Lyophyllum fumosum* extract, *Lyophyllum decastes* extract, *Lyophyllum connatum* extract, *Hypsizygus* extract, *Lyophyllum shimeji* extract, *Lyophyllum marmoreus* extract, *Magnolia quinquepeta* extract, *Asarum* extract, cactus extract, *Ophiopogon japonicus* extract, *Gypsophila paniculata* extract, *Dichroa febrifuga* extract, *Sesbania grandiflora* extract, *Vigna* extract, daphne extract, watermelon extract, *Cynanchum paniculatum* extract, *Portulacea* extract, *Dendrobium* extract, *Dendrobium linawianum* extract, *Dendrobium nobile* extract, oobana sekkoku extract, *Dendrobium okinawense* extract, *Dendrobium officinale* extract, *Dendrobium phalaenopsis* extract, shikaku sekkoku extract, *Dendrobium tosaense* extract, cedron (lemon verbena) extract, *Malva sylvestris* extract, celery extract, *Andrographis paniculata* extract, Oriental radish extract, *Eclipta* extract, *Dioscorea gracillima* extract, dahlia extract, tarwi extract, *Euphorbia* extract, *Atractylodis lanceae* extract, chancapiedra (*Phyllanthus amarus*) extract, tulip extract, Chinese artichoke extract, common mushroom extract, camellia extract, trefoil extract, *Dipsacus* extract, corn extract, corn floss extract, horsetail extract, *Fraxinus* extract, *Prairie gentian* extract, sword bean extract, *Canavalia gladiate* extract, jujube extract, *Dipsacus japonicus* extract, nameko extract, *Boehmeria* extract, sweet violet extract, *Myristica* extract, silk tree extract, neb extract, nebi extract, *Albizia julibrissn* extract, *Amaryllis* extract, *Celosia argentea* extract, *Gentiana* extract, *Acer japonicum* extract, *Lupinus perennis* extract, papaya extract, paharobobo extract, fiddle leaf fig extract, field mushroom extract, field mushroom extract, horse mushroom extract, *Agaricus abruptibulbus* extract, *aspidistra* extract, pariurusu (*Paliurus ramosissimus*) extract, balsamina (*Momordica charantia*) extract, *Gentiana Thunbergii* extract, Handaikai extract, *Dahlia coccinea* extract, beet extract, hiba extract, sunflower extract, bell pepper extract, *Aquilegia* extract, *Agaricus blazei* extract, *Cordyceps sinensis* extract, *Periandra dulcis* extract, hazelnut extract, sponge cucumber extract, *crassulaceae* extract, *Sedum alboroseum* extract, cotton bush extract, *Kochia scoparia* extract, *Kochia scoparia* extract, *Kochia scoparia*/*Kochia* extract, garden balsam extract, *Stellaria dichotoma* extract, *Sedum aizoon* extract, jojoba extract, borage extract, bordeaux extract, *Cistanche* extract, *Dendrobium officinale* extract, maca extract, macadamia nut extract, marguerite extract, *Tropaeolum* extract, *Tropaeolum tuberosum* extract, silver vine extract, pine corn extract, *Poria cocos* extract, *Buddleja globosa* extract, *oblonga* extract, mullein *(Verbascum thapsus)* extract, manthanya (Andes camomile) extract, *Ottelia* extract, *Ottelia alismoides* extract, *Mentha viridis* extract, strawflower extract, munya extract, *Callicarpa* extract, *Callicarpa japonica* extract, *Milletia reticulata* extract, moje (pepper tree) extract, holly extract, cornflower extract, yacon extract, palm tree extract, *Carpesium* extract, Horse chesnut extract, *Chaenomeles lagenaria* extract, lily extract, *Polyporus mylittae* extract, lime extract, rye extract, shallot extract, eschalot extract, apple extract, gentian extract, lychee extract, milk vetch extract, *Coicis Semen* extract, minisasanishiki extract, *Chlorella vulgaris* extract, *Chlorella pyrenoidsa* extract, *Chlorella elypsoidia* extract, *Macrocystis pyrifera* extract, *Acanthopeltis* extract, *Meristotheca papulosa* extract, *Gelidium japonicum* extract, *Porphyra* extract, spirogyra extract, *Prasiola* extract, spherical moss extract, *Kornmannia* extract, *Dictyopteris divaricata* extract, *Petalonia binghamiae* extract, *Petalonia fascia* extract, *Colpomenia bullosa* extract, *Agarum* extract, *Costaria* extract, *Kjellmaniella* extract, *Eckloniopsis* extract, *Ecklonia stolonifera* extract, *Hedophyllum* extract, *Arthrothamnus* extract, *Alaria praelonga* extract, *Alaria crassifolia* extract, *Silvetia babingtonii* extract, *Hormophysa cuneiformis* extract, *Turbinaria conoides* extract, *Cystoseira hakodatensis* extract, *Myagropsis myagroides* extract, *Myagropsis turneri* extract, *Sargassum nipponicum* extract, *Sargassum fulvellum* extract, *Coccophora langsdorfii* extract, *Bangia atropurpurea* extract, *Porphyra yezoensis* extract, *Trichogloea lubrica* extract, *Nemalion vermiculare* extract, *Scinaia okamurae* extract, *Portieria hornemannii* extract, *Gloiopeltis complanata* extract, *Gloiosiphonia capillaris* extract, *Bonnemaisonia hamifera* extract, *Solieria pacifica* extract, *Solieria tenuis* extract, *Meristotheca coacta* extract, *Turnerella mertensiana* extract, *Hypnea charoides* extract, *Hypnea japonica* extract, *Hypnea saidana* extract, *Hypnea variabilis* extract, *Gracilaria ermiculophylla* extract, *Gracilaria chorda* extract, *Gelidiopsis hachijoensis* extract, *Ahnfeltiopsis flabelliformis* extract, *Mazzaella japonica* extract, sponge cucumber water, birch or red pine sap, *Rehmannia glutinosa* var. *purpurea* extract, fennel extract, *Acanthopanax senticosus* extract, barley extract, *Echinops latifolius* extract, *Hypericum perforatum* var. *angustifolium* extract, *Hypericum perforatum* extract, parsley extract, celery extract, *Rehmannia glutinosa* extract, roman chamonmile extract, *Garcinia* extract, *Artemisia apiacea* extract, *Epipactis thunbergii* extract, Quillaja extract, *Cephalanthera falcata* extract, *Cephalanthera erecta* extract, *Calendula officinalis* extract, *Neottia nidus-avis* var. *mandshurica* extract, *Cephalanthera longibracteata* extract, *Pterocarya rhoifolia* extract, *Filipendula multijuga* extract, *Glehnia littoralis* extract, *Prunus salicina* extract, walnut extract, pineapple extract, *Orchis aristata* extract, *Belamcanda chinensis* extract, *Fagus crenata* extract, Chinese lantern plant extract, *Grifola frondosa* extract, loose strife extract, *Hibiscus syriacus* extract, *Sapindus mukurossi* extract, bean sprout extract, eucalyptus extract, meadow saxifrage extract, *Rubus suavissimus* extract, lettuce extract, tanka bean extract, *Gentiana* extract, burdock extract, *Lithospermum erythrorhizon* extract, carrot extract, *Hammamelis* extract, hop extract, *Coix lachryma-jobi* extract, *Lamium album var. barbatum* extract, *Swertia japonica* extract, *Thymus vulgaris* extract, parsely extract, *Pachydictyon coriaceum* extract, *Dilophus okamurae* extract, gatugarakonbu extract, *Laminaria longipedalis* extract, *Laminaria diabolica* extract, *Laminaria yezoensis* extract, *Laminaria longissima* extract, *Laminaria yendoana* extract, oochijimikonbu extract, *Tricleocarpa cylindrica* extract, *Galaxaura falcata* extract, *Helminthocladia australis* extract, *Helminthocladia yendoana* extract, kagekinori extract, *Cirrulicarpus gmelini* extract, *Gracilaria bursa-pastoris* extract, *Gracilaria textorii* extract, *Akebia quinata* extract, *Hydrangea serrata* var. *thunbergii* extract, oolong tea extract, *Psoralea corylifolia* extract, *Cymbidium kanran* extract, *Fortunella japonica* extract, *Rhus chinensis* extract, Gen-no-shoko extract, sesame extract, sesame cultured cell extract, *Scrophularia buergeriana* extract, rice bran extract, pomegranate extract, *Zanthoxylum piperitum* extract, *Pyrus communis* extract, *Rheum palmatum* extract, tomate extract, nandina extract, *Cirsium japonicum* extract, *Rosa multiflora* extract, paprika extract, pistachio extract, Japanese medlar extract, betel extract, *Petasites japonicus* extract, *Chaenomeles speciosa* extract, *lnula linariifolia* extract, ephedra extract, mimosa extract, *Ocimum basilicum* extract, himekumayanagi extract, *Polygonum hydropiper* extract, watahujiutsugi extract, *Chlamydomonas* sp. extract, akayukimo extract, *Padina crassa* extract, *Padina japonica* extract, hammamelis extract, clove extract, melissa extract, *Isodon japonicus* extract, white birch extract, ginkgo extract, clove extract, *Haematoxylon campechianum* (logwood) extract, *Mallotus japonicus* extract, akaminoakane extract, *Rubia tinctorum* extract, *Rubia tinctorum* extract, hydrangea extract, *Quercus variabills* extract, fig extract, asparagus extract, *Prunus mume* extract, *Quercus stenophylla* extract, *Arctostaphylos uva-ursi* (bear-berry) extract, estragon extract, *Sophora japonica* extract, *Quercus* dentate extract, *Inula salicina* var. *asiatica* extract, birch extract, Betula extract, *Quercus acutissima* extract, *Nerium indicum* extract, *Citrus kinokuni* (*Citrus leiocarpa, Citrus tachibana, Citrus tangerina, Citrus tumida,* sagamikoji, *Citrus reticulata*) extract, santalum extract, coffee extract, comfrey extract, bamboo extract, perilla extract, *Perilla frutescens* extract, *Perilla frutescens* var. *crispa* extract, *Perilla frutescens* f. *discolor* extract, *Paeonia lactiflora* extract, *Quercus serrata* extract, *Quercus crispula* extract, *Lagerstroemia indica* extract, *Lycium chinense* extract, *Quercus myrsinaefolia* extract, *Trifolium repens* (clover) extract, horseradish extract, *Farfugium japonicum* extract, tentya extract, *Lindera strychnifolia* extract, *Rosa laevigata* extract, *Arachis hypogea* (peanut) extract, *Cinnamomum* extract, *Cinnamomum okinawense* extract, *Cinnamomum zeylanicum* extract, *Cinnamomum japonicum* (bark) extract, cinnamon stick extract, *Campsis grandiflora* extract, lotus extract, napav extract, rye extract, *Anemarrhena asphodeloides* extract, *Rosa rugosa* extract, rose extract, barbasco extract, spinach extract, *Fagus grandifolia* extract (beech acrospire extract or the like), *Fagus japonica* extract, *Fagus sylvatica* extract, *Calystegia japonica* extract, hosobanaokera extract, *Paeonia suffruticaoa* extract, marjoram extract, melon extract, *Oenothera* extract, *Spiraea thunbergii* extract, orchid extract, *Momordica grosvenori* extract, rooibos extract, *Portieria japonica* extract, *Plocamium telfairiae* extract, *Plocamium telfairiae* extract, *Betula grossa* extract, basil extract, saussureae extract, *Cocculus trilobus* extract, thistle extract, *Breea segetum* extract, *Cirsium borealinipponense* extract, *Cirsium maritinum* extract, *Cirsium japonicum* extract, oobana-azami extract, cacao extract, Hercampuri extract, *Sinomenium acutum* extract, *Curcuma zedoaria* extract, *Fumaria officinalis* extract, *Campanula punctata* extract, *Hedera rhombea* extract, pepper extract, *Cola acuminata* extract, *Alisma plantago-aquatica* var. *orientale* extract, shaddock extract, *Cornus officinalis* extract, *Sinomenium acutum* extract, *Dendropolyporus umbellatus* extract, *Centella aiatica* extract, *Gelidium* extract, *Gentiana scabre* extract, *Gentiana scabre* var. *scabra* extract extract, *Elsholtzia ciliata* extract, banana extract, Puer tea extract, plum extract, bohu extract, benzoin extract, habu extract, *Cladophora japonica* extract, *Cladophora sakaii* extract, *Cladophora glomerata* extract, *Peniophora quercina* extract, *Nostoc commune* extract, *Nostoc flagelliforme* extract, *Spirulina* extract, *Trichodesmium* extract, hunorinoushike extract, *Gelidium divarivcatum* extract, *Gelidium* pussillum extract, *Gelidium pacificum* extract, *Porphyridium cruentum* extract, red grape extract, adzuki extract, althea extract, nettle extract, cascarilla extract, ceiba extract, guarana extract, *Rubus idaeus var. aculeatissimus* extract, strawberry extract, *Rubus phoenicolasius* extract, *Rubus parvifolius* extract, *Rubus palmatus* extract, raspberry extract, *Aloe arborescens* extract, *Crataegus* sp. extract, *Melissa officinalis* extract, cranberry extract, *Cola bella* extract, sugar cane extract, shinahonoki extract, *Lonicera japonica* extract, *Equisetum arvense* extract, *Larix occidentalis* extract, *Sambucus nigra* (elder) extract, dandelion extract, *Centaurium* extract, *Taraxacum albidum* extract, *Taraxacum platycarpum* extract, black gram extract, *Galeola altissima* extract, *Juglans regia* var. *orientis* extract, tormentila extract, *Rosa multiflora* extract, *Casuarina equisetifolia* extract, Lady's Mantle extract, castor extract, grape extract (grape leaf extract), prune extract, veronica extract, visnaga extract, bilberry extract, *Juglans mandshurica* extract, buckbean extract, myrrh extract, hamamelis extract, *Populus maximowiczii* extract, rhatany (krameria) extract, lemon extract, corn poppy extract, shikon extract, rose fruit extract, propolis extract, *polygonum tinctorium* extract, *Gambir* extract, avocado extract, solomon's seal extract, *Fritillaria verticillata* extract, *Fritillaria* extract, *Elephantopus* extract, adders-wort extract, turmeric extract, echinacea (*E. angustifolia*) extract, *Coptidis japonica* extract, *inula britannica* extract, *Hypericum* extract, *Juglans mandshurica* var. *sieboldiana* extract, orange extract, cassia extract, *tetrapanax papyriferus* extract, *Agastache rugosa* extract, *Artemisia capillaris* extract, water cress extract, *Smilax glabra* extract, gentiana extract, *Dendrobium* extract, ligusticum extract, *Alipinia officinarum* extract, *Rubus chingii* extract, *Cremastra appendiculata* extract, cherry extract, salvia (sage) extract, digitalis extract, Thilia *japonica* extract, sal tree extract, *ficus religiosa* extract, *Amomi Semen* extract, *Coix lacryma-jobi* extract, *Nandina domestica* extract, ground ivy extract, medowsweet extract, yarrow (milfoil) extract, *Polygala senega* extract, *Echinops grijisii* extract, scythian lamb extract, *Thymus vulgaris* extract, *Stephania cepharantha* extract, *Persicae Semen* extract, *Imperata* extract, *Benincasa* extract, *Houttuynia cordata* extract, parsley (*Petroselinum crispum*) extract, *Eschscholtzia* extract, *Cyperus rotundus* extract, Chinese water chestnut extract, *Althea officinalis* extract, Piper extract, *Phaseolus radiatus* extract, sandalwood extract, butchers broom extract, pansy extract, *Chrysanthemum indicum* extract, *Arisaema heterophyllum* extract, *Aurantii Fructus Immaturus* extract, miracle fruit extract, *Aphananthe aspera* extract, peach extract, cornflower extract, sweet gale extract, mistletoe extract, *Ardisia japonica* extract, sagebrush extract, mugwort extract, rosemary extract, *Polygala tenuifolia* extract, *Chaetomorpha spiralis* extract, *Chaetomorpha moniligera* extract, *Chaetomorpha okamurai* extract, *Desmarestia ligulata* extract, *Desmarestia viridis* extract, *Myelophycus simplex* extract, *Sargassum horneri* extract, *Sargassum micracanthum* extract, *Neodilsea yendoana* extract, *Neodilsea tenuipes* extract, *Ahnfeltiopsis paradoxa* extract, *Palmaria palmata* extract, *Chondria crassicaulis* extract, belladonna extract, *Magnolia obovata* extract, elm extract, hemp extract, dead nettle extract, watercress (cress) extract, valerian extract, clematis extract, cumaceba extract, *Saponaria* extract, rosewood extract, horse Chestnut extract, soybean (Saponin) extract (for example, soybean seed extract), *Calendula officinalis* (common marigold) extract, Chinese yam extract, Japanese yam extract, nasturtium extract, coltsfoot extract, *Cayratia japonica* extract, giant kelp extract, *Cyrtymenia sparsa* extract, *Sophorae* Radix extract, Ferula (Agi) extract, *Hiba arborvitae* extract, alpinia extract, *Alpinia katsumadai* extract, *Amaranthus lividus (Amaranthus viridis*) extract, *Amaranthus retroflexus* extract, *Amaranthus spinosus* extract, *Amaranthus tricolor* subsp. *mangostanus* extract, *Amaranthus tricolor* extract, hosonagabiyu extract, *Isodon japonicus* extract, *Lonicera gracilipes* extract, *Asarum* sieboldii extract, *Asarum europaeum* extract, *Ulmus laciniata* extract, *Patrinia scabiosaefolia* extract, *Uncaria rhynchophylla* extract, *Zanthoxylum piperitum* extract, camomile extract, *Cinchona* extract, *Cinchona succirubra* extract, *Phellodendron* bark extract, caraway extract, *Piper betle* extract, *Sasa veitchii* extract, cranberry extract, grapefruit extract, *Celosia cristata* extract, bay extract, *Rosa chinensis* extract, copaiba balsam extract, *Prunus jamasakura* extract, *Prunus pendula* extract, *Prunus maximowiczii* extract, *Prunus yedoensis* extract, *Prunus verecunda* extract, *Prunus* x *kanzakura* extract, sugarbeet extract, *Cimicifuga simplex* extract, *Quisqualis indica* extract, cinnamon extract, *Rumex acetosa* extract, *Taraxacus officinale* extract, *Juniperus communis* extract, *Mentha piperita* extract, *Melissa officinalis* extract, *Amomum tsao-ko* extract, onion extract, chicory extract, *Ulmus macrocarpa* (fruit) extract, *Tetragonia tetragonoides* extract, *Cephaelis* extract, *Allium tuberosum* (Allium seed) extract, *Allium fistuiosum* extract, *Prunus davidiana* extract, patchouli extract, *Lycoris radiata* extract, Japanese cypress extract, pimenta extract, *Pulsatilla chinensis* extract, blackberry extract, *Digenea simplex* extract, *Magnolia sprengeri* extract, *Lobelia chinensis* extract, *Cryptotaenia japonica* extract, *Lithospermum erythrorhizon* (root) extract, *Phyllostachys pubescens* extract, *Phytolacca* (root) extract, yucca extract, Angelica *dahurica* extract, lavender extract, *Forsythia suspensa* extract, *Forsythia viridissima* extract, *Chondrus giganteus* extract, *Chondrus crispus* extract, akamomijinori extract, *Ulva pertusa* extract, *Monostroma nitidum* extract, *Monostroma latissimum* extract, *Monostroma sravillei* extract, *Ishige sinicola* extract, *Ishige okamurae* extract, *Eisenia bicyclis* extract, *Delisea japonica* extract, *Grateloupia elliptica* extract, *Grateloupia divaricata* extract, *Grateloupia imbricata* extract, *Neorhodomela aculeata* extract, *Batrachospermum iriomoteensis* extract, *Batrachospermum sirodotii* extract, *Batrachospermum gallaei* extract, *Alpinia oxyphylla* extract, *Angelica keiskei* extract, arnica extract, *Scirpus fluviatilis* extract, *Carum carvi* extract, *Citrus unshiu* (peel) extract, *Corylus heterophylla* extract, *Disopyros Kaki* extract, *Cinchona* extract, *Cymbidium pumilum* extract, *Verbena officinalis* extract, *Papaver rhoeas* extract, *Nuphar japonicum* extract, *Arctium lappa* extract, *Lobelia sessiflora* extract, *Forsythia viridissima* extract, ginger extract, *Acorus* (calamus) extract, hawthorn extract, *Swertia japonica* extract, *Citrus aurantium* (peel) extract, *Salvia miltiorrhiza* extract, thyme extract, *Panax japonicus* extract, *Polygonum multiflorum* (tuberous root) extract, *Codonopsis lanceolata* extract, capsium extract, *Angelica* extract, *Ligustrum lucidum* extract, *Citrus natsudaidai* extract, *Sambucus sieboldiana* extract, *Corylus* heteophylla extract, *Scopolia japonica* (root) extract, *Mentha arvensis* extract, *Glehnia littoralis* extract, *Echinops setifer* extract, *Daphne genkwa* extract, blackcurrant extract, linden extract, melilot extract, *Citrus junos* extract, *Chimonanthus praecox* extract, *Ostericum sieboldii* extract, hibiscus extract, rosehip extract, *Gentiana* extract, *Thymus serpyllum* subsp. *quinquecostatus* extract, *Prunella vulgaris* extract, *Lagerstroemia speciosa* (banaba) extract, Chinese hawthorn extract, *Crataegus cuneata* extract, *Magnolia officinalis* extract, guava extract, *Schisandrae fructus* extract, Plantago *asiatica* extract, *Rosa centifolia* extract, *Hypericum perforatum* extract, Stellaria extract, *Achyranthes fauriei* extract, *Magnolia obovata* extract, fox grape extract, European grape extract, *vitis coignetiae* extract, madarin extract, *Bupleurum scorzoneraefolium* extract, *Pisum sativum* extract, *Callophyllis* extract, *Mastocarpus yendoi* extract, *Gigartina ochotensis* extract, *Mastocarpus pacifficus* extract, *Ceramium kondoi* extract, *Ceramium tenerrimum* extract, *Ceramium paniculatum* extract, *Ceramium japonicum* extract, *Champia expansa* extract, *Polysiphonia morrowii* extract, *Polysiphonia crassa* extract, *Chondria armata* extract, *Chondria dasyphylla* extract, *Chondria intertexta* extract, *Chondria ryukyuensis* extract, *Chondria expansa* extract, *Chondria lancifolia* extract, *Wasabia japonica* extract, *Epimedium grandiflorum* extract, *Epimedium grandiflorum* var. *grandiflorum* (*E. sagittatum*) extract, *Polygonatum falcatum* extract, *Polygonatum sibiricum* (root) extract, oregano extract, *Dryopteris* extract, *Polygala* root extract, prunus bark extract, *Pinellia ternata* extract, *Citrus aurantium* extract, *Lycium chinense* extract, *Evodia rutaecarpa* extract, senega extract, *Melia azedarach* extract, tamarind extract, tarragon extract, *Rheum coreanum* extract, *Phyllostachys nigra* var. *henonis* extract, daisy extract, myrobalan extract, *Hibiscus syriacus* extract, laurel extract, *Cyrtomium fortunei* extract, *Ulva pertusa* extract, *Macrocystis integrifolia* extract, *neocystis ruetokeana* extract, *Ptilophora subcostata* extract, *Pterocladia capillacea* extract, *Pterocladia capillacea* extract, *Acanthopeltis hirsuta* extract, *Gelidiella acerosa* extract, *Chondrus yendoi* extract, *Chondrus armatus* extract, *Caulerpa okamurae* extract, *Bryopsis plumosa* extract, *Codium fragile* extract, *Codium divaricatum* extract, *Codium contractum* extract, *Codium cylindricum* extract, *Codium latum* extract, *Ceratophyllum demersum* extract, *Cladosiphono okamuranus* extract, *Nemacystus decipiens* extract, *Colpomenia sinuosa* extract, *Ecklonia cava* extract, *Gloiopeltis furcata* extract, *Gloiopeltis tenax* extract, *linum* extract, *Broussonetia papyrifera* (fruit) extract, *Broussonetia kazinoki* extract, *Origanum vulgaris* extract, *Artcarpus altilis* extract, *Chondrus pinnulatus* extract, *Chondrus elatus* extract, *Ulva reticulata* extract, *Ulva arasakii* extract, *Callophyllis japonica* extract, *Callophyllis crispata* extract, *Callophyllis palmata* extract, *Callophyllis adhaerens* extract, *Callophyllis adnata* extract, *Callophyllis rhynchocarpa* extract, *Callophyllis hayamensis* extract, *Callophyllis mageshimensis* extract, *Callophyllis rhynchocarpa* extract, *Panax japonicus* extract, *Lilium lancifolium* extract, *Lilium japonicum* extract, *Lilium brownii* extract, corn poppy extract, *Trichosanthes kirilowii* var. *japonica* (Karokon) extract, fenugreek extract, *Lablab purpurea* extract, lentil extract, Chick pea extract, mung-bean extract, *Arnica unalaschkensis* var. *tschonoskyi* extract, *Humulus japonicus* extract, *Humulus lupulus* var. *cordifolius* extract, raspberry extract, *Acorus gramineus* (root) extract, *Quercus glauca* extract, angelica extract, *Prunus armeniaca* var. *ansu* (apricot kernel) extract, *Prunus armeniaca* extract, *Pyrola japonica* extract, *Cytisus scoparius* extract, *Plantago asiatica* extract, *Alnus sieboldiana* extract, olive extract, *Veronica persica* extract, *Polygonatum* extract, *Trichosanthes cucumeroides* extract, *Trichosanthes kirilowi* extract, *Chrysanthemum* extract, *Chrysanthemum indicum* extract, *Chrysanthemum zawadskii* var. *latilobum* extract, *Catalpa ovata* extract, kukui nut extract, *Cinnamonum camphora* extract, cubeb extract, *Rhamnus japonica* var. *decipiens* extract, *Schizonepetae* extract, *Osmanthus fragrans* var. *aurantiacus* extract, *Antirrhinum majus* extract, *Citrus tachibana* peel extract, *Camellia*×*hiemalis* extract, coentro extract, *Magnolia kobus* extract, *Magnolia liliflora* extract, *Kadsura japonica* extract, *Kadsura japonica* extract, *Schisandra nigra* extract, colombo extract, condurango extract, *Camellia sasanqua* extract, sweet potato extract, *Zizyphus jujuba* extract, *Euchresta japonica* (root) extract, potato extract, *Blechnum niponicum* extract, *Gardeniae Fructus* fruit extract, coriander extract, stevia extract, *Euphorbia pekinensis* extract, *Ilex latifolia* extract, *Aralia elata* extract, *Picrasma quassioides* extract, *Rhus javanica* var. *roxburghii* (fruit) extract, *Drynaria fortunei* (root) extract, *Magnolia heptapeta* extract, *Iris florentina* extract, *Dryopteris nipponensis* extract, *Gnaphalium affine* extract, *Codonopsis pilosula* (root) extract, mangosteen extract, *Zingiber mioga* extract, *Melaleuca alternifolia* extract, *Alnus firma* extract, *Alnus pendula* extract, *Alnus sieboldiana* extract, *Leonurus sibiricus* extract, *Alnus hirsuta* var. *sibirica* extract, longan extract, *Phragmites communis* extract, logwood extract, lantana extract, *Iris tectorum* extract, Polygoni Multiflori extract, kanto extract, *Tulipa edulis* extract, *Gracilaria bursa-pastoris* extract, *Atractylodis Lanceae* root extract, *Genista tinctoria* extract, common duckweed extract, myrrha extract, *Champia parvula* extract, *Sargassum siliquastrum* extract, blueberry extract, mango extract, dil extract, elemi extract, dammar extract, vanilla bean extract, pine extract, *Sabia japonica* extract, *Gastrodia elata f. viridis* extract, red currant extract, *Ulmus parvifolia* (bark) extract, aguaii guasu extract, abiu extract, abiurana extract, yellow zapote extract, ilang-ilang extract, *Orchis graminifolia* extract, uva tea extract, *Epipactis papillosa* extract, unboku extract, elderberry extract, *Agave filifera* cv. *Compacta* extract, *Agave victorae-reginae* extract, saguaro cactus extract, *Calocarpum sapota* extract, *Orchis fauriei* extract, *Lygodium Japonicum* extract, karaya extract, garyu extract, *Trametes versicolor* extract, candelilla extract, *Canna generalis* extract, savory extract, *Cereus peruvianus* extract, cabeb extract, *Quillaja saponaria* extract, *Opuntia leucotricha* extract, *Opuntia tuna* extract, *guapeba velmelha* extract, *Cephalanthera erecta* var. *shizuoi* extract, gooseberry extract, kutitiriba extract, *Curculigo latiforia* extract, *Ilex rotunda* extract, *Sorghum nervosum* extract, sapote amarillo extract, salmonberry extract, *Echinops gmelinii* extract, *Hylocereus* cactus extract, *Epimedium semperuirens* extract, *Cymbidium goeringii* extract, shozuku extract, sirobana-tuta extract, *Cinchona succirubra* extract, suimu-berry extract, starapple extract, *Cymbidium ensifolium* extract, *Pinus sylvestris* extract, bilberry extract, caper extract, European mistletoe extract, *Opuntia dillenii* extract, senboku extract, zougechu extract, *Chimonanthus praecox* f. *concolor* extract, *Dioscorea matsudai* extract, darksweet cherry extract, *Opuntia vulgaris* extract, chokonosutei extract, *Artemisia argyi* extract, *Neottia kiusiana* extract, *Galeola septentrionalis* extract, *Commelina communis* extract, duberry extract, toukagikazura extract, tragacanth extract, triacanthos extract, Nigeria berry extract, rapeseed extract, *Orchis joo-iokiana* extract, paudoce extract, hakukayumato extract, huckleberry extract, batata extract, *Epipactis papillosa* var. *sayekiana* extract, barata extract, *Cymbidium* x *nishiuchianum* extract, pecannut extract, hinachiyodori extract, *Neottia asiatica* extract, *Pfaffia iresinoudes* extract, brahnen extract, *Furcellaria* extract, blond psyllium extract, *Cymbidium dayanum* extract, henequen extract, berry extract, persea extract, peruvianberg extract, *Pereskia grandifolia* extract, boysen berry extract, *Cymbidium sinense* extract, *Pouteria sapota* extract, *Pouteria lucuma* extract, *Echinacea angustifolia* extract, whortleberry extract, masaranduba extract, masaranduba do sealar extract, masheila de boi extract, matta orro extract, mulberry extract, green sapote extract, purple corn extract, *Opuntia megacantha* extract, *Pereskia aculeata* extract, *Rheum palmatum* extract, morello cherry extract, yawaraka-murasakiimo extract, *Monarda didyma* extract, *Echinops ritro* extract, red currant extract, red pitaya extract, lemongrass extract, *Copernicia cerifera* extract, logan berry extract, green algae extracts such as *Dunariella* sp. (genus *Dunariella*) extract, *Chlorococcus* sp. (genus *Chlorococcus*) extract, *Pandorina* sp. (genus *Pandorina*) extract, *Volvox globator* (genus *Volvox*) extract, *Volvox* sp. (genus *Volvox*) extract, *Palmera* extract, *Tetraspora* extract, *Mougeotia* (genus *Spirogyra*) extract, *Draparnaldia plumosa* extract, *Ulothrix implexa* (genus *Ulothrix*) extract, furicchera extract, *Valonia macrophysa* (genus *Valonia*) extract, *Valonia aegagropila* (genus Valonia) extract, *Boergesenia forbesii* (genus *Boergesenia*) extract, *Caulerpa recemosa* (genus *Boergesenia*) extract, *Caulerpa brachypus* (genus *Boergesenia*) extract, *Caulerpa scalpelliformis* (genus *Boergesenia*) extract, *Acetabularia ryukyuensis* (genus *Acetabularia*) extract, *Closterium* sp. extract, korekaete extract, *Cosmarium* sp. extract, *Dictyosphaeria cavernosa* (genus *Dictyosphaeria)* extract, *Oedogonium* sp. extract, *Pediastrum* sp. extract, *Trentephohlia* sp. (genus *Trentephohlia*) extract, *Zygnema* sp. extract, and *Vaucheria* sp. extract, blue-green algae extracts such as *Aphanothece sacrum* (genus *Aphanothece*) extract, microcystis extract, and *Oscillatoria* sp. extract, brown algae extracts such as *Pilayella* sp. (genus *Pilayella*) extract, *Ectocarpus indicus* (genus *Ectocarpus*) extract, *Sorocarpus micromorus* (genus *Sorocarpus*) extract, *Ralfsia fungiformis* (genus *Ralfsia*) extract, *Sphacelaria tribuloides* (genus *Sphacelaria*) extract, *Halopteris filicina* (genus *Halopteris)* extract, *Cutleria cylindrica* (genus *Cutleria*) extract, *Cutleria multifida* (genus *Cutleria*) extract, *Cutleria adspersa* (genus *Cutleria*) extract, *Dictyota dilatata* (genus *Dictyota*) extract, *Spatoglossum pacificum* (genus *Spatoglossum*) extract, *Stypopodium zonale* (genus *Stypopodium*) extract, *Elachista taeniaeformis* (genus *Elachista*) extract, *Halothrix ambigua* (genus *Halothrix*) extract, *Leathesia difformis* (genus *Leathesia*) extract, *Agarum clathratum* (genus *Agarum*) extract, *Chordaria flagelliformis* (genus *Chordaria*) extract, *Eudesme virescens* (genus *Eudesme*) extract, *Tinocladia crassa* (genus *Tinocladia*) extract, *Hydrilla verticillata* (genus *Hydrillata*) extract, *Acrothrix pacifica* (genus *Acrothrix*) extract, *Carpomitra costata* (genus *Carpomitra*) extract, *Sporochnus radiciformis* (genus *Sporochnus*) extract, *Nereia intricata* (genus *Nereia*) extract, *Desmarestia tabacoides* (genus *Desmarestia*) extract, *Akkesiphycus lubricum* (genus *Akkesiphycus*) extract, *Punctaria latifolia* (genus *Punctaria*) extract, *Asperococcus bullosus* (genus *Asperococcus*) extract, *Coilodesme japonica* (genus *Coilodesme*) extract, chishimahukuronori (genus *Shimahukuronori*) extract, *Hydroclathrus clathratus* (genus *Hydroclathrus*) extract, *Chnoospora implexa* (genus *Chnoospora*) extract, *Stictyosiphon soriferus* (genus *Stictyosiphon*) extract, *Striaria attenuata* (genus *Striaria*) extract, *Scytosiphon lomentaria* (genus *Scytosiphon*) extract, *Dictyosiphon foeniculaceus* (genus *Dictyosiphon*) extract, *Chorda filum* (genus *Chorda*) extract, *Ecklonia kurome* (genus *Ecklonia*) extract, *Thallasiophyllum clathrus* (genus *Thallasiophyllum*) extract, nejirekonbu (genus Nejirekonbu) extract, *Macrocystis pyrifera* (genus *Macrocystis*) extract, buruukimo (genus *Buruukimo*) extract, and *Scytosiphon lomentaria* (genus *Scytosiphon*) extract, red algae extracts such as *Porphyra pseudolinearis* (genus *Porphyra*) extract, *Porphyra dentata* (genus *Porphyra*) extract, tasa (genus *Porphyra*) extract, *Porphyra variegata* (genus *Porphyra*) extract, *Porphyra amplissima* (genus *Porphyra*) extract, *Audouinella howei* (genus *Audouinella*) extract, *Liagora caenomyce* (genus *Liagora*) extract, *Ligora japonica* (genus *Liagora*) extract, aokonahada (genus *Liagora*) extract, *Nemalion multifidum* (genus *Nemalion)* extract, *Dermonema pulvinatum* (genus *Nemalion)* extract, *Dermonama frappieri* (genus *Dermonama)* extract, *Scinaia okamurae* (genus *Scinaia)* extract, *Actinotorichia fragilis* (genus *Actinotorichia)* extract, *Ptilonia okadai* (genus *Ptilonia)* extract, *Gelidium subfastigiatum* (genus *Gelidium)* extract, *Gelidium tenue* (genus *Gelidium)* extract, yohira (genus *Gelidium)* extract, *Gelidium linoides* (genus *Gelidium)* extract, *Dudresnaya japonica* (genus *Dudresnaya)* extract, *Dudresnaya minima* (genus *Dudresnaya)* extract, *Hyalosiphonia caespitosa* (genus *Hyalosiphonia)* extract, *Pikea yoshizakii* (genus *Pikea)* extract, *Dumontia contorta* (genus *Dumontia)* extract, *Dumontia simplex* (genus *Dumontia)* extract, *Masudaphycus irregulare* (genus *Masudaphycus)* extract, *Constantinea subulifera* (genus *Constantinea)* extract, *Rhodopeltis borealis* (genus *Stylaster)* extract, *Contarinia okamurae* (genus *Contarinia)* extract, *Peyssonnelia caulifera* (genus *Peyssonnelia)* extract, *Cruoriella japonica* (genus *Cruoriella)* extract, *Amphiroa dilatata* (genus *Amphiroa)* extract, *Corallina officinalis* (genus *Corallina)* extract, *Prionitis ramosissima* (genus *Grateloupia)* extract, *Grateloupia livida* (genus *Grateloupia)* extract, *Grateloupia okamurae* (genus *Grateloupia)* extract, *Grateloupia carnosa* (genus *Grateloupia)* extract, *Grateloupia turuturu* (genus *Grateloupia)* extract, *Grateloupia lanceolata* (genus *Grateloupia)* extract, *Grateloupia kurogii* (genus *Grateloupia)* extract, *Prionitis patens* (genus *Prionitis)* extract, *Polyopes polyideoides* (genus *Polyopes)* extract, *Carpopeltis maillardii* (genus *Carpopeltis)* extract, *Prionitis angusta* (genus *Prionitis)* extract, *Carpopeltis prolifera* (genus *Prionitis)* extract, *Prionitis crispata* (genus *Prionitis)* extract, *Prionitis divaricata* (genus *Prionitis)* extract, *Prionitis articulata* (genus *Prionitis)* extract, *Prionitis cornea* (genus *Prionitis)* extract, *Prionitis elata* (genus *Prionitis)* extract, *Prionitis ramosissima* (genus *Prionitis)* extract, *Cryptonemia schmitziana* (genus *Cryptonemia)* extract, *Schimmelmannia plumosa* (genus *Schimmelmannia)* extract, *Tichocarpus crinitus* (genus *Tichocarpus)* extract, *Callophyllis okamurae* (genus *Callophyllis*) extract, *Kallymenia sessilis* (genus *Kallymcnia*) extract, *Kallymenia sagamiana* (genus *Kallymcnia*) extract, *Kallymenia callophylloides* (genus *Kallymcnia*) extract, *Schmitzia japonica* (genus *Schmitzia*) extract, *Nemastoma nakamurae* (genus *Nemastoma*) extract, *Nemastoma lancifolia* (genus *Nemastoma*) extract, *Platoma izunosimensis* (genus *Platoma*) extract, *Schizymenia dubyi* (genus *Schizymenia*) extract, *Halarachnion latissimum* (genus *Halarachnion*) extract, *Sebdenia yamdae* (genus *Sebdenia*) extract, *Catenela caespitosa* (genus *Catenela*) extract, *Phacelocarpus japonicus* (genus *Phacelocarpus*) extract, *Caulacanthus ustulatus* (genus *Caulacanthus*) extract, *Sarcodia ceylanica* (genus *Sarcodia*) extract, *Gracilaria incurvata* (genus *Gracilaria*) extract, *Gracilaria rhodocaudata* (genus *Gracilaria*) extract, *Gracilaria salicornia* (genus *Gracilaria*) extract, *Gracilaria srilankia* (genus *Gracilaria*) extract, *Gracilaria sublittoralis* (genus *Gracilaria*) extract, *Gracilaria ieillardii* (genus *Gracilaria)* extract, *Gracilaria edulis* (genus *Gracilaria)* extract, *Gracilaria eucheumoides* (genus *Gracilaria)* extract, *Gracilaria lemaneiformis* (genus *Gracilaria)* extract, *Gracilaria punctata* (genus *Gracilaria)* extract, *Gracilaria* arcuata (genus *Gracilaria*) extract, *Gracilaria blodgettii* (genus *Gracilaria*) extract, *Gracilaria coronopifolia* (genus *Gracilaria*) extract, *Gracilaria cuneifolia* (genus *Gracilaria*) extract, *Gracilaria salicornia* (genus *Gracilaria*) extract, *Tylotus lichenoides* (genus *Tylotus*) extract, *Ahnfeltia plicata* (genus *Ahnfeltiopsis*) extract, *Ahnfeltiopsis consinna* (genus *Ahnfeltiopsis*) extract, *Stenogramma interrupta* (genus *Stenogramma*) extract, *Chondrus yendoi* (genus *Chondrus)* extract, *Gloiocladia japonica* (genus *Gloiocladia*) extract, *Fauchea spinulosa* (genus *Fauchea)* extract, *Fauchea stipitata* (genus *Fauchea*) extract, Chrysymenia dwrightii (genus *Chrysymenia*) extract, *Chrysymenia okamurae* (genus *Chrysymenia*) extract, *Coelarthrum opuntia* (genus *Coelarthrum*) extract, *Chamaebotrys oergesenii* (genus *Coelarthrum*) extract, *Botryocladia leptopoda* (genus *Botryocladia*) extract, *Cryptarachne polyglandulosa* (genus *Cryptarachne*) extract, *Rhodymenia intricata* (genus *Rhodymenia*) extract, *Rhodymenia pertusa* (genus *Rhodymenia*) extract, *Webberella micans* (genus *Webberella*) extract, *Halosaccion saccatum* (genus *Halosaccion*) extract, *Champia bifida* (genus *Champia*) extract, *Champia expansa* (genus *Champia*) extract, *Campylaephora hypnaeoides* (genus *Campylaephora*) extract, *Campylaephora crassa* (genus *Campylaephora*) extract, *Herpochondria elegans* (genus *Herpochondria*) extract, *Reinboldiella schmitziana* (genus *Reinboldiella*) extract, *Marionella schmitziana* extract, *Congregatocarpus pacificus* extract, *Neoholmesia japonica* extract, *Sorella repens* (genus *Sorella*) extract, *Nienburgia japonica* (genus *Nienburgia*) extract, *Neohypophyllum middendorfii* (genus *Neohypophyllum*) extract, *Myriogramme polyneura* (genus *Myriogramme*) extract, *Hideophyllum yezoense* (genus *Myriogramme*) extract, *Acrosorium venulosum* (genus *Acrosorium*) extract, *Acrosorium flabellatum* (genus *Acrosorium*) extract, *Acrosorium polyneurum* (genus *Acrosorium*) extract, *Acrosorium yendoi* (genus *Acrosorium*) extract, *Hymenena tenuis* (genus *Hymenena*) extract, *Martensia fragilis* (genus *Martensia*) extract, *Caloglossa continua* (genux *Caloglossa*) extract, *Dasya sessilis* (genus *Dasya*) extract, *Heterosiphonia japonica* (genus *Heterosiphonia*) extract, *Heterosiphonia pulchra* (genus *Heterosiphonia*) extract, *Rhodoptilum plumosum* (genus *Rhodoptilum*) extract, *Digenea simplex* (genus *Digenea*) extract, *Pterosiphonia pinnulata* (genus *Pterosiphonia*) extract, *Kintarosiphonia fibrillosa* (genus *Pterosiphonia*) extract, *Symphyocladia marchantioides* (genus *Symphyocladia*) extract, *Symphyocladia latiuscula* (genus *Symphyocladia*) extract, *Symphyocladia linearis* (genus *Symphyocladia*) extract, *Herposiphonia suodisticha* (genus *Herposiphonia*) extract, *Herposiphonia subdisticha* (genus *Herposiphonia*) extract, *Amansia glomerata* (genus *Melanamansia*) extract, *Melanamansia japonica* (genus *Melanamansia*) extract, *Melanamansia mitsuii* (genus *Melanamansia*) extract, *Enantiocladia okamurai* (genus *Enantiocladia*) extract, *Lenormandiopsis lorenzii* (genus *Lenormandiopsis*) extract, *Neurymenia flaxinifolia* (genus *Neurymenia*) extract, *Cyanidium caldarium* (genus *Cyanidium)* extract, *Nemalionopsis tortuosa* (genus *Nemalionopsis*) extract, and *Thorea okadai* (genus *Thorea)* extract, carophyote extracts such as stonewort extract, *Lamprothamnium succinctum* extract, *Nitellopsis obtusa* (genus *Nitellopsis*) extract, *Lychnothamnus* extract, *Nitella flexilis* var. *flexilis* (genus *Nitella*) extract, *Nitella acuminata* var. *capitulifera* extract, and *Tolypella* extract, yellow algae extract such as *Chromulina* sp. (genus *Chromulina)* extract, plant extracts such as a mixed plant extract (arnica, hypericum, camomile, *Tilia japonica, Equisetum arvense*, yarrow, sage, mallow, and *Calendula officinalis*; arinica, nettle, cucumber, ground ivy, mallow, and eldertree) and mixed fruit extract (lemon, apple, peach, and orange).

An animal, plant, or microbe raw material or another naturally-occurring raw material is arbitrarily selected and processed by the conventional process on the basis of the type of a product to which it is added and the form thereof, such as a process of any combination of those selected from grinding, milling, washing, extraction, decomposition, fermentation or metabolic conversion by microorganism, fractionation, purification, compression, filtration, desiccation, pulverization, granulation, dissolution, sterilization, pH adjustment, deodorization, and depigmentation to produce an animal, plant, or microbe extract to be used as a bioactive component in the present invention.

Of the animal, plant, and microbe extracts to be used as the bioactive components in the present invention, for the plant extract, various parts of a plant to be used for the production can be arbitrarily chosen from, for example, a flower, spica, pericarp, malleolus calyx, fruit, stem, lobe, branch, foliage, trunk, bark, rhizome, root bark, root, seed, or the entire plant. For the animal and microbe extracts, a substance to be used for the production can be arbitrarily chosen from, for example, extracted products from various cells, intracellular organelles, tissues, and organs. Alternatively, any part, cell, tissue, organ, or metabolite from a transgenic or cell fusion product can be also used. Alternatively, the extracts can be obtained by subjecting any part, cell, tissue, organ, and the like to cell culture. For example, cultured cells from the respective tissues (e.g., cultured cells of animal origins, such as embryonic stem cells, fibroblasts, Langerhans cells, macrophases, epidermic cells, and hepatocytes), undifferentiated cells, cells in the middle of differentiation, metabolites thereof, and so on may be also used.

The solvents to be used for the extraction may be suitably selected in consideration of the intended purpose and type of the product to be provided, or the process or the like to be performed later and generally include one or a mixture of two or more selected from: water; lower alcohols such as methanol, ethanol, propyl alcohol, isopropyl alcohol, butanol, and isobutanol, or hydrous lower alcohols; polyalcohols such as propylene glycol, 1,3-butylene glycol, 1,2-butylene glycol, 1,4-butylene glycol, 1,5-pentane diol, 1,2-pentane diol, 1,3-pentane diol, 1,4-pentane diol, 1,3,5-pentane triol, glycerin, and polyethylene glycol (molecular weight of 100 to 100,000), or hydrous polyalcohol; various organic solvents such as acetone, ethyl acetate, diethyl ether, dimethyl ether, ethyl methyl ether, dioxane, acetonitrile, xylene, benzene, chloroform, carbon tetrachloride, phenol, and toluene; acids adjusted to appropriate normality (e.g., hydrochloric acid, sulfuric acid, nitric acid, phosphoric acid, formic acid, and acetic acid); and alkalis (e.g., sodium hydroxide, potassium hydroxide, calcium hydroxide, and ammonia). However, when the presence of an organic solvent is not preferable in accordance with the intended use, it is possible to use a method in which water may be used alone or extraction may be performed using a high volatile solvent which can be easily removed after the extraction, followed by dissolving into purified water or the like after solvent removal.

With regard to the extraction method, in consideration of a raw material, the type of a solvent, and so on to be used, extraction temperature, an extraction time period, and a usage ratio of the solvent to the raw material can be arbitrarily defined, respectively. The extraction temperature, which can be arbitrarily defined within the range of -4°C to 100°C in consideration of the nature of the solvent and so on, is preferably in the range of approximately 10°C to 40°C in terms of the stability of components contained in the raw material. In addition, the usage ratio of the solvent to the raw material can be arbitrarily defined within the range of 4 : 1 to 1 : 100 (raw material : solvent in weight ratio), and is particularly preferably in the range of 1 : 1 to 1 : 10 (raw material : solvent in weight ratio).

Examples of the decomposition mainly include acidic decomposition, alkali decomposition, enzymatic decomposition, and high temperature and high pressure decomposition. In the acidic decomposition, it is preferable to use any of inorganic acids such as hydrochloric acid, sulfuric acid, nitric acid, phosphoric acid, acetic acid, formic acid, oxalic acid, hydrogen bromide, perchloric acid, and periodic acid, or organic acids. In the alkali decomposition, it is preferable to use any of those including sodium hydroxide, potassium hydroxide, calcium hydroxide, ammonium hydroxide, barium hydroxide, sodium carbonate, ammonium carbonate, calcium carbonate, magnesium hydroxide, and sodium silicate. In the acidic or alkali decomposition, the concentration of an acid or alkali, reaction time, reaction temperature, and so on can be arbitrarily defined for a raw material of interest.

In the enzymatic decomposition, in particular, it is preferable to use an enzyme having a function of decomposing a protein, polysaccaride, lipid, or a combination thereof having a function important to a cell or tissue structure or the like. Examples of the enzyme include: proteolytic enzymes (proteases) such as amino peptidase, dipeptidase, dipeptidyl peptidase, tripeptidyl peptidase, carboxypeptidase, serine protease, trypsin, chymotrypsin, cysteine protease, thiol protease, papain, aspartate endopeptidase, metallo endopeptidase, promeline, thermolysin, pronase, pepsin, rennin, pancreatin, chymopapain, ficin, collagenase, and elastase; polysaccharide catabolic enzymes such as amylase, Taka-amylase, cellulase, hemicellulase, pectinase, polygalacturonase, dextranase, and planase; and cell-wall digesting enzymes such as hen egg-white lysozyme, human lysozyme, papaya lysozyme, turnip lysozyme, barley lysozyme, zymolyase, lysozyme chloride, glucanase, glucuronidase, chitinase, and chitosanase. In the enzymatic decomposition, the concentration of an enzyme, reaction time, reaction temperature, pH of the solution, and so on can be arbitrarily defined. In particular, it is preferable to define them so that they correspond to optimal levels of an enzyme to be used.

Microbial fermentation or metabolic conversion can be performed by inoculating at least one microorganism to a plant or animal raw material or a processing product thereof provided as a substrate to grow the microorganism. The inoculation of microorganism to the substrate may be carried out by: a method involving directly adding the microorganism to the substrate; adsorbing microorganism on such a carrier as alginate, polyvinyl, gelatin, etc., followed by adding it in the form of fine beads constructed of for example, microorganisms and carrier; or using the microorganism immobilized on the tube wall of a bioreactor. In the microbial fermentation or metabolic conversion, the microorganisms to be used can be defined as but not particularly limited to, those other than pathogenic microorganisms, which typically represent remarkable toxicity to the living body. Examples of a microorganism to be used and to be classified as yeast include the genus *Aciculoconidium,* the genus *Actonia,* the genus *Aessosporon,* the genus *Ambrosiozyma,* the genus *Amphierna,* the genus *Anthomyces,* the genus *Apiotrichum,* the genus *Arthroascus,* the genus *Arxula,* the genus *Ascotrichosporon, Ashbia,* the genus *Ashbya,* the genus *Asporomyces, Atelosaccharomyces,* the genus *Azymocandida,* the genus *Azymohansenula,* the genus *Azymomyces,* the genus *Azymoprocandida,* the genus *Babjevia,* the genus *allistosporomyces,* the genus *Basidiotrichosporon,* the genus *Bensingtonia,* the genus *Blastobotrys,* the genus *Blastodendrion,* the genus *Blastoderma,* the genus *Blastoschizomyces,* the genus *Botryoascus,* the genus *Botryozyma,* the genus *Brettanomyces* (such as *Brettanomyces bruxellensis* or *Brettanomyces anomalus*), the genus *Bullera,* the genus *Bulleromyces,* the genus *Candida* (*such as Candida albicans, Candida amylolenta, Candida anomala, Candida boidinii, Candida entomaea, Candida etchellsii, Candida famata, Candida fermentati, Candida guilliermondii, Candida halophila, Candida intermedia, Candida krusei, Candida lactosa, Candida lipolytica, Candida mogii, Candida parapsilosis, Candida sake, Candida tropicalis, Candida versatilis,* or *Candida vulgaris*), the genus *Castellania,* the genus *Chlamydozyma,* the genus *Chromotorula,* the genus *Citeromyces,* the genus *Cladosporium,* the genus *Clavispora,* the genus *Crebrothecium,* the genus *Cryptococcus,* the genus *Debaryomyces* (such as *Debaryomyces delbrueckii, Debaryomyces halotolerans,* or *Debaryomyces hansenii*), the genus *Debaryozyma,* the genus *Dekkera,* the genus *Dekkeromyces,* the genus *Dematium,* the genus *Dipodascus,* the genus *Eeniella,* the genus *Enantiothamnus,* the genus *Endoblastoderma,* the genus *Endoblastomyces,* the genus *Endomyces,* the genus *Endomycopsis,* the genus *Endyllium,* the genus *Entelexis,* the genus *Eremascus,* the genus *Eremothecium,* the genus *Eutorula,* the genus *Eutorulopsis,* the genus *Fabospora,* the genus *Fellomyces,* the genus *Fermentotrichon,* the genus *Filobasidium,* the genus *Galactomyces,* the genus *Geotrichoides,* the genus *Geotrichum,* the genus *Guilliermondella,* the genus *Hanseniaspora,* the genus *Hansenula* (such as *Hansenula anomala, Hansenula kluyveri, Hansenula miso, Hansenula polymorpha,* or *Hansenula wickerhamii*), the genus *Hypomyces,* the genus *Issatchenkia,* the genus *Kloeckera* (such as *Kloeckera brevis, Kloeckera fluorescens,* or *Kloeckera japonica*), the genus *Kloeckeraspora,* the genus *Kluyveromyces* (such as *Kluyveromyces bulgaricus, Kluyveromyces marxianus,* or *Kluyveromyces thermotolerans*), the genus *Kockovaella,* the genus *Kurtzmanomyces,* the genus *Leucosporidium,* the genus *Lipomyces,* the genus *Magnusiomyces,* the genus *Metschnikowia,* the genus *Microanthomyces,* the genus *Monilia,* the genus *Monospora,* the genus *Monosporella,* the genus *Mrakia,* the genus *Myceloblastanon,* the genus *Mycocandida,* the genus *Mycoderma,* the genus *Mycotorula,* the genus *Mycokluyveria,* the genus *Mycotoruloides,* the genus *Myxozyma,* the genus *Nadsonia,* the genus *Nectaromyces,* the genus *Nematospora,* the genus *Octosporomyces,* the genus *Ogataea,* the genus *Oidium,* the genus *Oospora,* the genus *Oosporidium,* the genus *Pachysolen,* the genus *Parasaccharomyces,* the genus *Paratorulopsis,* the genus *Parendomyces,* the genus *Petasospora,* the genus *Pichia* (such as *Pichia amylophila, Pichia farinose, Pichia guilliermondii, Pichia membranifaciens,* or *Pichia mogii*) the genus *Pityrosporum,* the genus *Procandida,* the genus *Procandida,* the genus *Prosporobolomyces,* the genus *Proteomyces,* the genus *Pseudohansenula,* the genus *Pseudomonilia,* the genus *Pseudomycoderma,* the genus *Pseudosaccharomyces,* the genus *Pseudozyma,* the genus *Rhodomyces,* the genus *Rhodosporidium,* the genus *Rhodotorula,* the genus *Saccharomyces* (such as *Saccharomyces aceti, Saccharomyces cerasi, Saccharomyces cerevisiae, Saccharomyces exiguus, Saccharomyces unisporus,* or *Saccharomyces fibuligera*), the genus *Saccharomycodes,* the genus *Saccharomycopsis,* the genus *Saturnispora,* the genus *Schizoblastosporion,* the genus *Schizosaccharomyces,* the genus *Schwanniomyces,* the genus *Selenotila,* the genus *Selenozyma,* the genus *Smithiozyma*, the genus *Sporidiobolus,* the genus *Sporobolomyces,* the genus *Sporothrix,* the genus *Stephanoascus,* the genus *Sterigmatomyces,* the genus *Sympodiomyces,* the genus *Syringospora,* the genus *Tetrapisispora,* the genus Torula, the genus *Torulaspora,* the genus *Torulopsis,* the genus *Trichosporon,* the genus *Udeniomyces,* the genus *Vanrija*, the genus *Waltomyces,* the genus *Willia,* the genus *Williopsis,* the genus *Wingea,* the genus *Xanthophyllomyces,* the genus *Yamadazyma,* the genus Zendera, the genus *Zygofabospora,* the genus *Zygopichia,* the genus *Zygohansenula,* the genus *Zygorenospora,* the genus *Zygosaccharomyces,* the genus *Zymodebaryomyces,* and the genus *Zymonema.* Examples of the microorganism to be classified as a bacterium include the genus Acetobacter (such as *Acetobacter aceti*), the genus *Achromobacter,* the genus *Acidianus,* the genus *Acidobacterium,* the genus *Acidithiobacillus,* the genus *Acrocarpospora,* the genus *Actinoalloteichus,* the genus *Actinocorallia,* the genus *Actinokineospora,* the genus *Actinomadura,* the genus *Actinoplanes,* the genus *Actinopolyspora*, the genus *Actinosynnema,* the genus *Aerococcus,* the genus *Aeromicrobium,* the genus *Agrobacterium,* the genus *Agromyces,* the genus *Ahrensia,* the genus *Alcaligenes,* the genus *Alicyclobacillus*, the genus *Alloiococcus,* the genus *Alteromonas,* the genus *Amorphosporangium,* the genus *Ampullariella,* the genus *Amycolata,* the genus *Amycolatopsis,* the genus *Aquaspirillum,* the genus *Arcanobacterium,* the genus *Arthrobacter,* the genus *Aureobacterium,* the genus *Azotobacter,* the genus *Bacteroides,* the genus *Beneckea,* the genus *Bifidobacterium* (such as *Bifidobacterium bifidum, Bifidobacterium longum, Bifidobacterium breve,* or *Bifidobacterium infantis*), the genus *Brachybacterium,* the genus *Brevibacillus,* the genus *Brevibacterium,* the genus *Brevundimonas,* the genus *Burkholderia,* the genus *Carnobacterium,* the genus *Catellatospora,* the genus *Cellulomonas,* the genus *Chainia,* the genus *Chromobacterium,* the genus *Chryseobacterium,* the genus *Citrobacter,* the genus *Clavibacter,* the genus *Corynebacterium,* the genus *Couchioplanes,* the genus *Cryptosporangium,* the genus *Curtobacterium,* the genus *Dactylosporangium,* the genus *Deinococcus,* the genus *Delftia,* the genus *Demetria,* the genus *Dermacoccus,* the genus *Dermatophilus,* the genus *Elytrosporangium,* the genus *Enterobacter,* the genus *Erwinia,* the genus *Escherichia,* the genus *Eubacterium,* the genus *Excellospora,* the genus *Exiguobacterium,* the genus *Faenia,* the genus *Flammeovirga,* the genus *Flavobacterium,* the genus *Flexibacter,* the genus *Geodermatophilus,* the genus *Globicatella,* the genus *Gluconacetobacter,* the genus *Gluconoacetobacter,* the genus *Glycomyces,* the genus *Gordona,* the genus *Gordonia,* the genus *Halobacterium,* the genus *Halococcus,* the genus *Herbidospora,* the genus *Hydrogenophilus,* the genus *Hyphomicrobium,* the genus *Hyphomonas,* the genus *Intrasporangium,* the genus *Janibacter,* the genus *Jonesia,* the genus *Kibdelosporangium,* the genus *Kineococcus,* the genus *Kineosporia,* the genus *Kitasatoa,* the genus *Kitasatospora,* the genus *Kitasatosporia,* the genus *Klebsiella,* the genus *Kocuria,* the genus *Kurthia,* the genus *Lactobacillus* (such as *Lactobacillus rimae, Lactobacillus divergens, Lactobacillus carnis, Lactobacillus piscicola, Lactobacillus acidophilus, Lactobacillus amylophilus, Lactobacillus animalis, Lactobacillus brevis, Lactobacillus casei, Lactobacillus curvatus, Lactobacillus bulgaricus, Lactobacillus delbrueckii, Lactobacillus fermentum, Lactobacillus fructivorans, Lactobacillus fructosus, Lactobacillus helveticus, Lactobacillus hilgardii, Lactobacillus homohiochii, Lactobacillus kefiri, Lactobacillus malefermentans, Lactobacillus murinus, Lactobacillus paracasei,* and *Lactobacillus paracasei* subsp. *tolerans, Lactobacillus parakefiri, Lactobacillus pentosus, Lactobacillus plantarum, Lactobacillus reuteri, Lactobacillus* rhamnosus, *Lactobacillus sakei, Lactobacillus confusus, Lactobacillus virideiscens, Lactobacillus johnsonii, Lactobacillus viscosus, Lactobacillus bunchneri*, *Lactobacillus fermentatae, Lactobacillus acidophilaerogenes, Lactobacillus leichmannii, Lactobacillus gasseri, Lactobacillus bifidus, Lactobacillus jugurt, Lactobacillus caucasicus, Lactobacillus arabinosus, Lactobacillus kunkeei, Lactobacillus nagelii, Lactobacillus fornicalis, Lactobacillus pentoaceticus, Lactobacillus xylosus,* or *Lactobacillus minutus*), the genus Leuconostoc (such as *Leuconostoc lactis, Leuconostoc dextranicum, Leuconostoc mesenteroides, Leuconostoc oenos, Leuconostoc paramesenteroides, Leuconostoc cremoris, or Leuconostoc citrovorum*), the genus *Listonella,* the genus *Lucibacterium,* the genus *Luteococcus,* the genus *Magnetospirillum,* the genus *Marinilabilia,* the genus *Marinospirillum,* the genus *Mesorhizobium,* the genus *Metallosphaera,* the genus *Methylobacterium,* the genus *Microbispora,* the genus *Micrococcus,* the genus *Microellobosporia,* the genus *Micromonospora,* the genus *Mycobacterium,* the genus *Mycoplasma,* the genus *Nocardia,* the genus *Nocardioides,* the genus *Nonomuraea*, the genus *Nonomuria,* the genus *Oceanospirillum,* the genus *Ochrobactrum,* the genus *Oerskovia,* the genus *Oligella,* the genus *Paenibacillus,* the genus *Pediococcus* (such as *Pediococcus halophilis, Pediococcus acidilactis, Pediococcus cerevisiae, Pediococcus pentosaceus,* or *Pediococcus urinae-equi*), the genus *Pedobacter,* the genus *Peptococcus,* the genus *Peptostreptococcus,* the genus *Pilimelia,* the genus *Pimelobacter,* the genus *Planobispora,* the genus *Planococcus,* the genus *Planomonospora,* the genus *Prevotella,* the genus *Propionibacterium,* the genus *Proteus,* the genus *Protomonas,* the genus *Pseudomonas,* the genus *Pseudonocardia,* the genus *Rahnella*, the genus *Rarobacter,* the genus *Rathayibacter,* the genus *Rhizobium,* the genus *Rhizomonas,* the genus *Rhodobacter,* the genus *Rhodococcus,* the genus *Rhodopseudomonas,* the genus *Rhodospirillum,* the genus *Rothia*, the genus *Rubrobacter,* the genus *Ruegeria,* the genus *Saccharomonospora,* the genus *Saccharothrix,* the genus *Serratia,* the genus *Sinorhizobium,* the genus *Sphingobacterium,* the genus *Sphingomonas,* the genus *Sporolactobacillus,* the genus *Stenotrophomonas,* the genus *Streptoalloteichus,* the genus *Streptococcus* (such as *Streptococcus durans, Streptococcus faecalis, Streptococcus faecium, Streptococcus bovis, Streptococcus equinus, Streptococcus mutans, Streptococcus salivarius, Streptococcus thermophilus, Streptococcus agalactiae, Streptococcus mitis, Streptococcus pyogenes, Streptococcus pneumoniae, Streptococcus lactis, Streptococcus dysgalactiae, Streptococcus sanguis, Streptococcus acidominimus, Streptococcus avium, Streptococcus uberis, Streptococcus cremoris,* or *Streptococcus diacetilactis*), the genus *Streptomyces,* the genus *Streptosporangium,* the genus *Streptoverticillium,* the genus *Terrabacter,* the genus *Thermoactinomyces,* the genus *Thermobifida,* the genus *Thermobispora,* the genus *Thermocrispum,* the genus *Thermomonospora,* the genus *Thermoplasma,* the genus *Thiobacillus,* the genus *Thiomonas,* the genus *Thiosphaera,* the genus *Weissella,* the genus *Xanthobacter,* the genus *Xanthomonas,* and the genus *Zymomonas.* Examples of the microorganism to be classified as ascomycetes, basidiomycetes, or deuteromycetes except yeast include the genus *Allomyces,* the genus *Amoebidium,* the genus *Amorphotheca,* the genus *Arthroderma,* the genus *Ascoidea,* the genus *Ascobolus,* the genus *Ascodesmis,* the genus *Aspergillus,* the genus *Aureobasidium,* the genus *Botryosphaeria,* the genus *Botryotinia*, the genus *Brachybasidium,* the genus *Byssochlamys,* the genus *Capnodium,* the genus *Ceratocystis,* the genus *Ceratomyces,* the genus *Chaetomium,* the genus *Chrysella*, the genus *Chrysocelia,* the genus *Chytridium,* the genus *Claviceps,* the genus *Cochliobolus,* the genus *Coemansia,* the genus *Coleosporium,* the genus *Coniochaetidium,* the genus *Cordyceps,* the genus *Cronartium,* the genus *Cyttaria*, the genus *Dasyspora,* the genus *Derchslera,* the genus *Dichomyces,* the genus *Dothidea*, the genus *Endogone,* the genus *Entomophthora,* the genus *Emericella,* the genus *Eupenicillium,* the genus *Eurotium,* the genus *Exobasidium,* the genus *Gibberella*, the genus *Glomus,* the genus *Graphiola*, the genus *Gymnoascus,* the genus *Harpella*, the genus *Helicomyces,* the genus *Helvella,* the genus *Hemicarpenteles,* the genus *Hyphochytrium,* the genus *Hypocrea,* the genus *Laboulbenia,* the genus *Labyrinthula,* the genus *Leptosphaeria,* the genus *Leptosphaerulina,* the genus *Lophodermium,* the genus *Melanotaenium,* the genus *Microascus,* the genus *Microstroma,* the genus *Medeolaria,* the genus *Melampsora,* the genus *Melamsporella,* the genus *Morchella,* the genus *Monascus,* the genus *Monilinia,* the genus *Monoblephalis,* the genus *Mycospharella,* the genus *Nannizzia,* the genus *Nectria,* the genus *Neolecta,* the genus *Neurospora,* the genus *Nodulosphaeria,* the genus *Olpidium,* the genus *Peziza,* the genus *Penicillium,* the genus *Perenospora,* the genus *Pestalotiopsis,* the genus *Phomopsis,* the genus *Phragmidiella,* the genus *Pneumocystis,* the genus *Preussia,* the genus *Pleospora,* the genus *Puccinia,* the genus *Pythium,* the genus *Ravenelia,* the genus *Rickia,* the genus *Rhinocladiella,* the genus *Rhizidiomyces,* the genus *Rhizoctonia,* the genus *Sclerocleista,* the genus *Saprolegnia,* the genus *Satioella,* the genus *Sclerotinia,* the genus *Sclerotum,* the genus *Septobasidium,* the genus *Sordaria,* the genus *Sporidiobolus,* the genus *Stibella,* the genus *Stigmatomyces,* the genus *Sydowiella,* the genus *Talaromayces,* the genus *Taphrina,* the genus *Thraustochytrium,* the genus *Tolyposporium,* the genus *Trichoglossum,* the genus *Trichoma,* the genus *Ustilago,* the genus *Verticillium,* and the genus *Xylaria.*

In the microbial fermentation or metabolic conversion, in addition to various kinds of raw materials including plant raw materials, animal raw materials, and naturally-occurring raw materials, various additional compounds and the like may be added to the raw materials to regulate or activate the growth or metabolism of the microorganism and further to induce a specific biosynthesis or decomposition pathway. Examples of those compounds to be added include: compounds containing, as carbon sources, carbohydrates such as glucose, fructose, galactose, sucrose, maltose, mannose, lactose, glycerol, and starch, hydrocarbons such as ethane, methane, propane, and butane, and aliphatic acids such as formic acid, acetic acid, propionic acid, lauric acid, palmitic acid, oleic acid, linolic acid, and linolenic acid; compounds containing, as nitrogen sources, ammonium salts such as ammonium sulfate, ammonium hydrochloride, and ammonium phosphate, urea, uric acid, or amino acids; and compounds containing vitamins, potassium, calcium, magnesium, sodium, sulfur, phosphorus, and chlorine, which are required in various microorganisms, and a compound containing iron, copper, zinc, cobalt, nickel, boron, manganese, molybdenum, tin, selenium, silicon, arsenic, vanadium, chromium, and fluorine. Furthermore, various factors such as optimal temperature, content of oxygen supplied, pH, and pressure which affect the growth or activation of metabolism of various microorganisms can be arbitrarily defined in response to the inherent properties of various microorganisms. For the temperature, for example, it can be arbitrarily defined within the range of 10°C to 50°C, and the pH can be also arbitrarily defined within the range of 1 to 14.

In the fractionation or purification, any technology well-known in the art can be appropriately used. In addition to the solvent extraction, for example, fractionation with liquid chromatography (such as ion-exchange chromatography, ion-exclusion chromatography, affinity chromatography, gel-filtration chromatography, size-exclusion chromatography, hydrophilic adsorption chromatography, hydrophobic adsorption chromatography, or ligand exchange chromatography); dialysis through a semi-permeable membrane, crystallization or recrystallization of components, filtration with filter paper, membrane filter, ultrafilter, activated charcoal, and filter agent, fractionation precipitation methods such as centrifugation and the application thereof, density gradient separation methods such as a red zonal centrifugation method and a density gradient sedimentation equilibrium method, and the like can be used, respectively.

Examples of preparation-regulating ingredients such as a colorant, surfactant, fragrance, and vehicle include: inorganic pigments such as silicic anhydride, magnesium silicate, bentonite, mica-titanium, bismuth oxychloride, zirconium oxide, magnesium oxide, zinc oxide, titanium oxide, calcium carbonate, magnesium carbonate, yellow iron oxide, red iron oxide, black iron oxide, ultramarine, chrome oxide, chrome hydroxide, carbon black, and calamine; inorganic reducers such as a hydrogen peroxide solution, sodium persulfate, ammonium persulfate, sodium perborate, urea peroxide, sodium percarbonate, sodium peroxytripolyphosphate, sodium bromate, potassium bromate, sodium peroxypyrophosphate, sodium peroxy-o-phosphate, a sodium silicate-hydrogen peroxide adduct, a sodium sulfate-hydrogen peroxide adduct, a sodium chloride-hydrogen peroxide adduct, a β-tyrosinase enzyme solution, a mushroom extract, strontium sulfate, sodium sulfide, barium sulfide, and calcium sulfide; redox agents such as thioglycolic acids and salts thereof (calcium thioglycollate, sodium thioglycollate, lithium thioglycollate, magnesium thioglycollate, and strontium thioglycollate); and dye agents such as 5-amino-o-cresol, 2-amino-4-nitrophenol, 2-amino-5-nitrophenol, 1-amino-4-methylaminoanthraquinone, 3,3'-iminodiphenol, 2,4-diaminophenoxyethanol hydrochloride, 2,4-diaminophenol hydrochloride, toluene-2,5-diamine hydrochloride, nitro-p-phenylenediamine hydrochloride, p-phenylenediamine hydrochloride, N-phenyl-p-phenylenediamine hydrochloride, m-phenylenediamine hydrochloride, o-aminophenol, N-phenyl-p-phenylenediamine acetate, 1,4-diaminoanthraquinone, 2,6-diaminopyridine, 1,5-dihydroxynaphthalene, toluene-2,5-diamine, toluene-3,4-diamine, nitro-p-phenylenediamine, p-aminophenol, p-nitro-o-phenylenediamine, p-phenylenediamine, p-methylaminophenol, picramic acid, sodium picramate, N,N'-bis(4-aminophenyl)-2,5-diamino-1,4-quinonediimine, 5-(2-hydroxyethylamino)-2-methylphenol, N-phenyl-p-phenylenediamine, m-aminophenol, m-phenylenediamine, 5-amino-o-cresol sulfate, 2-amino-5-nitrophenol sulfate, o-aminophenol sulfate, o-chloro-p-phenylenediamine sulfate, 4,4'-diaminodiphenylamine sulfate, 2,4-diaminophenol sulfate, toluene-2,5-diamine sulfate, nitro-p-phenylenediamine sulfate, p-aminophenol sulfate, p-nitro-o-phenylenediamine sulfate, p-nitro-m-phenylenediamine sulfate, p-phenylenediamine sulfate, p-methylaminophenol sulfate, m-aminophenol sulfate, m-phenylenediamine sulfate, catechol, diphenylamine, α-naphthol, hydroquinone, phloroglucin, 2-hydroxy-5-nitro-2',4'-diaminoazobenzene-5'-sodium sulfonate, and hematein.

Examples of the adjustment components further include: natural animal fragrances such as musk, civet, castor, and amber grease; plant fragrances such as anise essential oil, angelica essential oil, ylang-ylang essential oil, orris essential oil, fennel essential oil, orange essential oil, cananga essential oil, caraway essential oil, cardamom essential oil, guaiac wood essential oil, cumin essential oil, *Lindera umbellata* essential oil, cinnaman essential oil, cinnamon essential oil, geranium essential oil, copaiba balsam essential oil, coriander essential oil, perilla essential oil, cedar wood essential oil, citronella essential oil, jasmine essential oil, ginger-grass essential oil, Japanese cedar essential oil, spearmint essential oil, peppermint essential oil, star anise essential oil, tuberose essential oil, clove essential oil, neroli essential oil, gauktheria essential oil, tolu balsam essential oil, patchouli essential oil, rose essential oil, palmarosa essential oil, hinoki essential oil, hiba essential oil, santal essential oil, petitgrain essential oil, bay essential oil, vetivert essential oil, bergamot essential oil, peru balsam essential oil, bois de rose Oil, ho-leaf essential oil, mandarin essential oil, eucalyptus essential oil, lime essential oil, lavender essential oil, linaloe essential oil, lemongrass essential oil, lemon essential oil, rosemary essential oil, and Japanese mint essential oil; fragrances such as other synthetic fragrances; natural alcohols such as ethanol, isopropanol, laurylalcohol, cetanol, stearylalcohol, cetostearylalcohol, oleylalcohol, lanolinealcohol, cholesterol, phytosterol, and phenoxyethanol; synthetic alcohols such as aminomethylpropanol, 2-hexyldecanol, isostearylalcohol, and 2-octyldodecanol; alcohols such as behenyl alcohol and cetyl alcohol; esters such as isopropylmyristate, isopropylpalmitate, palmitate/myristate, butylstearate, hexyllaurate, glycerinlaurate, glyceryl behenate/eicosadioate, myristylmyristate, oleyloleate, decyloleate, octyldodecyl myristate, hexyldecyldimethyl octanoate, diisobutyladipate, 2-hexyldecyladipate, diheptylundecyladipate, N-alkylglycol monoisostearate, ethyleneglycol distearate, glycerin monostearate, glycerylmonostearate (glycerin monostearate), hexaglyceryl tristearate, isocetylisostearate, trimethyrolpropane triisostearate, ethyleneglycol diethylhexanoate, neopentylglycol diethylhexanoate, neopentylglycol diisooctanoate, cetyl 2-ethylhexanoate, trimethylolpropane triethylhexanoate, pentaerythritol tetraethylhexanoate, cetyloctanoate, octyldodecyl gum, cetyllactate, myristyllactate, diethylphthalate, dibutylphthalate, acetylated lanoline, ethyleneglycol distearate, ethyleneglycol monostearate, propyleneglycol monostearate, and propyleneglycol dioleate; and metalsoaps such as aluminum stearate, magnesium stearate, zinc stearate, calcium stearate, zinc palmitate, magnesium myristate, zinc laurate, and zinc undecylate.

Examples of the adjustment components furthermore include: pigments/colorants such as 5-amino-o-cresol, o-aminophenol, m-aminophenol, p-aminophenol, 2,6-diaminopyridine, 5-(2-hydroxyethylamino)-2-methylphenol, N,N-bis(β-hydroxyl)-p-phenylenediamine/sulfate, N,N-bis(2-hydroxyethyl)-p-phenylenediamine, p-nitro-o-phenylenediamine, p-phenylenediamine, m-phenylenediamine, N-phenyl-p-phenylenediamine, resorcin, hydroquinone, 2-hydroxy-5-nitro-2',4'-diaminoazobenzene/sodium sulfate, toluene-2,5-diamine, 2-(2'-hydroxyethylamino)-5-aminotoluene, N,N-bis(β-hydroxyl)-p-phenyldiamine, N,N-bis(2-hydroxyethyl)-p-phenylenediamine/sulfate, 5-amino-o-cresol/sulfate, p-aminophenol/sulfate, o-chloro-p-phenylenediamine/sulfate, 2-(2'-hydroxyethylamino)-5-aminotoluene/sulfate, 4,4'-diaminodiphenylamine/sulfate, p-methylaminophenol/sulfate, p-phenylenediamine/sulfate, m-phenylenediamine/sulfate, toluene-2,5-diamine/sulfate, 2,4-diaminophenoxyethanol/hydrochloride, toluene-2,5-diamine/hydrochloride, m-phenylenediamine/hydrochloride, 2,4-diaminophenol/hydrochloride, 3,3'-iminodiphenol, p-phenylenediamine/hydrochloride, N-phenyl-p-phenylenediamine/hydrochloride, N-phenyl-p-phenylenediamine/acetate, 1,5-dihydroxynaphthalane, toluene-3,4-diamine, p-methylaminophenol, N,N'-bis(4-aminophenyl)-2,5-diamino-1,4-quinonediimine, o-aminophenol/sulfate, 2,4-diaminophenol/sulfate, m-aminophenol/sulfate, 2-amino-4-nitrophenol, 2-amino-5-nitrophenol, 1-amino-4-methylaminoanthraquinone, nitro-p-phenylenediamine/hydrochloride, 1,4-diaminoanthraquinone, nitro-p-phenylenediamine, picramic acid, sodium picramate, 2-amino-5-nitrophenol/sulfate, resorcinol, nitro-p-phenylenediamine/sulfate, p-nitro-o-phenylenediamine/sulfate, p-nitro-m-phenylenediamine/sulfate, Red No.2, Red No.3, Red No.102, Red No.104 (1), Red No.105 (1), Red No.106, Red No.201, Red No.227, Red No.230 (1), Red No.230 (2), Red No.231, Red No.232, Red No.401, Red No.502, Red No.503, Red No.504, Red No.506, Yellow No.4, Yellow No.5, Yellow No.202 (1), Yellow No.202 (1), Yellow No.202 (2), Yellow No.203, Yellow No.402, Yellow No.403 (1), Yellow No.406, Yellow No.407, Orange No.205, Orange No.207, Orange No.402, Green No.3, Green No.204, Green No.205, Green No.401, Green No.402, Brown No.201 Purple No.401, Blue No.1, Blue No.2, Blue No.202, Blue No.203, Blue No.205, Black No.401, a red cabbage pigment, a red rice pigment a madder pigment, an annatto pigment, a sepia pigment, a turmeric pigment, a scholar pigment, a krill pigment, a persimmon pigment, caramel, gold, silver, a gardenia pigment, a corn pigment, an onion pigment, a tamarind pigment, a spirulina pigment, buckwheat (whole plant) pigment, a cherry pigment, a laver pigment, a hibiscus pigment, grape juice colour, a marigold pigment, a purple potato colour, purple yam colour, lac colour, and rutin.

Examples of the adjustment components still further include: surfactants such as anioic surfactants (alkylcarboxylate, alkylsulfonate, alkylsulfate, and alkylphosphate such as sodium laurylsulfate, potassium laurylsarcosine, sodium C14-α-olefinsulfonate, sodium paraffinsulfonate, and sodium lauroyl-N-methyltaurine), cationic surfactants (an alkylamine salt and alkyl quaternary ammonium salt such as cetyltrimethylammonium bromide, benzalkonium chloride, benzethonium chloride, methylrosanilinium chloride, stearyltrimethylammonium chloride, stearyltrimethylammonium chloride, N-palm oil fatty acid acyl-L-triethanolamine glutamate, lauroylmethyl-β-alaninetriethanolamine salt, dimethylaminopropylamide stearate, triethanolamine cocoylglutamate salt, isopropanolamine laurate, ammonium laurylsulfate, dipolyoxyethylene methylammonium alkylchloride, cationizated polyalkylpolymers, cetyltrimethyl chloride, and dimethyldistearyl chloride), ampholytic surfactants (carboxylic acid type-ampholytic surfactants (amino-type and betaine-type such as dodecylamino propionate), a sulfate-type ampholytic surfactant, a sulfonate-type ampholytic surfactant, and a phosphate-type ampholytic surfactant), non-ionic surfactants (an ether-type non-ionic surfactant, an etherester-type non-ionic surfactant, an ester-type ionic surfactant, a blockpolymer-type non-ionic surfactant, and non-ionic surfactants having nitrogen such as polyoxyethylene lauryl ether (POE(12)laurylether and the like), POE(3)sodium laurylethersulfate, POE(2.5)sodium tridecylether sulfate, POE(15)dodecyl ether, polyoxyethylene (POE) cetyl ether (EO3.0, 2, and 20, 10EO, 6, and 60, and the like), polyoxyethylene nonylphenyl ether, polyoxyethylene octylphenyl ether, polyoxyethylene(20EO)stearyl ether, sorbitan monostearate, sorbitan sesquioleate, palm oil fatty acid sorbitan, glyceride monolaurate, 4-4'dichloro-2-hydroxyphenyl ether, pentaerythritol fatty acid ester, 4-guanidinobutyllauroamide, and the like), and other surfactants (natural surfactants, derivatives of protein hydrolysates, polymer surfactants, surfactants containing titanium/silicon, and fluorocarbonbased surfactants); and treatment agents for hair such as selenium disulfide, an isoquinolinium alkylbromide solution, zinc pyrithione, biphenamine, thiantol, quinine hydrochloride, and strong ammonia water.

Other adjustment components, which can be used in combination, include: hormones; a sequestrant; a pH adjuster; chelators such as EDTA (EDTA-2Na and EDTA-3Na); antivirus and antifungus agents such as paraben, methyl paraben, ethyl paraben, and propyl paraben; refrigerant; stabilizer; emulsifier; animal and plant proteins and digests thereof (urea, for instance); animal and plant polysaccharides and digests thereof; animal and plant glycoproteins and digests thereof; antiflash and antiallergic agents; a therapeutic agent for wounds; refoamer; thickening agents such as xanthan gum, quinceseed gum, and toraja gum; enzyme; purified water (electron water, small clustered water); deodorant; and solvents such as trichlorofluoroethane.

Hereinafter, production examples, test examples, and prescription examples will be provided for further detailed explanation of the present invention. However, the present invention is not limited to these examples at all.

### (Production Example 1)

*Bacillus subtilis* stored in the cold at 4°C was inoculated in a liquid pre-culture medium with 1-loop inoculation and then cultivated at 25°C for 72 hours with shaking at 100 strokes/min. Bacterial cells were collected from the medium by centrifugation after cultivation and then washed with sterilized purified water. The bacterial cells after washing were all added to a liquid medium for the production of γ-polyglutamic acid and then cultivated under the culture conditions of an oxygen-transfer coefficient of 0.009 min⁻¹ at 28°C, pH 8.0, during the cultivation from 0 to 24 hours and then cultivated under the culture conditions of an oxygen-transfer coefficient of 0.007 min⁻¹ at 25°C, pH 6.0, during the cultivation from 24 to 168 hours, while they were kept under aerobic conditions for 7 days in total. After cultivations, the culture medium was filtrated to remove the bacterial cells. To the resulting filtrate added was 99.9% ethanol at a volume corresponding to one half of the total amount of filtrate and the whole was then stirred, thereby obtaining a precipitate. Subsequently, the precipitate thus obtained was further washed with 99.9% ethanol for removal of excess moisture. After washing, the precipitate was dissolved in purified water, thereby obtaining a γ-polyglutamic acid solution. The concentration of the γ-polyglutamic acid solution was adjusted with purified water, to produce a 0.002%(w/v) γ-polyglutamic acid solution.

**[Composition of liquid pre-culture medium]**

| | |
|---|---|
| Glucose | 1.0 w/v% |
| Sucrose | 1.0 w/v% |
| Sodium L-glutamate | 5.0 w/v% |
| Sodium chloride | 0.01 w/v% |
| Magnesium sulfate | 0.005w/v% |
| 7-hydrate | |
| Monopotassium phosphate | 0.5 w/v% |
| Biotin | 0.0001 w/v% |
| Yeast extract | 0.5 w/v% |
| Total amount | 500 mL |
| | (a 100 mL/500 mL-shake flask x 5) |

**[Composition of liquid medium for the production of γ-polyglutamic acid]**

| | |
|---|---|
| Glucose | 1.5 w/v% |
| Sucrose | 1.5 w/v% |
| Sodium L-glutamate | 5.0 w/v% |
| Sodium chloride | 0.02 w/v% |
| Magnesium sulfate | 0.01 w/v% |
| 7-hydrate | |
| Monopotassium phosphate | 0.5 w/v% |
| Biotin | 0.00001 w/v% |
| Yeast extract | 0.2 w/v% |
| Total amount | 10 L (a 15 L-jar fermenter) |

### [Determination of molecular weight of γ-polyglutamic acid obtained in Production Example 1]

The average molecular weight of γ-polyglutamic acid obtained in Production Example 1 was determined to be 520,000. As a procedure for the determination, HPLC analysis with a gel-filtration column was carried out and the molecular weight was then calculated from a calibration curve prepared from a retention time corresponding to the resulting peaks using a known molecular weight marker. The known molecular weight marker used was pullulan having a known molecular weight.

### [Conditions for molecular weight determination]

Column: TSK-GEL GMPWXL
Mobile phase: 50-mM phosphate buffer (pH 6.5)
Flow rate: 0.5 mL/min
Column temperature: 40°C
Detector: ABS 214 nm

### (Test Example 1) Comparative test for improvement effect on hair strength

### [Test method and evaluation method]

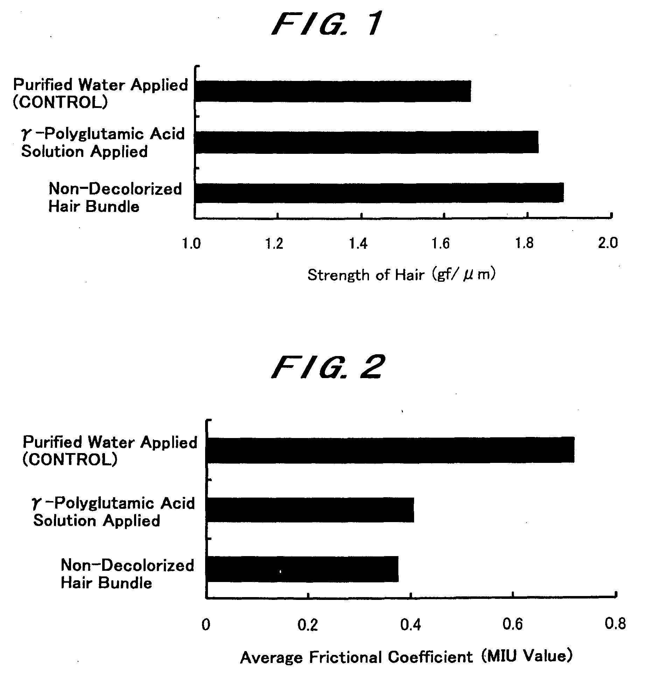

A numerical value, which was obtained by dividing the maximum stress measured at the time of pulling hair provided as a test sample by the diameter of the hair, was defined as an index of hair strength. A bundle of black hair (13 cm in length and 2 g in weight) from a healthy human individual was dipped in a decolorizer (3.0 w/v hydrogen peroxide, 3.0 w/v ammonia aqueous solution) for 30 min, and then washed sufficiently with purified water, followed by standing in a drying machine at 40°C to dry the hair bundle. The same operation was repeated three times in total and the resulting hair bundle was then provided as decolorized hair. The decolorized hair thus obtained was dipped for 5 minutes in the γ-polyglutamic acid solution of Production Example 1, which was adjusted at 40°C, and then washed with purified water sufficiently, followed by standing in a drying machine at 40°C to dry the hair bundle. As a control for comparison, purified water adjusted at 40°C was used instead of the γ-polyglutamic acid solution and then the same operation was carried out. Each of various hair bundles obtained by the above operation and hair bundles without decolorization (non-decolorized hair bundles) was subjected to a rheometer (manufactured by Fudou Kogyo K.K) to determine the maximum stress of each hair and the strength of hair (gf/µm) was then determined by dividing the maximum stress by the diameter of hair. The results are shown in Fig. 1.

### (Results of Test Example 1)

As a result of the measurement of hair strength in each of the hair bundle on which the γ-polyglutamic acid solution of Production Example 1 was applied, the hair bundle as a control on which purified water was applied, and the non-decolorized hair bundle, the hair on which the γ-polyglutamic acid solution of Production Example was applied showed higher hair strength than that of the hair as a control on which purified water was applied, and also showed hair strength similar to that of the non-decolorized hair. The foregoing found that the γ-polyglutamic acid of Production Example 1 has a superior hair strength improvement effect and advantageously acts against the hair strength deterioration of damaged hair.

### (Test Example 2) Comparative test for improvement effect on frictional coefficient of hair

### [Test method and evaluation method]

Various hair bundles (hair bundle on which the γ-polyglutamic acid solution was applied, the hair bundle as a control on which purified water was applied, and non-decolorized hair bundle) obtained in Test Example 1 were also used in the present test in a similar manner, respectively. From the hair bundle on which the γ-polyglutamic acid solution was applied, 20 hairs having almost the same hair diameter were mounted on a slide glass at 1-mm intervals such that their cuticles were aligned in a certain direction. While each of the hairs was kept in straight, both ends of the hair were fixed by an epoxy resin. Then, the average frictional coefficient (MIU) of the fixed hairs was determined using a friction-sensing tester KES-SE (manufactured by Kato-Tech, Co., Ltd.). Then, the hair bundle as a control on which purified water was applied and the non-decolorized hair bundle were also subjected to the same operation to determine the average frictional coefficient of the respective hairs. The results are shown in Fig. 2.

### (Results of Test Example 2)

As a result of determining the average fictional coefficient of each of the hair bundles on which the γ-polyglutamic acid solution of Production Example 1 was applied, the hair bundle as a control on which purified water was applied, and the non-decolorized hair bundle, the hair on which the γ-polyglutamic acid solution of Production Example 1 was applied showed a lower average frictional coefficient than that of the hair as a control on which purified water was applied and showed the average frictional coefficient approximate to that of the non-decolorized hair. The foregoing found that the γ-polyglutamic acid of Production Example 1 has an excellent improvement effect on the frictional force of hair and advantageously acts on an increase in frictional force of damaged hair.

According to the foregoing evaluation results, their corresponding prescription examples will be described below. However, in each of the prescription examples, each product may be one manufactured by the conventional method and only the amount to be mixed will be shown. In addition, the present invention is not limited to these prescription examples.

**(Prescription Example 1) Composition for hair treatment**

| | Mass% |
|---|---|
| Liquid paraffin | 12.0 |
| Vaseline | 12.0 |
| White beeswax | 2.0 |
| Carboxyvinyl polymer | 0.1 |
| Xanthan gum | 0.1 |
| Glycerin | 5.0 |
| γ-Sodium polyglutamate (MW about 520,000) | 10.0 |
| Polyoxyethylene hydrogenated castor oil | 3.0 |
| EDTA-2Na | 0.05 |
| Antiseptic | Adequate amount |
| Perfume | Adequate amount |
| Purified water | Balance |

**(Prescription Example 2) Composition for hair treatment**

| | Mass% |
|---|---|
| 1,3-Butylene glycol | 2.0 |
| Glycerin | 1.0 |
| Stearyl trimethylammonium chloride | 0.5 |
| Methylphenyl polysiloxane | 1.0 |
| Ethanol | 40.0 |
| γ-Sodium polyglutamate (MW about 330,000) | 10.0 |
| Antiseptic | Adequate amount |
| Perfume | Adequate amount |
| Purified water | Balance |

**(Prescription Example 3) Shampoo**

| | Mass% |
|---|---|
| Ammonium lauryl sulfate | 10.0 |
| N-Coconut oil fatty acid acyl-L-glutamic acid triethanolamine | 4.0 |
| 2-Alkyl-N-carboxymethyl-N-hydroxyethyl imidazolinium betaine | 3.0 |
| Lauric acid isopropanol amide | 2.0 |
| Ethylene glycol distearate | 1.0 |
| Undecylenic acid trehalose | 0.5 |
| γ-Sodium polyglutamate (MW about 120,000) | 1.5 |
| Dipotassium glycyrrhizinate | 0.2 |
| Lithospermum root extract | 0.3 |
| Antiseptic | 0.1 |
| Perfume | Adequate amount |
| Purified water | Balance |

**(Prescription Example 4) Shampoo**

| | Mass% |
|---|---|
| POE (3) Sodium lauryl ether sulfate | 4.0 |
| Lauroyl methyl-β-alanine triethanolamine salt | 5.0 |
| Triethanolamine cocoyl glutamate | 5.0 |
| Lauryl amido propyl betaine | 5.0 |
| dodecyl aminopropionate | 1.0 |
| POE hydrogenated castor oil (40) | 2.0 |
| γ-Sodium polyglutamate (MW about 350,000) | 2.0 |
| Pyridoxine hydrochloride | 0.3 |
| Camomile extract | 0.3 |
| Decyl glyceryl glucoside | 1.0 |
| Glycerin laurate | 3.0 |
| Polydimethyl siloxane | 4.0 |
| Cetyl isooctanate | 3.0 |
| Wax | 1.0 |
| Soy-protein hydrolysate | 0.2 |
| Ethanol | 3.0 |
| Dipropylene glycol | 0.5 |
| Sodium chloride | 1.0 |
| Sodium benzoate | 0.3 |
| Paraben | 0.2 |
| Dibutylhydroxytoluene | 0.05 |
| Perfume | Adequate amount |
| Citric acid | 1.5 |
| Purified water | Balance |

**(Prescription Example 5) Shampoo**

| | Mass% |
|---|---|
| POE (2.5) Sodium tridecyl ether sulfate | 10.0 |
| Sodium C14-α-olefin sulfonate | 5.0 |
| Sodium paraffin sulfonate | 1.0 |
| Amido propyl betaine laurate | 2.0 |
| Dodecyl carboxyethyl hydroxyethyl betaine | 1.0 |
| POE (15) dodecylether | 3.0 |
| POE hydrogenated castor oil (40) | 3.0 |
| γ-Sodium polyglutamate (MW about 600,000) | 2.5 |
| Catechin | 0.1 |
| Basil extract | 0.3 |
| Dodecyl glycerin ether | 2.0 |
| Cetyl isooctanate | 1.0 |
| Liquid paraffin | 1.0 |
| Collagen-derived tripeptide | 0.5 |
| Ethanol | 2.0 |
| Sodium chloride | 0.5 |
| Sodium citrate | 0.2 |
| Paraben | 0.2 |
| Dibutylhydroxytoluene | Adequate amount |
| Ethylene glycol distearate | 2.0 |
| Citric acid | 0.8 |
| Purified water | Balance |

**(Prescription Example 6) Shampoo**

| | Mass% |
|---|---|
| Lauroyl sodium N-methyl taurate | 5.0 |
| POE (12) laurylether | 2.0 |
| PEG 400 | 50.0 |
| Glycerin | 10.0 |
| Ethylene glycol distearate | 1.5 |
| Amino denaturated silicone | 0.1 |
| Dimethyl amino propyl amide stearate | 2.0 |
| γ-Sodium polyglutamate (MW about 520,000) | 3.0 |
| ε-Caproic acid | 0.2 |
| Peach leaf extract | 0.5 |
| Menthol | Adequate amount |
| Camphor | Adequate amount |
| Antiseptic | Adequate amount |
| Coloring matter | Adequate amount |
| Perfume | Adequate amount |
| Purified water | 4.0 |
| Ethanol | Balance |

**(Prescription Example 7) Rinse**

| | Mass% |
|---|---|
| Alkyl dipolyoxy ethylene methyl ammonium chloride | 4.0 |
| Cetanol | 2.5 |
| Cationized polyacrylic polymer | 0.3 |
| Glycerin | 3.5 |
| Polyoxyethylene cetyl ether | 0.5 |
| Dimethylpolysiloxane | 2.0 |
| γ-Sodium polyglutamate (MW about 190,000) | 2.0 |
| Trehalose | 0.5 |
| Blackberry lily extract | 0.3 |
| Sodium chloride | 1.0 |
| Ethanol | 3.0 |
| Perfume | Adequate amount |
| Citric acid | 1.3 |
| Purified water | Balance |

**(Prescription Example 8) Rinse**

| | Mass% |
|---|---|
| Betaine-type ampholytic surfactant | 4.0 |
| Cetanol | 2.5 |
| Sorbitol | 5.0 |
| γ-Sodium polyglutamate (MW about 480,000) | 2.5 |
| Tocotrienol nicotinate | 0.3 |
| Rosemary extract | 0.3 |
| Polyoxyethylene cetyl ether (EO 3.0) | 0.5 |
| Carboxymethyl cellulose | 0.8 |
| Ethanol | 5.0 |
| Perfume | Adequate amount |
| Citric acid | 1.0 |
| Purified water | Balance |

**(Prescription Example 9) Rinse**

| | Mass% |
|---|---|
| Stearyl trimethyl ammonium chloride | 3.0 |
| Sphingomyelin | 0.2 |
| Casein hydrolysate | 0.02 |
| γ-Sodium polyglutamate (MW about 360,000) | 3.0 |
| Sodium N-methyl taurate | 0.2 |
| Anthocyanidin | 0.2 |
| Soybean seed extract | 0.5 |
| 2-Octyl dodecanol | 10.0 |
| Cetostearyl alcohol | 2.0 |
| Propylene glycol | 10.0 |
| Citric acid | 0.2 |
| Potassium chloride | 0.3 |
| Perfume | Adequate amount |
| Purified water | Balance |

**(Prescription Example 10) Treatment**

| | Mass% |
|---|---|
| 1,3-Butanediol | 5.0 |
| Nemacystus extract | 0.2 |
| Methyl paraben | 0.2 |
| Phenoxyethanol | 0.3 |
| Cetyl trimethyl chloride | 2.0 |
| Dimethyl distearyl chloride | 3.0 |
| Castor oil | 1.0 |
| Neopentyl glycol di-iso-octanate | 5.0 |
| Liquid paraffin | 8.0 |
| Cetanol | 6.0 |
| Dimethylpolysiloxane | 1.0 |
| γ-Sodium polyglutamate (MW about 360,000) | 3.0 |
| DL-tocopherol acetate | 0.2 |
| Purified water | Balance |

**(Prescription Example 11) Treatment**

| | Mass% |
|---|---|
| Avocado oil | 5.0 |
| Squalene | 5.0 |
| Liquid paraffin | 10.0 |
| Stearic acid | 3.0 |
| γ-Sodium polyglutamate (MW about 620,000) | 1.5 |
| Carboxymethyl chitin | 0.5 |
| Hydroxypropyl chitosan | 0.5 |
| Glycerin monostearate | 3.0 |
| Lanoline alcohol | 5.0 |
| Swertia extract | 1.0 |
| 1,3-Butylene glycol | 5.0 |
| Triethanolamine | 1.0 |
| Methyl paraben | Adequate amount |
| Purified water | Balance |

**(Prescription Example 12) Treatment**

| | Mass% |
|---|---|
| Stearyl trimethyl ammonium chloride | 1.5 |
| Phosphatidyl choline | 0.05 |
| Casein hydrolysate | 0.02 |
| γ-Sodium polyglutamate (MW about 510,000) | 2.5 |
| 3-Methyl-1,3-butanediol | 0.1 |
| Carpronium chloride | 0.2 |
| Japanese angelica extract | 0.5 |
| Liquid paraffin | 2.0 |
| Silicone oil | 3.0 |
| Dipropylene glycol | 5.0 |
| POE hydrogenated castor oil derivative (60 EO) | 5.0 |
| Perfume | Adequate amount |
| Ethanol | 30.0 |
| Purified water | Balance |

**(Prescription Example 13) Hair cream**

| | Mass% |
|---|---|
| Organopolysiloxane | 15.0 |
| Stearic acid | 0.5 |
| Squalene | 5.0 |
| Liquid paraffin | 10.0 |
| POE (20) cetylether | 1.5 |
| POE (2) cetylether | 2.5 |
| Sorbitan monostearate | 1.5 |
| Glyceryl monostearate | 0.5 |
| Triethanolamine | 0.2 |
| Polyvinylpyrrolidone | 5.0 |
| Propylene glycol | 10.0 |
| γ-Sodium polyglutamate (MW about 370,000) | 5.0 |
| Sesame extract | 0.5 |
| Tocopherol acetate | 0.2 |
| Antiseptic | Adequate amount |
| Perfume | Adequate amount |
| Purified water | Balance |

**(Prescription Example 14) Hair cream**

| | Mass% |
|---|---|
| Liquid paraffin | 10.0 |
| Squalene | 7.0 |
| Jojoba oil | 3.0 |
| Solid paraffin | 3.0 |
| Polyoxyethylene cetyl ether | 2.0 |
| γ-Sodium polyglutamate (MW about 270,000) | 5.0 |
| γ-Aminobutyrate | 0.2 |
| *Sanguisorba officinalis* extract | 0.3 |
| Sorbitan sesquioleate | 1.0 |
| Potassium hydroxide | 0.1 |
| Glycerin | 3.0 |
| Ethylparaben | 0.1 |
| Purified water | Balance |

**(Prescription Example 15) Hair oil**

| | Mass% |
|---|---|
| Decamethyl cyclopenta siloxane | 70.0 |
| γ-Sodium polyglutamate (MW about 520,000) | 2.0 |
| Green tea extract | 0.2 |
| Perilla extract | 0.3 |
| Trichlorotrifluoroethane | 7.0 |
| Isopropyl alcohol | 3.0 |
| Dimethylpolysiloxane | 4.0 |
| Soybean lecithin | 1.0 |
| Perfume | Adequate amount |
| Ethanol | Balance |

**(Prescription Example 16) Hair lotion**

| | Mass% |
|---|---|
| Ethyl glucoside | 2.0 |
| Eugenyl glucoside | 0.5 |
| Glycerin | 10.0 |
| Vinylpyrrolidone/N,N-dimethylaminoethyl methacrylate copolymer diethyl sulfate solution | 1.0 |
| Polyoxyethylene hydrogenated castor oil (60 EO) | 0.3 |
| Ethanol | 20.0 |
| γ-Sodium polyglutamate (MW about 440,000) | 2.5 |
| Allantoin | 0.3 |
| *Fragaria ananassa* extract | 0.4 |
| Grape leaf extract | 0.4 |
| Perfume | Adequate amount |
| Antiseptic | Adequate amount |
| Purified water | Balance |

**(Prescription Example 17) Hair lotion**

| | Mass% |
|---|---|
| Polyoxyethylene (50 EO) hydrogenated castor oil | 2.0 |
| Ethanol | 50.0 |
| γ-Sodium polyglutamate (MW about 250,000) | 2.0 |
| Hydroxypropyl cellulose | 1.0 |
| Lemon peel extract | 0.5 |
| Avocado oil | 1.0 |
| 1,2-Pentanediol | 1.0 |
| Pyridoxine hydrochloride | 0.5 |
| 1,3-Butylene glycol | 5.0 |
| Perfume | Adequate amount |
| Purified water | Balance |

**(Prescription Example 18) Hair lotion**

| | Mass% |
|---|---|
| Ethanol | 40.0 |
| Polyoxyethylene hydrogenated castor oil (60 EO) | 0.5 |
| Triethyl citrate | 3.0 |
| Methylphenyl polysiloxane | 4.0 |
| Dipropylene glycol | 2.0 |
| Diglycerin | 1.0 |
| γ-Sodium polyglutamate (MW about 400,000) | 1.5 |
| γ-Orizanol | 0.2 |
| Aspartic acid | 0.4 |
| Asparagine | 0.3 |
| Mixed fruit extract (lemon, apple, peach, and orange) | 1.0 |
| Mixed plant extract (arnica, hypericum, camomile, *Tilia japonica, Equisetum arvense,* yallow, sage, mallow, and *Calendula officinalis*) | 1.0 |
| *Panax ginseng* extract | 0.1 |
| Purified water | Balance |

**(Prescription Example 19) Hair-styling spray**

| | Mass% |
|---|---|
| Octylacrylamide/acryl hydroxypropyl | 3.0 |
| /butylaminoethyl methacrylate copolymer | |
| Acryl resin alkanolamine | 0.5 |
| Aminomethyl propanol | 0.6 |
| Camomile extract | 1.0 |
| γ-Sodium polyglutamate (MW about 180,000) | 1.0 |
| Grapefruit extract | 0.5 |
| Purified water | 2.0 |
| Perfume | Adequate amount |
| Ethanol | Balance |
| Propellant (LPG) | 6.0 |

**(Prescription Example 20) Hair foam**

| | Mass% |
|---|---|
| Alkyl denaturated organopolysiloxane | 2.0 |
| Mixed plant extract (arnica, stinging nettle, cucumber, *Hedera helix,* mallow, eldertree) | 1.0 |
| Scutellaria extract | 0.1 |
| Polyoxyethylene hydrogenated castor oil (60 EO) | 0.5 |
| Polyoxyethylene cetyl ether (10 EO) | 0.3 |
| γ-Sodium polyglutamate (MW about 330,000) | 5.0 |
| Sodium pyrrolidone carboxylic acid | 0.5 |
| Ergocalciferol | 0.1 |
| Chondroitin sulfate sodium salt | 1.0 |
| Purified water | Balance |

**(Prescription Example 21) Hair gel**

| | Mass% |
|---|---|
| Carboxyvinyl polymer | 0.5 |
| Glycerin | 2.0 |
| γ-Sodium polyglutamate (MW about 520,000) | 5.0 |
| Tocotrienol acetate | 0.2 |
| *Hydrangea macrophylla* extract | 0.5 |
| Clove extract | 0.3 |
| Hinokithiol | 1.0 |
| Ethanol | 20.0 |
| Polyoxyethylene (20 EO) stearyl ether | 0.2 |
| Perfume | Adequate amount |
| Purified water | Balance |

**(Prescription Example 22) Hair water**

| | Mass% |
|---|---|
| Orange leaf extract | 0.5 |
| Polyoxyethylene denaturated silicone | 0.5 |
| 3-Methyl-1,3-butanediol | 2.0 |
| γ-Sodium polyglutamate (MW about 210,000) | 1.5 |
| Tocotrienol nicotinate | 0.1 |
| Nor-dihydro-guaiaretic acid | 0.1 |
| Stearyl trimethyl ammonium chloride | 0.5 |
| Polyoxyethylene laurate | 0.4 |
| Lauryldimethylamine oxide | 0.2 |
| Sodium pyrrolidone carboxylic acid | 1.0 |
| Methyl paraben | 0.3 |
| Sodium citrate | 0.1 |
| Hydroxy methoxy benzophenone sodium sulfonate | 0.1 |
| Perfume | Adequate amount |
| Ethanol | 20.0 |
| Purified water | Balance |

**(Prescription Example 23) Hair-coloring pre-treatment agent**

| | Mass% |
|---|---|
| Beeswax | 3.0 |
| Behenyl trimethyl ammonium chloride | 8.0 |
| Egg-yolk lecithin | 2.5 |
| γ-Sodium polyglutamate (MW about 380,000) | 5.0 |
| Urea | 0.4 |
| Aloe extract | 0.5 |
| Vaseline | 5.0 |
| Stearyl alcohol | 5.0 |
| Squalene | 10.0 |
| Liquid paraffin | 30.0 |
| Polyoxyethylene (20) cetylether | 1.5 |
| Polyoxyethylene (6) cetylether | 2.5 |
| Glycerin monostearate | 0.5 |
| 4-4'dichloro-2-hydroxy diphenylether | 0.5 |
| EDTA-3Na | 0.1 |
| Perfume | Adequate amount |
| Purified water | Balance |

**(Prescription Example 24) Hair-coloring pre-treatment agent**

| | Mass% |
|---|---|
| Myristate palmitic acid | 6.0 |
| Glycerin behenic acid eicosane dicarboxylate | 0.4 |
| 2-Cetyl ethylhexanoate | 3.0 |
| 2-Octyl dodecanol | 3.0 |
| Cetanol | 2.0 |
| γ-Sodium polyglutamate (MW about 450,000) | 5.0 |
| Sphingomyelin | 0.2 |
| Cladosiphon okamuranus | 0.8 |
| extract | |
| Glycerin | 5.0 |
| Hydroxyethyl cellulose | 0.5 |
| Citric acid | 1.0 |
| Perfume | Adequate amount |
| Coloring matter | Adequate amount |
| Antiseptic | Adequate amount |
| Purified water | Balance |

**(Prescription Example 25) Hair-coloring post-treatment agent**

| | Mass% |
|---|---|
| Hexaglyceryl monoisostearate | 5.0 |
| Carboxyvinyl polymer | 0.5 |
| Quince seed gum | 0.3 |
| Methylsiloxane/polyoxyethylene copolymer | 2.0 |
| γ-Sodium polyglutamate (MW about 520,000) | 4.5 |
| Glucosyl ascorbic acid | 0.3 |
| Tocopherylretinoate | 0.1 |
| *Nasturtium Officinale* extract | 0.6 |
| Polyoxyethylene hydrogenated castor oil | 0.3 |
| Dihydroxybenzophenone | 0.1 |
| Methyl paraben | 0.1 |
| Triethanolamine | 0.5 |
| Perfume | Adequate amount |
| Ethanol | 5.0 |
| Purified water | Balance |

**(Prescription Example 26) Hair-coloring post-treatment agent**

| | Mass% |
|---|---|
| Dipentaerythrite fatty acid ester | 1.0 |
| 4-Guanidinobutyl lauroamide | 1.0 |
| Stearyl trimethyl ammonium chloride | 0.2 |
| γ-Sodium polyglutamate (MW about 220,000) | 5.0 |
| Lutein | 0.1 |
| Beech leaf extract | 0.2 |
| Scutellaria extract | 0.2 |
| Cetanol | 1.6 |
| Behenyl alcohol | 0.2 |
| Oleyl alcohol | 0.2 |
| Oleic acid | 0.2 |
| Isostearic acid | 0.2 |
| Oleyl oleate | 0.2 |
| Glyceryl monostearate | 0.1 |
| Sorbitan monolaurate | 0.1 |
| Polyoxyethylene (40) hydrogenated castor oil | 1.0 |
| Pyrrolidone carboxylate | Adequate amount |
| Glycolic acid | Adequate amount |
| Methyl p-hydroxybenzoate | Adequate amount |
| Propyl p-hydroxybenzoate | Adequate amount |
| Perfume | Adequate amount |
| Purified water | Balance |

**(Prescription Example 27) Permanent pre-treatment agent**

| | Mass% |
|---|---|
| Beeswax | 3.0 |
| Vaseline | 5.0 |
| Stearyl alcohol | 5.0 |
| γ-Sodium polyglutamate (MW about 180,000) | 3.0 |
| Retinoic acid | 0.1 |
| Cystine | 0.2 |
| Liquid paraffin | 30.0 |
| Squalene | 10.0 |
| POE (60) cetylether | 2.5 |
| Maltitol | 0.2 |
| Dimethylpolysiloxane | 13.0 |
| EDTA-2Na | 0.1 |
| Perfume | Adequate amount |
| Antiseptic | Adequate amount |
| Purified water | Balance |

**(Prescription Example 28) Permanent pre-treatment agent**

| | Mass% |
|---|---|
| Cnidium extract | 0.5 |
| Hexaglyceryl tristearate | 3.0 |
| Alkyl denaturated carboxyvinyl polymer | 0.4 |
| Karaya gum | 0.1 |
| Methylpolysiloxane | 5.0 |
| Liquid paraffin | 5.0 |
| Paraffin wax | 3.0 |
| Polyoxyethylene hydrogenated castor oil | 2.0 |
| 3-Methyl-1,3-butanediol | 10.0 |
| γ-Sodium polyglutamate (MW about 260,000) | 3.0 |
| Chamazulene | 0.2 |
| Diclofenac sodium | 0.2 |
| Dihydroxy benzophenone | 0.1 |
| Propylparaben | 0.1 |
| Methyl paraben | 0.3 |
| Phenoxyethanol | 0.5 |
| Coconut oil-fatty acid sorbitan | 2.0 |
| Glycerin monostearate | 1.0 |
| Polypropylene glycol monostearate | 2.0 |
| Triethanolamine | 0.5 |
| Perfume | Adequate amount |
| Purified water | Balance |

**(Prescription Example 29) Permanent post-treatment agent**

| | Mass% |
|---|---|
| Polyoxyethylene cetyl ether | 4.0 |
| Lanolin | 4.0 |
| γ-Sodium polyglutamate (MW about 510,000) | 5.0 |
| Cetyl alcohol | 0.5 |
| Ethanol | 5.0 |
| Liquid paraffin | 40.0 |
| P-dimethylamino octyl benzoate | 1.5 |
| Salvia extract | 5.0 |
| Methyl p-hydroxybenzoate | 0.1 |
| Perfume | 0.1 |
| Purified water | Balance |

**(Prescription Example 30) Permanent post-treatment agent**

| | Mass% |
|---|---|
| Beeswax | 3.0 |
| γ-Sodium polyglutamate (MW about 420,000) | 5.0 |
| Vaseline | 5.0 |
| Stearyl alcohol | 5.0 |
| Liquid paraffin | 25.0 |
| Squalene | 10.0 |
| POE (60) cetylether | 2.5 |
| Dimethylpolysiloxane | 2.0 |
| Rooibos extract | 0.4 |
| EDTA-2Na | 0.1 |
| Paraben | 0.1 |
| Perfume | 0.1 |
| Purified water | Balance |

**(Prescription Example 31) Hair-restoration and growth agent**

| | Mass% |
|---|---|
| Ethanol | 60.0 |
| γ-Sodium polyglutamate (MW about 310,000) | 2.0 |
| DL-α-Tocopherol acetate | 0.5 |
| Capsicum tincture | 0.5 |
| Aloe extract | 3.0 |
| Resorcin | 0.5 |
| Dipotassium glycyrrhizinate | 0.5 |
| Carboxymethyl chitin | 0.5 |
| Collagen protein enzymolysis product | 0.2 |
| Arginine | 0.25 |
| Glutamine | 0.25 |
| Antibacterial agent | Adequate amount |
| Perfume | Adequate amount |
| Purified water | Balance |

**(Prescription Example 32) Hair-restoration and growth agent**

| | Mass% |
|---|---|
| γ-Sodium polyglutamate (MW about 250,000) | 2.0 |
| Ethanol | 60.0 |
| Minoxidil | 5.0 |
| DL-α-Tocopherol acetate | 0.5 |
| Cantharis tincture | 0.5 |
| Birch extract | 1.0 |
| Calcium pantothenate | 0.5 |
| β-Glycyrrhetic acid | 0.5 |
| Hydroxyethyl chitosan | 0.5 |
| Collagen protein enzymolysis product | 0.2 |
| Antibacterial agent | Adequate amount |
| Perfume | Adequate amount |
| Purified water | Balance |

### Industrial Applicability

The composition for hair treatment containing γ-polyglutamic acid or a salt thereof used according to the present invention has excellent improvement effects on the strength and frictional force of hair, so that it can provide tension or elasticity, or the like to damaged hair to alleviate split hair and broken hair as well as improvements in combing and touch. In addition, the use of the γ-polyglutamic acid or a salt thereof on damaged hair prevents or alleviates various problems associated with the deterioration of the hair strength mentioned above and prevents or alleviats various problems associated in an increase in hair frictional force. Furthermore, the effect of moisture retention inherent to γ-polyglutamic acid or a salt thereof is achieved, thereby preventing or alleviating the generation of dandruff on the basis of such an effect, preventing the feeling of stickiness or creak.

## Claims

1. Use of γ-polyglutamic acid or a salt thereof for imparting strength to damaged hair and lowering the frictional force of hair.

## Patentansprüche

1. Verwendung von γ-Polyglutaminsäure oder eines Salzes davon, um geschädigtem Haar Festigkeit zu verleihen und die Reibkraft von Haar zu vermindern.

## Revendications

1. Utilisation d'acide γ-polyglutamique ou d'un de ses sels pour renforcer des cheveux endommagés ou diminuer la force de friction des cheveux.
